# EUROPÄISCHE PATENTSCHRIFT

(11) **EP 1 408 962 B1**
(45) Veröffentlichungstag und Bekanntmachung des Hinweises auf die Patenterteilung: **15.03.2006**
(21) Anmeldenummer: 02772093.7
(22) Anmeldetag: 03.07.2002
(51) Int. Cl.: A61K 31/4035, C07D 409/12, C07D 409/14, C07D 471/04, C07D 487/04, A61P 7/02

(54) **SUBSTITUIERTE ISOINDOLE UND IHRE VERWENDUNG**
SUBSTITUTED ISOINDOLES AND THE USE THEREOF
ISOINDOLES SUBSTITUES ET LEUR UTILISATION

(30) Priorität: 16.07.2001 DE 10134482
(43) Veröffentlichungstag der Anmeldung: 21.04.2004
(73) Patentinhaber: Bayer HealthCare AG, 51368 Leverkusen (DE)
(72) Erfinder: STRAUB, Alexander, 42117 Wuppertal (DE); LAMPE, Thomas, 40223 Düsseldorf (DE); POHLMANN, Jens, 42285 Wuppertal (DE); RÖHRIG, Susanne, 40724 Hilden (DE); JORDAN, Stephan, 51061 Köln (DE); PERZBORN, Elisabeth, 42327 Wuppertal (DE); SCHLEMMER, Karl-Heinz, 42113 Wuppertal (DE)
(86) Internationale Anmeldenummer: PCT/EP2002/007326
(87) Internationale Veröffentlichungsnummer: WO 2003/007942

(56) Entgegenhaltungen:
- WO-A-99/00121
- US-A- 6 140 351
- AL-OBEIDI F ET AL: "FACTOR XA INHIBITORS" EXPERT OPINION ON THERAPEUTIC PATENTS, ASHLEY PUBLICATIONS, GB, Bd. 9, 1999, Seiten 931-953, XP001000512 ISSN: 1354-3776 in der Anmeldung erwähnt
- BETZ A: "Recent advances in Factor Xa inhibitors" EXPERT OPINIONS ON THERAPEUTIC PATENTS, ASHLEY PUBLICATIONS, GB, Bd. 11, Nr. 2001, Seiten 1007-1017, XP002220120

## Beschreibung

Die vorliegende Erfindung betrifft das Gebiet der Blutgerinnung. Insbesondere betrifft die vorliegende Erfindung neue Isoindol-Derivate, Verfahren zu ihrer Herstellung sowie ihre Verwendung als Wirkstoffe in Arzneimitteln.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden bzw. minimiert werden. Die Blutstillung nach Gefäßverletzung erfolgt im wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und extrinsischen System, die in einem abschließenden gemeinsamen Reaktionsweg münden. Hierbei kommt dem Faktor Xa, der aus dem Proenzym Faktor X gebildet wird, eine Schlüsselrolle zu, da er beide Gerinnungswege verbindet. Die aktivierte Serinprotease Xa spaltet Prothrombin zu Thrombin. Das entstandene Thrombin wiederum spaltet seinerseits Fibrinogen zu Fibrin, einem faserig-gallertigen Gerinnungsstoff. Darüber hinaus ist Thrombin ein potenter Auslöser der Thrombozytenaggregation, die ebenfalls einen erheblichen Beitrag bei der Hämostase leistet.

Die Aufrechterhaltung der normalen Hämostase - zwischen Blutung und Thrombose - unterliegt einem komplexen Regulationsmechanismus. Die unkontrollierte Aktivierung des Gerinnungssystems oder eine defekte Hemmung der Aktivierungsprozesse kann die Bildung von lokalen Thromben oder Embolien in Gefäßen (Arterien, Venen, Lymphgefäßen) oder Herzhöhlen bewirken. Dies kann zu schwerwiegenden Erkrankungen wie Herzinfarkt, Angina Pectoris (eingeschlossen instabile Angina), Reokklusionen und Restenosen nach einer Angioplastie oder aortokoronarem Bypass, Hirnschlag, transitorische ischämische Attacken, periphere arterielle Verschlusskrankheiten, Lungenembolien oder tiefen venösen Thrombosen führen; diese Erkrankungen werden im folgenden zusammenfassend auch als thromboembolische Erkrankungen bezeichnet. Darüber hinaus kann eine Hyperkoagulabilität - systemisch - bei einer Verbrauchskoagulopathie zur disseminierten intravasalen Gerinnung führen.

Diese thromboembolischen Erkrankungen sind die häufigste Ursache von Morbidität und Mortalität in den meisten industrialisierten Ländern (Pschyrembel, Klinisches Wörterbuch, 257. Auflage, 1994, Walter de Gruyter Verlag, Seite 199 ff., Stichwort "Blutgerinnung"; Römpp Lexikon Chemie, Version 1.5, 1998, Georg Thieme Verlag Stuttgart, Stichwort "Blutgerinnung"; Lubert Stryer, Biochemie, Spektrum der Wissenschaft Verlagsgesellschaft mbH Heidelberg, 1990, Seiten 259 ff.).

Die aus dem Stand der Technik bekannten Antikoagulantien, d.h. Stoffe zur Hemmung oder Verhinderung der Blutgerinnung, weisen verschiedene, oftmals gravierende Nachteile auf. Eine effiziente Behandlungsmethode bzw. Prävention von thromboembolischen Erkrankungen erweist sich in der Praxis deshalb als sehr schwierig und unbefriedigend.

Für die Therapie und Prävention von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Da Heparin gleichzeitig mehrere Faktoren der Blutgerinnungskaskade hemmt, kommt es zu einer unselektiven Wirkung. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen (Pschyrembel, Klinisches Wörterbuch, 257. Auflage, 1994, Walter de Gruyter Verlag, Seite 610, Stichwort "Heparin"; Römpp Lexikon Chemie, Version 1.5, 1998, Georg Thieme Verlag Stuttgart, Stichwort "Heparin").

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung aber nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig. Darüber hinaus sind weitere Nebenwirkungen wie gastrointestinale Störungen, Haarausfall und Hautnekrosen beschrieben (Pschyrembel, Klinisches Wörterbuch, 257. Auflage, 1994, Walter de Gruyter Verlag, Seite 292 ff., Stichwort "Cumarinderivate"; Ullmann's Encyclopedia of Industrial Chemistry, 5. Auflage, VCH Verlagsgesellschaft, Weinheim, 1985 - 1996, Stichwort "Vitamin K").

In jüngster Zeit ist ein neuer Therapieansatz für die Behandlung und Prävention von thromboembolischen Erkrankungen beschrieben worden. Ziel dieses neuen Therapieansatzes ist die Inhibierung von Faktor Xa (vgl. WO-A-99/37304; WO-A-99/06371; J. Hauptmann, J. Stürzebecher, Thrombosis Research **1999,** *93,*203; F. Al-Obeidi, J. A. Ostrem, Factor Xa inhibitors by classical and combinatorial chemistry, DDT **1998,** *3*, 223; F. Al-Obeidi, J. A. Ostrem, Factor Xa inhibitors, Exp. Opin. Ther. Patents **1999,** *9*, 931; A. Betz, Recent advances in Factor Xa inhibitors, Exp. Opin. Ther. Patents **2001,** *11*, 1007; B. Kaiser, Thrombin and factor Xa inhibitors, Drugs of the Future **1998,** *23*, 423; A. Uzan, Antithrombotic agents, Emerging Drugs **1998,** *3*, 189; B.-Y. Zhu, R. M. Scarborough, Curr. Opin. Card. Pulm. Ren. Inv. Drugs **1999,** *1* (*1*), 63). Dabei ist gezeigt worden, dass verschiedene, sowohl peptidische wie nichtpeptidische Verbindungen in Tiermodellen als Faktor Xa-Inhibitoren wirksam sind.

WO 99/00121 A beschreibt aromatische Faktor Xa-Inhibitoren wie beispielsweise N-(4-Methoxybenzoyl)-2-(5-amino-1,3-dihydro-1,3-dioxo-2H-isoindol-2-yl)benzoldiamin oder N¹-(5-Chlorthiophen-2-ylcarbonyl)-N²-[1-(4-pyridyl)piperidin-4-yl-methoxycarbonyl]-1,2-benzoldiamin-hydrochlorid. US-A-6 140 351 beschreibt *ortho*-Anthranilamid-Derivate als Faktor Xa-Inhibitoren.

Aufgabe der vorliegenden Erfindung ist nunmehr die Bereitstellung neuer Substanzen zur Bekämpfung von Erkrankungen, die eine große therapeutische Bandbreite aufweisen.

Sie sollen insbesondere zur effizienteren Prävention und/oder Behandlung von thromboembolischen Erkrankungen geeignet sein und hierbei die zuvor geschilderten Nachteile des Standes der Technik - zumindest teilweise - vermeiden, wobei unter dem Begriff "thromboembolische Erkrankungen" im Sinne der vorliegenden Erfindung insbesondere schwerwiegende Erkrankungen wie Herzinfarkt, Angina Pectoris (eingeschlossen instabile Angina), Reokklusionen und Restenosen nach einer Angioplastie oder aortokoronarem Bypass, Hirnschlag, transitorische ischämische Attacken, periphere arterielle Verschlusskrankheiten, Lungenembolien oder tiefe venöse Thrombosen verstanden werden.

Weitere Aufgabe der vorliegenden Erfindung ist die Bereitstellung neuer Antikoagulantien, welche mit erhöhter Selektivität den Blutgerinnungsfaktor Xa inhibieren und hierbei die Probleme der aus dem Stand der Technik bekannten Therapiemethoden für thromboembolische Erkrankungen - zumindest teilweise - vermeiden sollen.

Gegenstand der vorliegenden Erfindung sind Verbindungen der Formel (I) 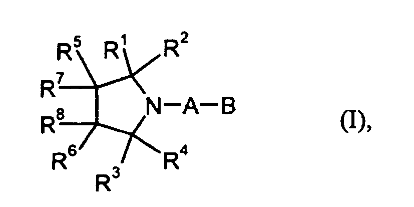 worin
- R¹ und R²: gemeinsam für O stehen und
- R³ und R⁴: gemeinsam für O stehen, oder
- R¹: Wasserstoff, Hydroxy oder (C₁-C₄)-Alkoxy bedeutet,
- R²: Wasserstoff bedeutet und
- R³ und R⁴: gemeinsam für O stehen,
oder
- R¹ und R²: gemeinsam für O stehen,
- R³: Wasserstoff, Hydroxy oder (C₁-C₄)-Alkoxy bedeutet und
- R⁴: Wasserstoff bedeutet,
- R⁵ und R⁶: für Wasserstoff stehen und
- R⁷ und R⁸: gemeinsam für 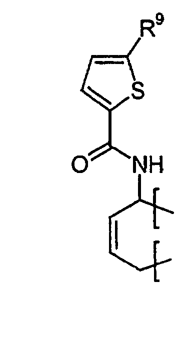 stehen,
worin
R⁹ Halogen, Trifluormethyl oder Methyl bedeutet,
oder
- R⁵, R⁶, R⁷ und R⁸: gemeinsam für 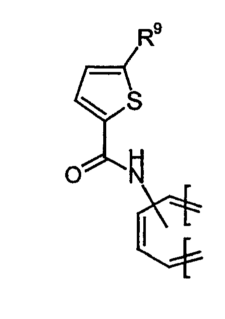
stehen,
worin
R⁹ die oben angegebene Bedeutung besitzt,
- A: (C₁-C₄)-Alkandiyl oder (C₂-C₄)-Alkendiyl bedeutet,
- B: Phenylen oder Cyclohexandiyl bedeutet, die durch Amino, Harnstoff, Sulfamoyl, -C(=N-NH-C(=NH)-NH₂)-H, -C(=NR¹⁰)-R¹¹
worin
R¹⁰ Wasserstoff oder -NH-C(=NH)-NH₂ bedeutet,
R¹¹ -NR¹²R¹³ oder 5- bis 10-gliedriges Heterocyclyl bedeutet,worin
R¹² und R¹³, unabhängig voneinander, Wasserstoff, (C₁-C₄)-Alkyl oder (C₃-C₇)-Cycloalkyl bedeuten, (C₁-C₄)-Alkyl, das seinerseits durch Cyano, (C₁-C₄)-Alkoxycarbonyl, gegebenenfalls durch (C₁-C₄)-Alkyl substituiertes 5- bis 10-gliedriges Heterocyclyl, 5- bis 10-gliedriges Heteroaryl, Tri-(C₁-C₄)-alkylammonium,
-NR¹⁴R¹⁵, -C(=NR¹⁶)-R¹⁷, -N-C(=O)-R¹⁸ oder -N-C(=O)-NH-R¹⁹ substituiert sein kann,
worin
R¹⁴ Wasserstoff, Mono- oder Di-(C₁-C₄)-alkylamino, gegebenenfalls durch 5- bis 10-gliedriges Heteroaryl substituiertes (C₁-C₄)-Alkyl, 5-bis 10-gliedriges Heterocyclyl, (C₆-C₁₀)-Aryl oder 5- bis 10-gliedriges Heteroaryl, wobei die Ringe ihrerseits durch Halogen substituiert sein können, bedeutet,
R¹⁵ Wasserstoff oder gegebenenfalls durch Cyano substituiertes (C₁-C₄)-Alkyl bedeutet,
R¹⁶ Wasserstoff oder Hydroxy bedeutet,
R¹⁷ Amino, 5- bis 10-gliedriges Heterocyclyl, gegebenenfalls durch Amino oder Trifluormethyl substituiertes Mono- oder Di-(C₁-C₄)-Alkylamino, gegebenenfalls durch Trifluormethyl substituiertes (C₃₋C₇)-Cycloalkylamino bedeutet,
R¹⁸ Trifluormethyl, (C₁-C₄)-Alkyl, (C₁-C₄)-Alkoxy oder gegebenenfalls durch (C₁-C₄)-Alkyl substituiertes 5- bis 10-gliedriges Heterocyclyl bedeutet,
R¹⁹ Wasserstoff, Amino, Dimethylamino, 5- bis 10-gliedriges Heterocyclyl, 5- bis 10-gliedriges Heteroaryl oder gegebenenfalls durch (C₁₋C₄)-Alkoxycarbonyl, Dimethylamino, Carbamoyl oder 5- bis 10-gliedriges Heteroaryl substituiertes (C₁-C₄)-Alkyl bedeutet, (C₁-C₄)-Alkoxy, das seinerseits ein- oder zweifach, unabhängig voneinander, durch Cyano, Thiocarbamoyl, gegebenenfalls durch (C₁-C₄)-Alkyl substituiertes 5- bis 10-gliedriges Heterocyclyl, gegebenenfalls durch Halogen substituiertes 5- bis 10-gliedriges Heteroaryl, gegebenenfalls durch -C(=NR²⁰)-R²¹ substituiertes (C₆-C₁₀)-Aryl oder -C(=NR²⁰)-R²¹ substituiert sein kann,
worin
R²⁰ Wasserstoff, Hydroxy, (C₃-C₇)-Cycloalkyl oder gegebenenfalls durch Hydroxy substituiertes (C₁-C₄)-Alkyl bedeutet,
R²¹ Amino, (C₁-C₄)-Alkylthio, (C₃-C₇)-Cycloalkylamino, Benzylamino oder 5- bis 10-gliedriges Heterocyclyl bedeutet, 5- bis 10-gliedriges Heterocyclyl, das seinerseits durch (C₁-C₄)-Alkyl substituiert sein kann,
(C₆-C₁₀)-Aryl, das seinerseits ein- oder zweifach, unabhängig voneinander, durch Halogen, Cyano, Carbamoyl oder gegebenenfalls durch Amino substituiertes (C₁-C₄)-Alkyl substituiert sein kann,
5- bis 10-gliedriges Heteroaryloxy, das seinerseits durch Amino oder N-(C₁-C₄)-Alkylaminocarbonyl substituiert sein kann,
oder 5- bis 10-gliedriges Heteroaryl, das seinerseits durch Amino substituiert sein kann, substituiert sein können,

sowie deren Salze, Hydrate, Hydrate der Salze und Solvate.

Die erfindungsgemäßen Verbindungen der Formel (I) können in Abhängigkeit von dem Substitutionsmuster in stereoisomeren Formen, die sich entweder wie Bild und Spiegelbild (Enantiomere) oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen.

Weiterhin können bestimmte Verbindungen der Formel (I) in tautomeren Formen vorliegen. Dies ist dem Fachmann bekannt, und derartige Verbindungen sind ebenfalls vom Umfang der Erfindung umfasst.

Salze der erfindungsgemäßen Verbindungen sind physiologisch unbedenkliche Salze der erfindungsgemäßen Verbindungen mit anorganischen oder organischen Säuren. Bevorzugt werden Salze mit anorganischen Säuren wie beispielsweise Chlorwasserstoffsäure, Bromwasserstoffsäure, Phosphorsäure oder Schwefelsäure, oder Salze mit organischen Carbon- oder Sulfonsäuren wie beispielsweise Essigsäure, Trifluoressigsäure, Propionsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Salze können ebenso physiologisch unbedenkliche Salze mit üblichen Basen sein, wie beispielsweise Alkalimetallsalze (z.B. Natrium- oder Kaliumsalze), Erdalkalisalze (z.B.

Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin oder Methylpiperidin.

Ausserdem umfasst die Erfindung auch Prodrugs der erfindungsgemäßen Verbindungen. Als Prodrugs werden erfindungsgemäß solche Formen der Verbindungen der Formel (I) bezeichnet, welche selbst biologisch aktiv oder inaktiv sein können, jedoch unter physiologischen Bedingungen in die entsprechende biologisch aktive Form über-Rihrt werden können (beispielsweise metabolisch oder solvolytisch).

Als "Hydrate" bzw. "Solvate" werden erfindungsgemäß solche Formen der Verbindungen der Formel (I) bezeichnet, welche in festem oder flüssigem Zustand durch Hydratation mit Wasser oder Koordination mit Lösungsmittelmolekülen eine Molekül-Verbindung bzw. einen Komplex bilden. Beispiele für Hydrate sind Sesquihydrate, Monohydrate, Dihydrate oder Trihydrate. Gleichermaßen kommen auch die Hydrate bzw. Solvate von Salzen der erfindungsgemäßen Verbindungen in Betracht.

Halogen steht für Fluor, Chlor, Brom und Iod. Bevorzugt sind Chlor, Brom oder Fluor.

(C₁-C₄)-Alkyl steht für einen geradkettigen oder verzweigten Alkylrest mit 1 bis 4 Kohlenstoffatomen. Beispielsweise seien genannt: Methyl, Ethyl, n-Propyl, Isopropyl, n-Butyl, Isobutyl und tert.-Butyl. Aus dieser Definition leiten sich analog die entsprechenden Alkylgruppen mit weniger Kohlenstoffatomen wie z.B. (C₁-C₃)-Alkyl ab. Im allgemeinen gilt, dass (C₁-C₃)-Alkyl bevorzugt ist.

Aus dieser Definition leitet sich auch die Bedeutung des entsprechenden Bestandteils anderer komplexerer Substituenten ab wie z.B. bei Mono- oder Dialkylamino, Trialkylammonium oder N-Alkylaminocarbonyl.

Monoalkylamino steht für eine Amino-Gruppe mit einem wie oben definierten Alkylsubstituenten. Dialkylamino steht für eine Amino-Gruppe mit zwei, gleichen oder verschiedenen, wie oben definierten Alkylsubstituenten. Trialkylammonium steht für eine Amino-Gruppe mit drei, gleichen oder verschiedenen, wie oben definierten Alkylsubstituenten. N-Alkylaminocarbonyl steht für eine Amino-Gruppe mit einem wie oben definierten Alkylrest, die über eine Carbonylgruppe verknüpft ist

(C₁-C₄)-Alkandiyl steht für einen geradkettigen oder verzweigten Alkandiylrest mit 1 bis 4 Kohlenstoffatomen. Bevorzugt ist ein geradkettiger oder verzweigter Alkandiylrest mit 1 bis 3 Kohlenstoffatomen. Beispielsweise seien genannt: Methandiyl, Ethandiyl, Propan-1,3-diyl, Propan-1,2-diyl, Propan-2,2-diyl, Butan-1,4-diyl, Butan-1,3-diyl.

(C₂-C₄)-Alkendiyl steht für einen geradkettigen oder verzweigten Alkendiylrest mit 2 bis 4 Kohlenstoffatomen, bevorzugt mit 2 bis 3 Kohlenstoüatomen. Beispielsweise seien genannt: Ethen-1,2-diyl, Ethen-1,1-diyl, Propen-1,1-diyl, Propen-1,2-diyl, Propen-1,3-diyl, Propen-3,3-diyl, Propen-2,3-diyl, But-2-en-1,4-diyl.

(C₃-C₇)-Cycloalkyl steht für einen cyclischen Alkylrest mit 3 bis 7 Kohlenstoffatomen. Beispielsweise seien genannt: Cyclopropyl, Cyclobutyl, Cyclopentyl, Cyclohexyl oder Cycloheptyl. Aus dieser Definition leiten sich die entsprechenden Cycloalkylreste mit weniger Kohlenstoffatomen wie (C₃-C₆)-Cycloalkyl ab. Bevorzugt sind Cyclopropyl, Cyclopentyl und Cyclohexyl.

Aus dieser Definition leitet sich auch die Bedeutung des entsprechenden Bestandteils anderer komplexerer Substituenten ab wie z.B. Cycloalkylamino. Cycloalkylamino steht für einen wie oben definierten Cycloalkylrest, der über eine Aminogruppe verknüpft ist

(C₁-C₄)-Alkoxy steht für einen geradkettigen oder verzweigten Alkoxyrest mit 1 bis 4 Kohlenstoffatomen. Beispielsweise seien genannt: Methoxy, Ethoxy, n-Propoxy, Isopropoxy, n-Butoxy, Isobutoxy, tert.-Butoxy. Aus dieser Definition leiten sich analog die entsprechenden Alkoxygruppen mit weniger Kohlenstoffatomen wie z.B. (C₁-C₃)-Alkoxy ab. Im allgemeinen gilt, dass (C₁-C₃)-Alkoxy bevorzugt ist.

(C₁-C₄)-Alkoxycarbonyl steht für einen geradkettigen oder verzweigten Alkoxyrest mit 1 bis 4 Kohlenstoffatomen, der über eine Carbonylgruppe verknüpft ist. Beispielsweise seien genannt: Methoxycarbonyl, Ethoxycarbonyl, n-Propoxycarbonyl, Isopropoxycarbonyl und t-Butoxycarbonyl. Bevorzugt ist ein Alkoxycarbonylrest mit 1 oder 2 Kohlenstoffatomen

(C₆-C₁₀)-Aryl steht für einen aromatischen Rest mit 6 bis 10 Kohlenstoffatomen. Beispielsweise seien genannt: Phenyl und Naphthyl. Im allgemeinen gilt, dass Phenyl bevorzugt ist.

5- bis 10-gliedriges Heteroaryl steht für einen mono- oder bicyclischen gegebenenfalls benzokondensierten aromatischen Heterocyclus (Heteroaromaten) mit bis zu 3 Heteroatomen aus der Reihe N, O und/oder S, der über ein Ringkohlenstoffatom oder Ringstickstoffatom des Heteroaromaten verknüpft ist. Beispielsweise seien genannt: Pyridyl, Pyridyl-N-oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl. Aus dieser Definition leiten sich analog die entsprechenden Heteroaromaten mit geringerer Ringgröße wie z.B. 5- oder 6-gliedriges Heteroaryl ab. Im allgemeinen gilt, dass 5- oder 6-gliedriges Heteroaryl wie z.B. Pyridyl, Pyrimidyl, Pyridazinyl und Pyrazolyl bevorzugt sind.

Aus dieser Definition leitet sich auch die Bedeutung des entsprechenden Bestandteils anderer komplexerer Substituenten ab wie z.B. Heteroaryloxy. Heteroaryloxy steht für einen wie oben definierten Heteroarylrest, der über ein Sauerstoffatom verknüpft ist.

5- bis 10-gliedriges Heterocyclyl steht für einen gesättigten oder teilweise ungesättigten, mono- oder bicyclischen, gegebenenfalls benzokondensierten Heterocyclus mit bis zu 3 Heteroatomen aus der Reihe S, N und/oder O, der über ein Ringkohlenstoffatom oder ein Ringstickstoffatom verknüpft ist. Beispielsweise seien genannt: Tetrahydrofuryl, Pyrrolidinyl, Pyrrolinyl, Piperidinyl, 1,2-Dibydropyridinyl, 1,4-Dihydropyridinyl, Piperazinyl, Morpholinyl, Morpholinyl-N-oxid, Thiomorpholinyl, Azepinyl, Imidazolinyl, Tetrahydropyrimidinyl und 1,4-Diazepinyl. Aus dieser Definition leiten sich analog die entsprechenden Heterocyclen mit geringerer Ringgröße wie z.B. 5- oder 6-gliedrige Heterocyclen ab. Im allgemeinen gilt, dass 5-oder 6-gliedrige Heterocyclen wie Piperidinyl, Morpholinyl, Piperazinyl, Imidazolinyl, Tetrahydropyrimidinyl und Pyrrolidinyl bevorzugt sind.

Bevorzugt sind Verbindungen der Formel (I),
worin
- R¹ und R²: gemeinsam für O stehen und
- R³ und R⁴: gemeinsam für O stehen,
oder
- R¹: Wasserstoff, Hydroxy, Methoxy oder Ethoxy bedeutet,
- R²: Wasserstoff bedeutet und
- R³ und R⁴: gemeinsam für O stehen,
oder
- R¹ und R²: gemeinsam für O stehen,
- R³: Wasserstoff, Hydroxy, Methoxy oder Ethoxy bedeutet und
- R⁴: Wasserstoff bedeutet,
- R⁵ und R⁶: für Wasserstoff stehen und
- R⁷ und R⁸: gemeinsam für 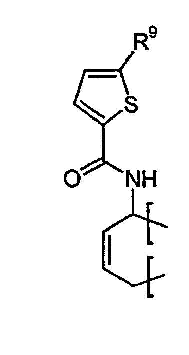
stehen,
worin
R⁹ Halogen oder Trifluormethyl bedeutet,
oder
- R⁵, R⁶, R⁷ und R⁸: gemeinsam für 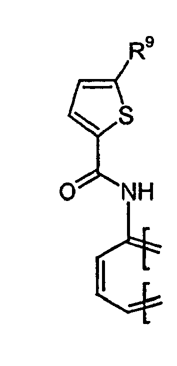
stehen,
worin
R⁹ die oben angegebene Bedeutung besitzt,
- A: (C₁-C₃)-Alkandiyl oder (C₂-C₃)-Alkendiyl bedeutet,
- B: Phenylen oder Cyclohexandiyl bedeutet, die durch -C(=NR¹⁰)-R¹¹
worin
R¹⁰ Wasserstoff bedeutet,
R¹¹ -NR¹²R¹³ oder 5- bis 10-gliedriges Heterocyclyl bedeutet,
worin
R¹² und R¹³, unabhängig voneinander, Wasserstoff, (C₁-C₄)-Alkyl
oder (C₃-C₇)-Cycloalkyl bedeuten, Methyl oder Ethyl, die ihrerseits durch Cyano, Methoxycarbonyl, gegebenenfalls durch Methyl oder Ethyl substituiertes 5- oder 6-gliedriges
Heterocyclyl, 5- oder 6-gliedriges Heteroaryl, Tri-(C₁-C₂)-alkylammonium, -NR¹⁴R¹⁵, -C(=NR¹⁶)-R¹⁷, -N-C(=O)-R¹⁸ oder -N-C(=O)-NH-R¹⁹ substituiert sein können,
worin
R¹⁴ Wasserstoff, Dimethylamino, gegebenenfalls durch 5- oder 6-gliedriges Heteroaryl substituiertes Methyl oder Ethyl, 5- oder 6-gliedriges Heterocyclyl oder 5- oder 6-gliedriges Heteroaryl, das durch Halogen substituiert sein kann, bedeutet,
R¹⁵ Wasserstoff, Methyl oder Ethyl bedeutet,
R¹⁶ Wasserstoff bedeutet,
R¹⁷ Amino, 5- oder 6-gliedriges Heterocyclyl, gegebenenfalls durch Amino oder Trifluormethyl substituiertes Mono- oder Di-(C₁-C₄)-Alkylamino, gegebenenfalls durch Trifluormethyl substituiertes (C₃₋C₇)-Cycloalkylamino bedeutet,
R¹⁸ Trifluormethyl oder (C₁-C₄)-Alkyl bedeutet,
R¹⁹ Wasserstoff, Amino oder gegebenenfalls durch Methoxy- oder Ethoxycarbonyl substituiertes (C₁₋C₄)-Alkyl bedeutet,
Methoxy oder Ethoxy, die ihrerseits ein- oder zweifach, unabhängig voneinander, durch gegebenenfalls durch Methyl substituiertes 5- oder 6-gliedriges Heterocyclyl oder -C(=NR²⁰)-R²¹ substituiert sein können,
worin

R²⁰ Wasserstoff bedeutet,
R²¹ Amino, (C₃-C₆)-Cycloalkylamino, Benzylamino oder 5- oder 6-gliedriges Heterocyclyl bedeutet,
oder Pyridyl substituiert sein können,

sowie deren Salze, Hydrate, Hydrate der Salze und Solvate.

Besonders bevorzugt sind Verbindungen der Formel (I),
worin
- R¹ und R²: gemeinsam für O stehen und
- R³ und R⁴: gemeinsam für O stehen,
oder
- R¹: Wasserstoff, Hydroxy oder Methoxy bedeutet,
- R²: Wasserstoff bedeutet und
- R³ und R⁴: gemeinsam für O stehen,
oder
- R¹ und R²: gemeinsam für O stehen,
- R³: Wasserstoff, Hydroxy oder Methoxy bedeutet und
- R⁴: Wasserstoff bedeutet,
- R⁵ und R⁶: für Wasserstoff stehen und
- R⁷ und R⁸: gemeinsam für 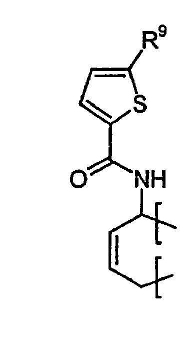
stehen,
worin
R⁹ Chlor oder Brom bedeutet,
oder
- R⁵, R⁶, R⁷ und R⁸: gemeinsam für 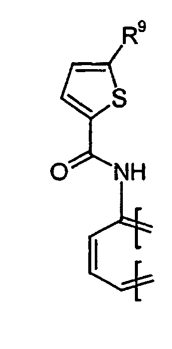
stehen,
worin
R⁹ die oben angegebene Bedeutung besitzt,
- A: Methandiyl oder Ethandiyl bedeutet,
- B: Phenylen bedeutet, das durch -C(=NR¹⁰)-R¹¹
worin
R¹⁰ Wasserstoff bedeutet,
R¹¹ Amino bedeutet, Methyl, das seinerseits durch Cyano, gegebenenfalls durch Methyl substituiertes Imidazolinyl oder Tetrahydropyrimidinyl, -NR¹⁴R¹⁵ oder -C(=NR¹⁶)-R¹⁷ substituiert sein kann,
worin

R¹⁴ gegebenenfalls durch Pyridyl substituiertes Methyl oder Pyridyl bedeutet,

R¹⁵ Wasserstoff bedeutet,
R¹⁶ Wasserstoff bedeutet,
R¹⁷ Amino, Piperidinyl, Morpholinyl, Pyrrolidinyl, gegebenenfalls durch Amino oder Trifluormethyl substituiertes Mono- oder Di-(C₁-C₃)-Alkylamino oder gegebenenfalls durch Trifluormethyl substituiertes Cyclopropyl-, Cyclopentyl- oder Cyclohexylamino bedeutet, oder Methoxy oder Ethoxy, die ihrerseits durch -C(=NH)-NH₂ substituiert sein können, substituiert sein kann,

sowie deren Salze, Hydrate, Hydrate der Salze und Solvate.

Ebenfalls besonders bevorzugt sind Verbindungen der Formel (I), worin B für Phenylen oder Cyclohexandiyl steht, dessen Substituenten sich in 3- oder 4-Position zur Anknüpfungstelle des Phenyl- oder Cyclohexanrings befinden.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen, beliebig kombiniert werden. Sie gelten für die Endprodukte sowie für die Vor- und Zwischenprodukte entsprechend.

Gegenstand der vorliegenden Erfindung ist auch ein Verfahren zur Herstellung der erfindungsgemäßen Verbindungen der Formel (I), wobei man entweder

### [A] Verbindungen der Formel (II) oder (IIa)

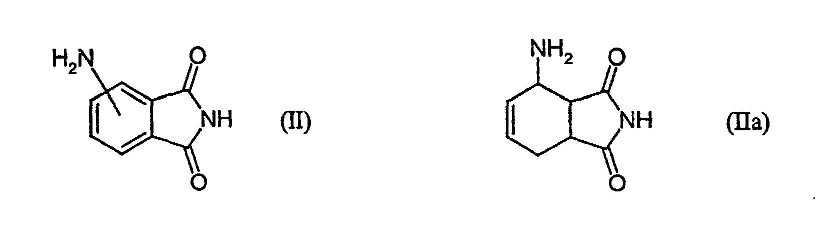

mit Verbindungen der Formel (III) 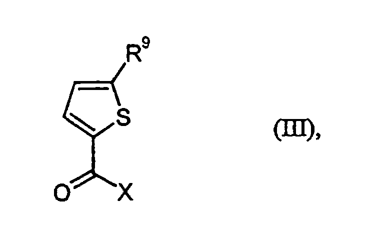
worin R⁹ die oben angegebene Bedeutung hat und X für eine Abgangsgruppe steht, in Verbindungen der Formel (IV) 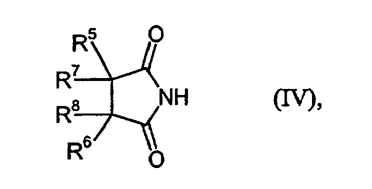
worin R⁵, R⁶, R⁷ und R⁸die oben angegebene Bedeutung besitzen, überftihrt und anschließend mit Verbindungen der Formel (V)

Y-A-B (V),

worin A und B die oben angegebene Bedeutung besitzen und Y für eine geeignete Abgangsgruppe steht,
zu Verbindungen der Formel (I) umsetzt,
worin R¹ und R², sowie R³ und R⁴, jeweils gemeinsam, für O stehen und R⁵, R⁶, R⁷, R⁸, A und B die oben angegebene Bedeutung besitzen,
oder

### [B] Verbindungen der Formel (VI) oder (VIa)

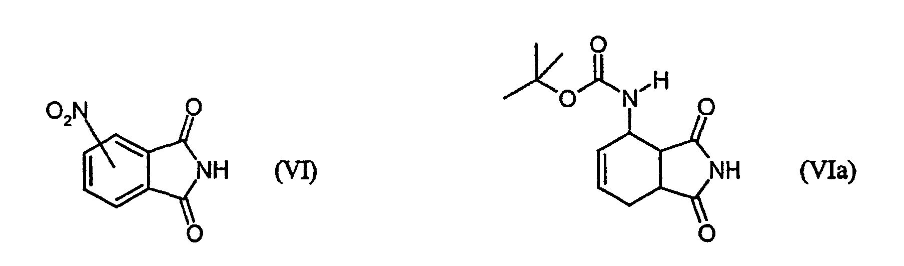

mit Verbindungen der Formel (V) zu Verbindungen der Formel (VII) oder (VIIa) 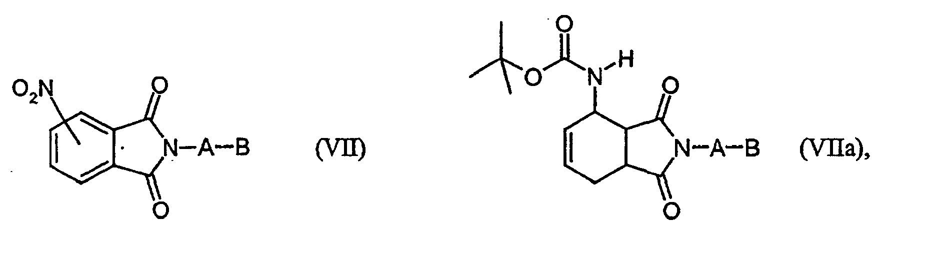
worin A und B die oben angegebene Bedeutung besitzen,
umsetzt und anschließend
entweder
[B1] Verbindungen der Formel (VII) durch Reduktion der Nitrogruppe in Verbindungen der Formel (VIII) oder Verbindungen der Formel (VIIa) durch Abspaltung der BOC-Schutzgruppe in Verbindungen der Formel (VIIIa) 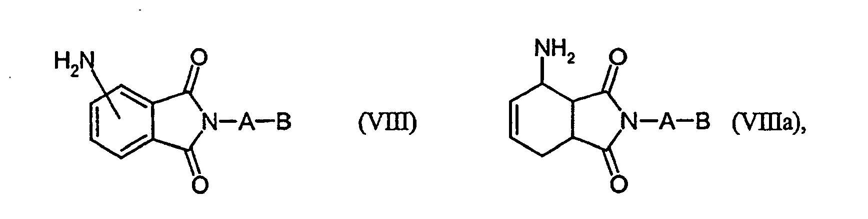
   worin A und B die oben angegebene Bedeutung besitzen,
   überführt und dann mit Verbindungen der Formel (III) zu Verbindungen der
   Formel (I) umsetzt,
   worin R¹ und R², sowie R³ und R⁴, jeweils gemeinsam, für O stehen und R⁵, R⁶, R⁷, R⁸, A und B die oben angegebene Bedeutung besitzen,
   oder
[B2] Verbindungen der Formel (VII) durch Reduktion einer Carbonylgruppe und der Nitrogruppe zu Verbindungen der Formel (IX) 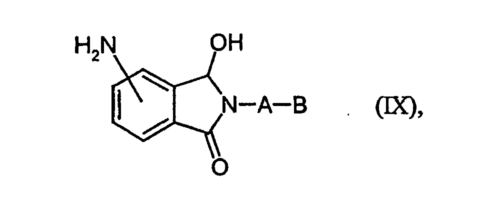
   worin A und B die oben angegebene Bedeutung besitzen,
   umsetzt, gegebenenfalls anschließend durch weitere Reduktion in Verbindungen der Formel (X) 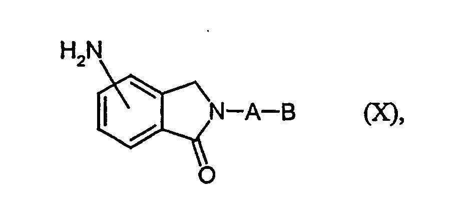
   worin A und B die oben angegebene Bedeutung besitzen,
   überführt und dann die so erhaltenen Verbindungen der Formel (IX) oder (X) mit Verbindungen der Formel (III) zu Verbindungen der Formel (I) umsetzt,
   worin R¹ und R² gemeinsam für O, R³ für Wasserstoff oder Hydroxy, R⁴ für Wasserstoff stehen oder R³ und R⁴ gemeinsam für O, R¹ für Wasserstoff oder Hydroxy, R² für Wasserstoff stehen und R⁵, R⁶, R⁷, R⁸, A und B die oben angegebene Bedeutung besitzen,
   oder
[C] Verbindungen der Formel (XI)

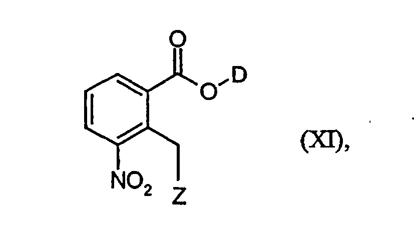

worin D für (C₁-C₄)-Alkyl und Z für eine Abgangsgruppe steht,
mit Verbindungen der Formel (XII)

H₂N-A-B (XII),

worin A und B die oben angegebene Bedeutung besitzen,
zu Verbindungen der Formel (XIII) 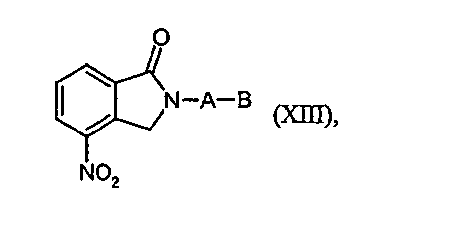
worin A und B die oben angegebene Bedeutung besitzen,
umsetzt, dann durch Reduktion der Nitrogruppe in Verbindungen der Formel (XIV) 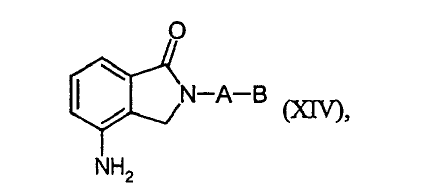
worin A und B die oben angegebene Bedeutung besitzen,
überführt und dann mit Verbindungen der Formel (III) zu Verbindungen der Formel (I) umsetzt,
worin R¹ und R² für Wasserstoff, R³ und R⁴ gemeinsam für O stehen und R⁵, R⁶, R⁷, R⁸, A und B die oben angegebene Bedeutung besitzen,
wobei sich bei den so erhaltenen Verbindungen der Formel (I) gegebenenfalls weitere Derivatisierungen, die nach üblichen Methoden durchgeführt werden können, anschließen können.

Das erfindungsgemäße Verfahren kann durch folgendes Formelschema beispielhaft erläutert werden:

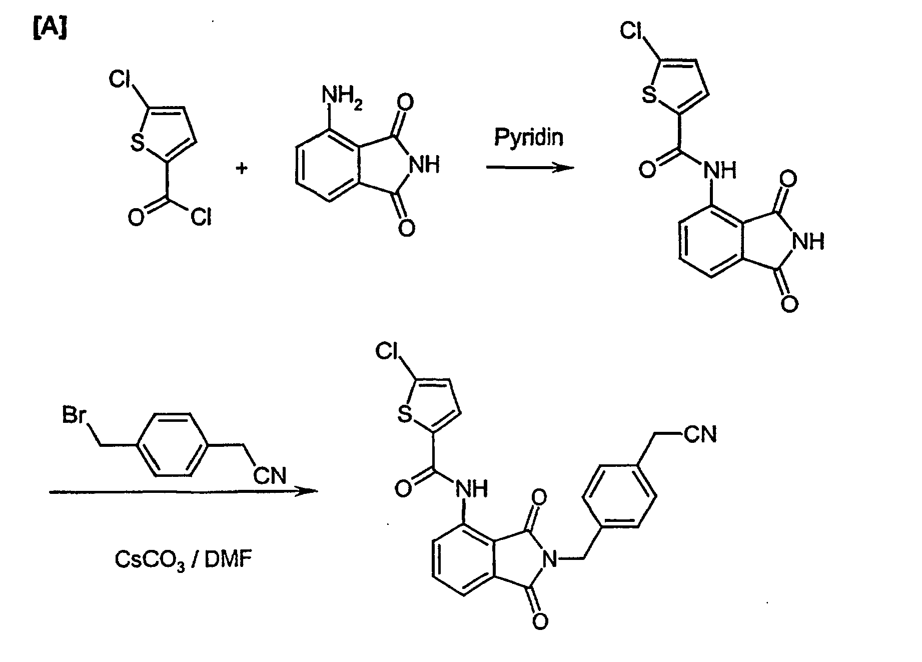

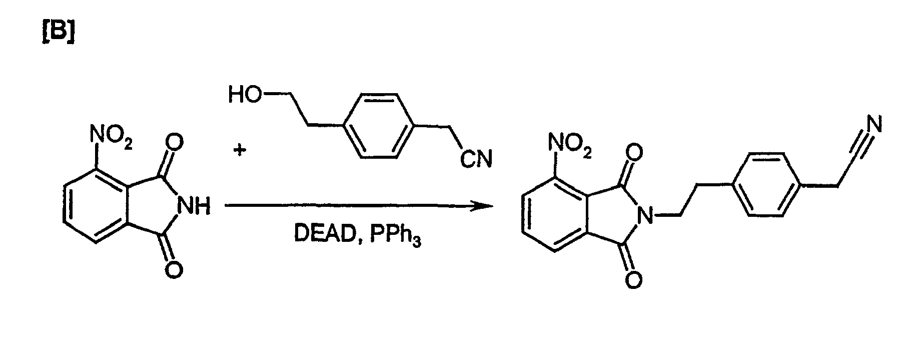

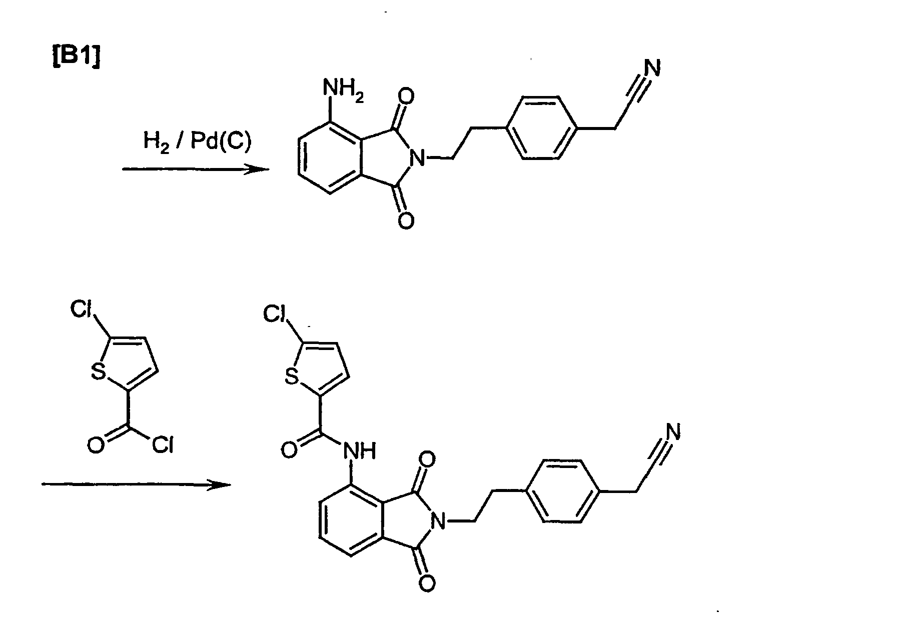

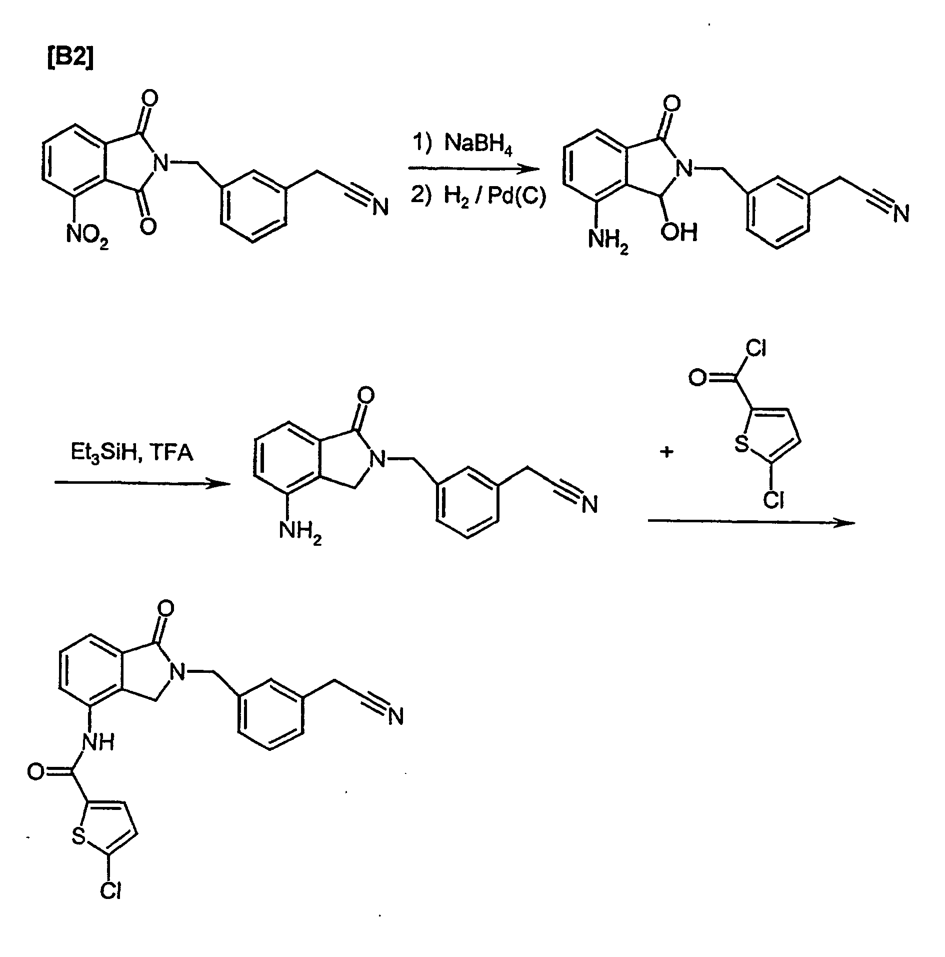

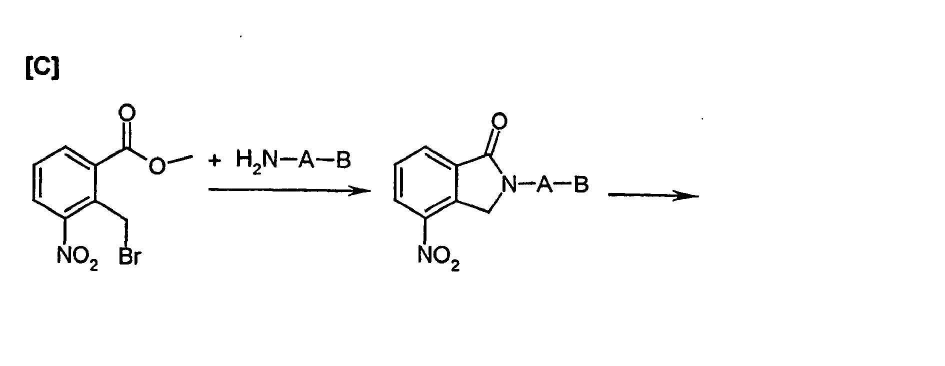

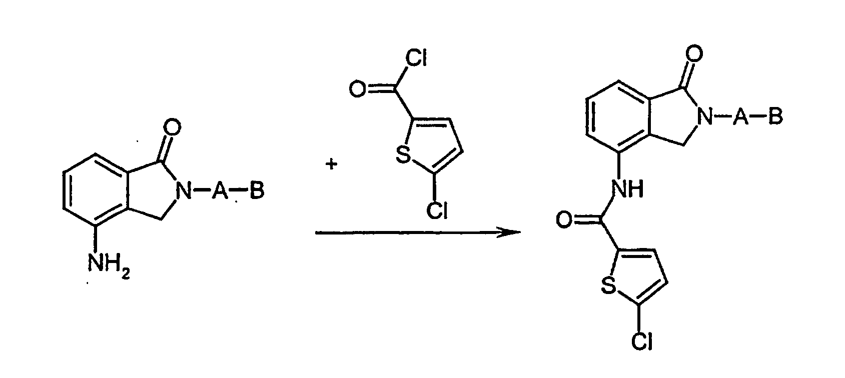

Als Lösemittel für das erfindungsgemäße Verfahren eignen sich alle organischen Lösemittel, die unter den Reaktionsbedingungen inert sind, oder Wasser. Hierzu gehören Alkohole wie Methanol, Ethanol und Isopropanol, Ketone wie Aceton und Methylethylketon, acyclische und cyclische Ether wie Diethylether, Tetrahydrofuran und Dioxan, Ester wie Essigsäureethylester oder Essigsäurebutylester, Kohlenwasserstoffe wie Benzol, Xylol, Toluol, Hexan oder Cyclohexan, Dimethylformamid, Acetonitril, Pyridin, Dimethylsulfoxid (DMSO), chlorierte Kohlenwasserstoffe wie Dichlormethan, Chlorbenzol oder Dichlorethan. Ebenso ist es möglich, Gemische der zuvor genannten Lösemittel einzusetzen.

Als Basen eignen sich die üblichen anorganischen oder organischen Basen. Hierzu gehören bevorzugt Alkalihydroxide wie beispielsweise Natrium- oder Kaliumhydroxid oder Alkalicarbonate wie Natrium- oder Kaliumcarbonat oder Natrium-oder Kaliumhydrogencarbonat oder Natrium- oder Kaliummethanolat oder Natrium-oder Kaliumethanolat oder Kalium-tert.-butylat oder aber Amide wie Natriumamid, Lithium-bis-(trimethylsilyl)amid oder Lithiumdiisopropylamid oder metallorganische Verbindungen wie Butyllithium oder Phenyllithium oder aber auch Amine wie Trialkylamine z.B. Triethylamin, N-Methylmorpholin (NMM), N-Methylpiperidin, Diisopropylethylamin (Hünigbase) oder 4*-N,N* Dimethylaminopyridin (DMAP) oder Pyridin.

Das erfindungsgemäße Verfahren kann bei normalem, erhöhtem oder erniedrigtem Druck durchgeführt werden (z.B. im Bereich von 0,5 bis 5 bar). Im allgemeinen arbeitet man bei Normaldruck.

Die Umsetzung mit Thiophencarbonsäurederivaten der Formel (III) wie im ersten Verfahrensschritt [A]: (II) / (IIa) + (III) → (IV); zweiten Verfahrensschritt [B1]: (VIII) / (VIIIa) + (III) → (I); dritten Verfahrensschritt [B2]: (IX) / (X) + (III) → (I) und dritten Verfahrensschritt [C]: (XIV) + (III) → (I) erfolgt im allgemeinen in einem Temperaturbereich von -78°C bis +120°C, bevorzugt im Bereich von -78°C bis +60°C, insbesondere bei 0°C bis +50°C. Als Abgangsgruppe X werden beispielsweise Halogene eingesetzt, also das entsprechende Säurechlorid oder -bromid, oder es werden die entsprechenden Säureanhydride eingesetzt. Bevorzugt ist das entsprechende Säurechlorid. Als Lösemittel bevorzugt ist Pyridin oder Tetrahydrofuran. Gegebenenfalls wird als Base 4-Dimethylaminopyridin (4-DMAP) oder Triethylamin zugegeben.

Im zweiten Verfahrensschritt [A]: (IV) + (V) → (I) und ersten Verfahrensschritt [B]: (VI) / (VIa) + (V) → (VII) / (VIIa) erfolgt eine Derivatisierung von Phthalimiden in einer nukleophilen Substitutionsreaktion mit Verbindungen der Formel (V). Als Abgangsgruppe Y in Verbindungen der Formel (V) kommen beispielsweise in Frage: Halogen, Tosylat, Mesylat oder eine durch Reagenzien wie Diethylazodicarboxylat (DEAD)/PPh₃ aktivierte Hydroxyfunktion (Mitsunobu-Reaktion). Als Lösemittel bevorzugt ist im Fall einer Reaktion unter Mitsunobu-Bedingungen Tetrahydrofuran, die Umsetzung mit Alkylhalogeniden erfolgt bevorzugt in Dimethylformamid als Lösungsmittel in Gegenwart einer Base wie beispielsweise Kaliumcarbonat. Die Umsetzung erfolgt im allgemeinen in einem Temperaturbereich von -78°C bis +120°C, bevorzugt bei 0°C bis Raumtemperatur.

Im ersten Verfahrensschritt [B1]: (VII) → (VIII), im zweiten Teil des ersten Verfahrensschrittes [B2]: (VII) → (IX) und im zweiten Verfahrensschritt [C]: (XIII) → (XIV) wird eine aromatische Nitrogruppe in das entsprechende Amin überführt.

Als Lösemittel bevorzugt sind Methanol, Ethanol, Tetrahydrofuran, und Essigsäureethylester oder Gemische dieser Lösungsmittel. Die Umsetzung erfolgt im allgemeinen in einem Temperaturbereich von -78°C bis +120°C, bevorzugt bei Raumtemperatur. Geeignet sind herkömmliche Hydrierverfahren, bevorzugt ist die Hydrierung mit Wasserstoff bei Atmosphärendruck in Gegenwart eines Katalysators wie beispielsweise Palladium auf Kohle [Pd(C); 10 Gew.-%].

Im ersten Verfahrensschritt [B1]: (VIIa) → (VIIIa) wird durch Abspaltung der BOC-Schutzgruppe unter dafür üblichen Reaktionsbedingungen in Gegenwart einer Säure die Aminogruppe freigesetzt. Bevorzugte Lösungsmittel-/Säuresysteme sind Dichlormethan/Trifluoressigsäure oder Dioxan/Salzsäure. Die Umsetzung erfolgt im allgemeinen in einem Temperaturbereich von -78°C bis +120°C, bevorzugt bei 0°C bis Raumtemperatur.

Im ersten Teil des ersten Verfahrensschrittes [B2]: (VII) → (IX) wird eine Carbonylfunktion des Phthalimidsystems zur entsprechenden Hydroxyfunktion reduziert. Es entsteht in der Regel ein Isomerengemisch, das chromatographisch getrennt werden kann. Als Lösemittel bevorzugt ist ein Gemisch aus Methanol und Dichlormethan. Geeignet sind herkömmliche Hydrierverfahren, vorzugsweise wird Natriumborhydrid als Reduktionsmittel verwendet.

Im gegebenenfalls erfolgenden zweiten Verfahrensschritt [B2]: (IX) → (X) wird eine Hydroxygruppe durch Reduktion in den entsprechenden Kohlenwasserstoff überführt. Die Umsetzung erfolgt im allgemeinen in einem Temperaturbereich von -78°C bis +120°C, bevorzugt bei Raumtemperatur. Als Lösemittel bevorzugt ist Dichlormethan. Geeignet sind herkömmliche Hydrierverfahren, vorzugsweise das System Trifluoressigsäure (TFA)/Triethylsilan.

Die Alkylierung im ersten Verfahrensschritt [C]: (XI) + (XII) →(XIII) erfolgt im allgemeinen in einem Temperaturbereich von -78°C bis +120°C, bevorzugt bei +50°C bis +80°C. Als Abgangsgruppe Z in Verbindungen der Formel (XI) kommen beispielsweise in Frage: Halogen, Tosylat oder Mesylat, bevorzugt ist Brom. Als Lösemittel bevorzugt ist Dimethylformamid, als zusätzliche Base wird beispielsweise Triethylamin eingesetzt.

Die Verbindungen der Formeln (II), (IIa), (III), (V), (VI), (VIa), (XI) und (XII) sind kommerziell erhältlich, literaturbekannt oder nach üblichen literaturbekannten Methoden herstellbar.

Die erfindungsgemäßen Verbindungen der Formel (I) zeigen ein nicht vorhersehbares, wertvolles pharmakologisches Wirkspektrum und sind daher insbesondere zur Prävention und/oder Behandlung von Erkrankungen geeignet.

Die erfindungsgemäßen Verbindungen der Formel (I) sind auch in Kombination mit einem oder mehreren anderen Wirkstoffen zur Prävention und/oder Behandlung von Erkrankungen geeignet. Geeignete Kombinationswirkstoffe sind insbesondere Plättchenaggregationshemmer, Antikoagulantien, Fibrinolytika, Lipidsenker, Koronartherapeutika und/oder Vasodilatatoren.

Die erfindungsgemäßen Verbindungen der Formel (I) wirken insbesondere als Antikoagulantien und können daher bevorzugt eingesetzt werden in Arzneimitteln zur Prävention und/oder Behandlung von thromboembolischen Erkrankungen. Zu den "thromboembolischen Erkrankungen" im Sinne der vorliegenden Erfindung zählen insbesondere schwerwiegende Erkrankungen wie Herzinfarkt, Angina Pectoris (eingeschlossen instabile Angina), Reokklusionen und Restenosen nach einer Angioplastie oder aortokoronarem Bypass, Hirnschlag, transitorische ischämische Attacken, periphere arterielle Verschlusskrankheiten, Lungenembolien oder tiefe venöse Thrombosen.

Darüber hinaus sind die erfindungsgemäßen Verbindungen der Formel (I) gleichermaßen zur Behandlung der disseminierten intravasalen Gerinnung (DIC) geeignet.

Schließlich kommen die erfindungsgemäßen Verbindungen der Formel (I) ebenso für die Prävention und/oder Behandlung von Atherosklerose und Arthritis in Betracht, darüber hinaus ebenso für die Prävention und/oder Behandlung der Alzheimer'schen Erkrankung und von Krebs.

Weiterhin umfasst die vorliegende Erfindung auch ein Verfahren zur Verhinderung der Blutkoagulation in vitro, insbesondere bei Blutkonserven oder biologischen Proben, die Faktor Xa enthalten, das dadurch gekennzeichnet ist, dass Verbindungen der Formel (I) zugegeben werden.

Die erfindungsgemäßen Verbindungen der Formel (I) wirken insbesondere als selektive Inhibitoren des Blutgerinnungsfaktors Xa und hemmen nicht oder erst bei deutlich höheren Konzentrationen auch andere Serinproteasen wie Thrombin, Plasmin oder Trypsin.

Als "selektiv" werden im Rahmen der vorliegenden Erfindung solche Inhibitoren des Blutgerinnungsfaktors Xa bezeichnet, bei denen die IC₅₀-Werte für die Faktor Xa-Inhibierung gegenüber den IC₅₀-Werten für die Inhibierung anderer Serinproteasen, insbesondere Thrombin, Plasmin und Trypsin, um das 100-fache, vorzugsweise um das 500-fache, insbesondere um das 1.000-fache, kleiner sind, wobei bezüglich der Testmethoden für die Selektivität Bezug genommen wird auf die im folgenden beschriebenen Testmethoden der Beispiele A-1) a.1) und a.2).

Für die Applikation der erfindungsgemäßen Verbindungen kommen alle üblichen Applikationsformen in Betracht. Vorzugsweise erfolgt die Applikation oral, lingual, sublingual, bukkal, rektal, lokal wie beispielsweise bei Implantaten oder Stents, oder parenteral (d.h. unter Umgehung des Intestinaltraktes, also intravenös, intraarteriell, intrakardial, intrakutan, subkutan, transdermal, intraperitoneal oder intramuskulär). Insbesondere geeignet sind die orale und intravenöse Applikation. Ganz besonders bevorzugt ist die orale Applikation, worin ein weiterer Vorteil gegenüber der aus dem Stand der Technik bekannten Therapie von thromboembolischen Erkrankungen liegt.

Die neuen Wirkstoffe der Formel (I) können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,1 bis 95 Gew.-%, bevorzugt in 0,5 bis 90 Gew.-%, insbesondere von 1 bis 85 Gew.-%, der Gesamtmischung vorhanden sein, d.h. in Mengen, die ausreichend sind, um den angegebenen Dosierungsspielraum zu erreichen.

Trotzdem kann es gegebenenfalls erforderlich sein, von den zuvor genannten Mengen abzuweichen, und zwar in Abhängigkeit vom Körpergewicht bzw. von der Art des Applikationsweges, vom individuellen Verhalten gegenüber dem Medikament, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Im allgemeinen hat es sich als vorteilhaft erwiesen, bei intravenöser Applikation Mengen von etwa 0,001 bis 10 mg/kg, vorzugsweise etwa 0,01 bis 10 mg/kg, insbesondere etwa 0,1 bis 8 mg/kg Körpergewicht, zur Erzielung wirksamer Ergebnisse zu verabreichen.

Im allgemeinen hat es sich als vorteilhaft erwiesen, bei oraler Applikation Mengen von etwa 0,01 bis 50 mg/kg, vorzugsweise etwa 0,1 bis 10 mg/kg, insbesondere etwa 0,5 bis 8 mg/kg Körpergewicht, zur Erzielung wirksamer Ergebnisse zu verabreichen.

Trotzdem kann es gegebenenfalls erforderlich sein, von den zuvor genannten Mengen bei intravenöser bzw. oraler Applikation abzuweichen, und zwar in Abhängigkeit vom Körpergewicht bzw. von der Art des Applikationsweges, vom individuellen Verhalten gegenüber dem Medikament, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese über den Tag zu verteilen, und zwar entweder in mehreren Einzelgaben oder als Dauerinfusion.

Die erfindungsgemäßen Verbindungen der Formel (I) zeichnen sich gegenüber herkömmlichen Präparaten zur Behandlung von thromboembolischen Erkrankungen insbesondere dadurch aus, dass durch die selektive Hemmung des Faktors Xa eine größere therapeutische Breite erreicht wird. Dies bedeutet für den Patienten ein geringeres Blutungsrisiko und für den behandelnden Arzt eine bessere Einstellbarkeit des Patienten. Außerdem erfolgt - durch den Mechanismus bedingt - ein schneller Wirkeintritt. Vor allem aber erlauben die erfindungsgemäßen Verbindungen eine orale Applikationsform, worin ein weiterer Vorteil der Therapie mit den erfindungsgemäßen Verbindungen liegt.

Die vorliegende Erfindung wird an den folgenden Beispielen veranschaulicht.

### A Bewertung der physiologischen Wirksamkeit

### 1. Allgemeine Testmethoden

Die besonders vorteilhaften biologischen Eigenschaften der erfindungsgemäßen Verbindungen können durch folgende Methoden festgestellt werden.

### a) Testbeschreibung (in vitro)

### a.1) Messung der Faktor Xa-Hemmung

Die enzymatische Aktivität von humanem Faktor Xa (FXa) wurde über die Umsetzung eines für den FXa-spezifischen chromogenen Substrats gemessen. Dabei spaltet der Faktor Xa aus dem chromogenen Substrat p-Nitroanilin ab. Die Bestimmungen wurden wie folgt in Mikrotiterplatten durchgeführt.

Die Prüfsubstanzen wurden in unterschiedlichen Konzentrationen in DMSO gelöst und für 10 Minuten mit humanem FXa (0,5 nmol/l gelöst in 50 mmol/l Tris-Puffer [C,C,C-Tris(hydroxymethyl)-aminomethan], 150 mmol/l NaCl, 0,1 % BSA (bovine serum albumine), pH = 8,3) bei 25°C inkubiert. Als Kontrolle dient reines DMSO. Anschließend wurde das chromogene Substrat (150 µmol/l Pefachrome® FXa von der Firma Pentapharm) hinzugefügt. Nach 20 Minuten Inkubationsdauer bei 25°C wurde die Extinktion bei 405 nm bestimmt. Die Extinktionen der Testansätze mit Prüfsubstanz wurden mit den Kontrollansätzen ohne Prüfsubstanz verglichen und daraus die IC₅₀-Werte berechnet.

### a.2) Bestimmung der Selektivität

Zum Nachweis der selektiven FXa-Inhibition wurden die Prüfsubstanzen auf ihre Hemmung anderer humaner Serinproteasen wie Thrombin, Trypsin, Plasmin hin untersucht. Zur Bestimmung der enzymatischen Aktivität von Thrombin (75 mU/ml), Trypsin (500 mU/ml) und Plasmin (3,2 nmol/l) wurden diese Enzyme in Tris-Puffer (100 mmol/l, 20 mmol/l CaCl₂,pH = 8,0) gelöst und für 10 Minuten mit Prüfsubstanz oder Lösungsmittel inkubiert. Anschließend wurde durch Zugabe der entsprechenden spezifischen chromogenen Substrate (Chromozym Thrombin® von der Firma Boehringer Mannheim, Chromozym Trypsin® von der Firma Boehringer Mannheim, Chromozym Plasmin® von der Firma Boehringer Mannheim) die enzymatische Reaktion gestartet und die Extinktion nach 20 Minuten bei 405 nm bestimmt. Alle Bestimmungen wurden bei 37°C durchgeführt. Die Extinktionen der Testansätze mit Prüfsubstanz wurden mit den Kontrollproben ohne Prüfsubstanz verglichen und daraus die IC₅₀-Werte berechnet.

### a.3) Bestimmung der antikoagulatorischen Wirkung

Die antikoagulatorische Wirkung der Prüfsubstanzen wurde in vitro in Humanplasma bestimmt. Dazu wurde Humanblut unter Verwendung einer 0,11 molaren Natriumcitrat-Lösung als Vorlage in einem Mischungsverhältnis Natriumcitrat/Blut 1/9 abgenommen. Das Blut wurde unmittelbar nach der Abnahme gut gemischt und 10 Minuten bei ca. 2000 g zentrifugiert. Der Überstand wurde abpipettiert. Die Prothrombinzeit (PT, Synonyme: Thromboplastinzeit, Quick-Test) wurde in Gegenwart variierender Konzentrationen an Prüfsubstanz oder dem entsprechenden Lösungsmittel mit einem handelsüblichen Testkit (Neoplastin® von der Firma Boehringer Mannheim) bestimmt. Die Testverbindungen wurden 10 Minuten bei 37°C mit dem Plasma inkubiert. Anschließend wurde durch Zugabe von Thromboplastin die Gerinnung ausgelöst und der Zeitpunkt des Gerinnungseintritts bestimmt. Es wurde die Konzentration an Prüfsubstanz ermittelt, die eine Verdoppelung der Prothrombinzeit bewirkt.

### b) Bestimmung der antithrombotischen Wirkung (in vivo)

### b.1) Arteriovenöses Shunt Modell (Ratte)

Nüchterne männliche Ratten (Stamm: HSD CPB:WU) mit einem Gewicht von 200 bis 250 g wurden mit einer Rompun/ Ketavet Lösung narkotisiert (12 mg/kg/50 mg/kg). Die Thrombusbildung wurde in einem arteriovenösen Shunt in Anlehnung an die von Christopher N. Berry et al., Br. J. Pharmacol. (1994), 113, 1209 bis 1214 beschriebene Methode ausgelöst. Dazu wurden die linke Vena jugularis und die rechte Arteria carotis freipräpariert. Ein extracorporaler Shunt wurde mittels eines 10 cm langen Polyethylenschlauchs (PE 60) zwischen den beiden Gefäßen gelegt. Dieser Polyethylenschlauch war in der Mitte in einen weiteren 3 cm langen Polyethylenschlauch (PE 160), der zur Erzeugung einer thrombogenen Oberfläche einen aufgerauhten und zu einer Schlinge gelegten Nylonfaden enthielt, eingebunden. Der extrakorporale Kreislauf wurde 15 Minuten lang aufrechterhalten. Dann wurde der Shunt entfernt und der Nylonfaden mit dem Thrombus sofort gewogen. Das Leergewicht des Nylonfadens war vor Versuchsbeginn ermittelt worden. Die Prüfsubstanzen wurden vor Anlegung des extrakorporalen Kreislaufs entweder intravenös über die Schwanzvene oder oral mittels Schlundsonde wachen Tieren verabreicht.

### b.2) Arterielles Thrombose-Modell (Ratte)

Männliche nüchterne Ratten (Stamm: HSD CPB: WU) wurden wie oben beschrieben narkotisiert. Die Ratten waren im Mittel etwa 200 g schwer. Die linke Arteria carotis wurde freipräpariert (ca. 2 cm). Die Bildung eines arteriellen Thrombus wurde durch eine mechanische Gefäßverletzung in Anlehnung an die von K. Meng et al., Naunyn-Schmiedeberg's Arch. Pharmacol. (1977), 301, 115-119 beschriebene Methode induziert. Dazu wurde die freipräparierte Arteria carotis vom Blutfluss abgeklemmt, für 2 Minuten in einer Metallrinne auf -12°C abgekühlt und zur Standardisierung der Thrombengröße gleichzeitig mit einem Gewicht von 200 g komprimiert. Anschließend wurde der Blutfluss durch einen um die Arteria carotis distal von dem verletzten Gefäßabschnitt gelegten Clip zusätzlich reduziert. Die proximale Klemme wurde entfernt, die Wunde verschlossen und nach 4 Stunden wieder geöffnet, um den verletzten Gefäßabschnitt zu entnehmen. Der Gefäßabschnitt wurde longitudinal geöffnet und der Thrombus von dem verletzten Gefäßabschnitt entfernt. Das Feuchtgewicht der Thromben wurde sofort ermittelt. Die Prüfsubstanzen wurden zu Versuchsbeginn entweder intravenös über die Schwanzvene oder oral mittels Schlundsonde wachen Tieren verabreicht.

### b.3) Venöses Thrombose-Modell (Ratte)

Männliche nüchterne Ratten (Stamm: HSD CPB: WU) wurden wie oben beschrieben narkotisiert. Die Ratten waren im Mittel etwa 200 g schwer. Die linke Vena jugularis wurde freipräpariert (ca. 2 cm). Die Bildung eines venösen Thrombus wurde durch eine mechanische Gefäßverletzung in Anlehnung an die von K. Meng et al., Naunyn-Schmiedeberg's Arch. Pharmacol. (1977), 301, 115-119 beschriebene Methode induziert. Dazu wurde die Vena jugularis vom Blutfluss abgeklemmt, für 2 Minuten in einer Metallrinne auf -12°C abgekühlt und zur Standardisierung der Thrombengröße gleichzeitig mit einem Gewicht von 200 g komprimiert. Der Blutfluss wurde wieder eröffnet und die Wunde verschlossen. Nach 4 Stunden wurde die Wunde wieder geöffnet, um die Thromben von den verletzten Gefäßabschnitten zu entfernen. Das Feuchtgewicht der Thromben wurde sofort ermittelt. Die Prüfsubstanzen wurden zu Versuchsbeginn entweder intravenös über die Schwanzvene oder oral mittels Schlundsonde wachen Tieren verabreicht.

### B Herstellungbeispiele

In den Beispielen werden die folgenden Abkürzungen verwendet:
DMF = Dimethylformamid
THF = Tetrahydrofuran

### HPLC-Parameter:

**Methode 1**: Säule: Kromasil C18; L-R Temperatur: 30°C; Fluss = 0.75 mlmin⁻¹;
   Eluent: A = 0.01 M HClO₄, B = CH₃CN, Gradient: → 0.5 min 98%A, → 4.5 min
   10%A, → 6.5 min 10%A.
**Methode 2:** Säule: Kromasil C18 60*2; L-R Temperatur: 30°C; Fluss = 0.75 mlmin⁻¹
   ; Eluent: A = 0.01 M H₃PO₄, B = CH₃CN, Gradient: → 0.5 min 90%A, → 4.5 min
   10%A, → 6.5 min 10%A.
**Methode 3:** Säule: Kromasil C18 60*2; L-R Temperatur: 30°C; Fluss = 0.75 mlmin⁻¹ ; Eluent: A = 0.005 M HClO₄, B = CH₃CN, Gradient: → 0.5 min 98%A, → 4.5 min
   10%A, → 6.5 min 10%A.
**Methode 4:** Säule: Symmetry C18, 2.1 mm x 150 mm; Säulenofen: 50°C; Fluss = 0.6 mlmin⁻¹; Eluent: A = 0.6 g 30%ige HCl/ 1 Wasser, B = CH₃CN, Gradient: 0.0 min 90%A, → 4.0 min 10%A, → 9 min 10%A.
**Methode 5:** Instrument Micromass Quattro LCZ
   Säule Symmetry C18, 50 mm x 2.1 mm, 3.5 µm; Temperatur: 40°C; Fluss = 0.5 mlmin⁻¹; Eluent A = CH₃CN + 0.1% Ameisensäure, Eluent B = Wasser + 0.1% Ameisensäure, Gradient: 0.0 min 10%A, → 4 min 90%A, → 6 min 90%A.
**Methode 6**: Instrument Micromass Platform LCZ
   Säule: Symmetry C18, 50 mm x 2.1 mm, 3.5 µm; Temperatur: 40°C; Fluss = 0.5 mlmin⁻¹; Eluent A = CH₃CN + 0.1% Ameisensäure, Eluent B = Wasser + 0.1% Ameisensäure, Gradient: 0.0 min 10%A,→ 4 min 90%A, → 6 min 90%A.
**Methode 7:** Instrument Micromass Quattro LCZ
   Säule: Symmetry C18, 50 mm x 2.1 mm, 3.5 µm; Temperatur: 40°C; Fluss = 0.5 mlmin⁻¹; Eluent A = CH₃CN + 0.1% Ameisensäure, Eluent B = Wasser + 0.1% Ameisensäure, Gradient: 0.0 min 5%A, → 1 min 5%A, → 5 min 90%A, → 6 min 90%A.
**Methode 8**: Instrument Micromass Platform LCZ
   Säule: Symmetry C18, 150 mm x 2.1 mm, 5 µm; Temperatur. 40°C; Fluss = 0.5 mlmin⁻¹; Eluent A = CH₃CN + 0.1% Ameisensäure, Eluent B = Wasser + 0.1% Ameisensäure, Gradient: 0.0 min 10%A, → 9 min 90%A, → 10 min 90%A.

### Synthesebeispiele

### Beispiel 1

### 5-Chlor-N-(1,3-dioxo-2,3-dihydro-1H-isoindol-4-yl)-2-thiophenearboxamid

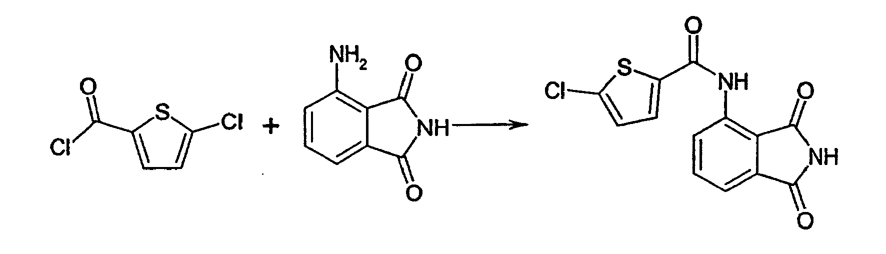

Man löst 7.8 g (43.7 mmol) 3-Aminophthalsäureimid bei 0°C unter Rühren in 200 ml Pyridin. Anschließend gibt man innerhalb von 5 min 5-Chlor-2-thiophencarbonsäurechlorid (erhalten durch Kochen von 5-Chlor-2-thiophencarbonsäure in SOCl₂) hinzu. Man rührt bei Raumtemperatur unter DC-Kontrolle nach, wobei aus der zunächst klaren Lösung ein Brei wird. Man gibt nochmals 200 ml THF hinzu und rührt über Nacht bei Raumtemperatur.

Anschließend wird der Ansatz in 500 ml Wasser gegeben und mit Essigester extrahiert. In der wässrigen Phase verbleiben noch größere Mengen an schwerlöslichen Kristallen, die abgesaugt werden. Man löst die Kristalle in 200 ml THF und trocknet mit Magnesiumsulfat. Die einrotierte Lösung wird nochmals mit Ether verrieben und abgesaugt. Es werden 3.5 g (26.4% d. Th.) der gewünschten Verbindung erhalten.

### Beispiel 2

### 2-[3-(Hydroxymethyl)phenoxy]acetonitril

Man löst 25.3 g (203.8 mmol) 3-Hydroxybenzylalkohol in 50 ml Dimethylformamid, gibt 400 ml Aceton und 28.17 g (203.8 mmol) fein zerriebenes Kaliumcarbonat hinzu. Nach 5-minütigem Rühren gibt man 24.45 g (203.8 mmol) Bromacetonitril hinzu und kocht 72 Stunden unter Rückfluss. Man filtriert und engt das Filtrat im Vakuum ein. Der Rückstand wird in Essigester gelöst, mit 10%iger wässriger Natronlauge und dann mit Wasser gewaschen. Die organische Phase wird mit Magnesiumsulfat getrocknet und über eine kurze Kieselgelsäule filtriert (Eluens Toluol -> Toluol/Essigester = 1:1). Nach Eindampfen erhält man 163.18 g (86% d.Th.) eines Öls. R_{f} (SiO₂, Toluol/Essigester =1:1) = 0.41.
Analog wurden folgende Verbindungen erhalten:

### Beispiel 3

### 2-[4-(Hydroxymethyl)phenoxy]acetonitril

R_{f} (SiO₂, Toluol/Essigester = 1: 1) = 0.5; Ausb. 34.1%.

### Beispiel 4

### 2-[3-(Hydroxymethyl)phenoxy]propannitril

R_{f} (SiO₂, Toluol/Essigester = 1:1) = 0.43; Ausb. 36.5%.

### Beispiel 5

### 5-Chlor-N-{2-[3-(cyanomethoxy)benzyl]-1,3-dioxo-2,3-dihydro-1H-isoindol-4-yl}-2-thiophencarboxamid

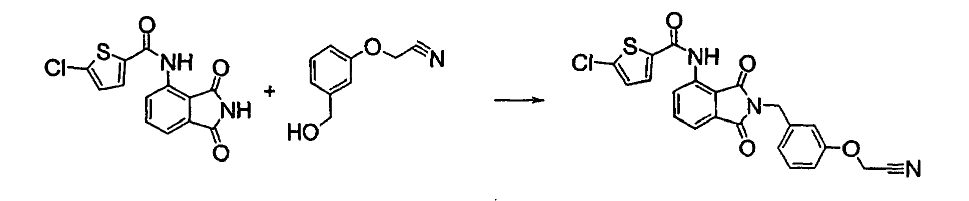

Man löst 1.46 g (8.94 mmol) 2-[3-(Hydroxymethyl)phenoxy]acetonitril, 2.5 g (8.15 mmol) 5-Chlor-*N*-(1,3-dioxo-2,3-dihydro-1*H*-isoindol-4-yl)-2-thiophencarboxamid und 4.28 g (16.3 mmol) Triphenylphosphin unter Argon in 250 ml Tetrahydrofuran. Man gibt 2.84 g (16.3 mmol) DEAD (Diethylazodicarboxylat) hinzu und rührt bei Raumtemperatur über Nacht. Man gibt 10g Kieselgel hinzu, engt im Vakuum ein und chromatographiert über eine Kieselgelsäule (Gradient: Toluol -> Toluol/Essigester = 4:1). Man erhält 2.37 g (64.4% d.Th.) der Zielverbindung mit einem Schmelzpunkt von 164°C. R_{f} (SiO₂, Toluol/Essigester = 4:1): 0.45.
Analog wurden erhalten:

### Beispiel 6

### 5-Chlor-N-{2-[4-(cyanomethoxy)benzyl]-1,3-dioxo-2,3-dihydro-1H-isoindol-4-yl}-2-thiophencarboxamid

Ausbeute 32%; Smp. 199°C; R_{f} (SiO₂, Toluol/Essigester=4:1)=0.66.

### Beispiel 7

### 5-Chlor-N-{2-[3-(1-cyanoethoxy)benzyl]-1,3-dioxo-2,3-dihydro-1H-isoindol-4-yl}-2-thiophencarboxamid

Ausb. 70.2%; Smp. 88°C; R_{f} (SiO₂, Toluol/Essigester 4:1)=0.74.

### Beispiel 8

### 2-{4-[(4-Nitro-1,3-dioxo-1,3-dihydro-2H-isoindol-2-yl)methyl]phenyl}acetonitril

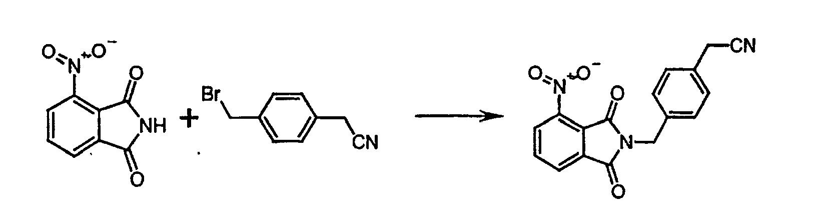

9.79 g (50.93 mmol) 3-Nitrophthalimid werden in 450 ml DMF gelöst und mit 10.56 g (76.39 mmol) Kaliumcarbonat versetzt. Die Suspension wird 10 min lang mit Ultraschall behandelt. Dann werden 10.7 g (50.93 mmol) 2-[4-(Brommethyl)-phenyl]acetonitril (erhalten durch Umsetzung von 2-(4-Methylphenyl)acetonitril mit *N*-Bromsuccinimid (NBS) und Azobisisobutyronitril (AIBN) in Tetrachlormethan, vgl. E. Laurent, B. Marquet, R. Tardivel; *Tetrahedron; 47;* 24; 1991; S. 3969-3980) zugegeben, und das Reaktionsgemisch wird 20 h lang bei Raumtemperatur gerührt. Anschließend wird die Suspension auf ein Gemisch von 300 ml 2 N Salzsäure und 3 Wasser gegossen. Die entstehenden Kristalle werden abgesaugt, mit Wasser gewaschen und im Vakuum getrocknet. Es werden 12 g (73% d. Th.) der gewünschten Verbindung erhalten. R_{f} (SiO₂, CH₂Cl₂/EtOH =100/1) = 0.55, MS = 322.2 (M+H⁺).
Analog wurden dargestellt:

### Beispiel 9

Aus 3-Nitrophthalimid und 2-[3-(Brommethyl)phenyl]acetonitril (erhalten durch Umsetzung von 2-(3-Methylphenyl)acetonitril mit NBS und AIBN in Tetrachlormethan):

### {3-[(4-Nitro-1,3-dioxo-1,3-dihydro-2H-isoindol-2-yl)methyl]phenyl}acetonitril

MS = 322 (M+H), rt (Methode 4) = 3.99 min.

### Beispiel 10

Aus 3-Nitrophthalimid und 4-Brombutymnitril:

### 4-(4-Nitro-1,3-diozo-1,3-dihydro-2H-isoindol-2-yl)butannitril

MS = 260 (M+H), rt (Methode 4) = 3.36 min.

### Beispiel 11

Aus 3-Nitrophthalimid und 5-Bromvaleronitril:

### 5-(4-Nitro-1,3-diozo-1,3-dihydro-2H-isoindol-2-yl)pentannitril

MS = 274 (M+H), rt (Methode 4) = 3.59 min.

### Beispiel 12

Aus 3-Nitrophthalimid und 4-(2-Bromethyl)benzonitril (Darstellung aus 4-(2-Hydroxyethyl)benzonitril s. G. Wagner, H. Vieweg, *Pharmazie; 37;* 1; 1982; S. 13-16):

### 4-[2-(4-Nitro-1,3-dioxo-1,3-dihydro-2H-isoindol-2-yl)ethyl]benzonitril

R_{f} (SiO₂, Cyclohexan/EtOAc = 5/1) = 0.6, rt (Methode 4) = 4.16 min.

### Beispiel 13

Aus *tert*-Butyl-1,3-dioxo-2,3,3a,4,7,7a-hexahydro-1*H*-isoindol-4-ylcarbalmat (Darstellung s. DE 4123918) und 4-(2-Bromethyl)benzonitil:

### tert-Butyl-2-[2-(4-cyanophenyl)ethyl]-1,3-dioxo-2,3,3a,4,7,7a-hexahydro-1H-isoindol-4-ylcarbamat

MS = 337 (M - *tert*-Bu), rt (Methode 4) = 4.56 min.

### Beispiel 14

### [4-(2-Hydroxyethyl)phenyl]acetonitril

3.85 g (18.81 mmol) 4-Brommethyl-phenylessigsäure werden in 150 ml THF gelöst und unter Rühren bei 0°C langsam mit 20 ml ein 1M-Lösung von BH₃ in THF versetzt. Dann wird das Reaktionsgemisch langsam auf Raumtemperatur erwärmt und über Nacht bei Raumtemperatur gerührt. Das Reaktionsgemisch wird mit 2M Salzsäure versetzt und mit Essigsäureethylester extrahiert. Die organische Phase wird über Magnesiumsulfat getrocknet, filtriert und eingeengt. 3.64 g (16.92 mmol) des so erhaltenen 2-[4-(Brommethyl)phenyl]ethanols werden ohne weitere Reinigung in 36 ml DMSO gelöst und mit 1.65 g (25.38 mmol) Kaliumcyanid versetzt. Das Reaktionsgemisch wird 1 h bei Raumtemperatur gerührt und dann auf Diethylether/ges. wässrige Natriumhydrogencarbonat Lösung gegossen. Die organische Phase wird dreimal mit Wasser gewaschen, über Magnesiumsulfat getrocknet, filtriert und eingeengt. Es werden 2.5 g des Produktes erhalten.
rt (Methode 4) = 2.66 min.

### Beispiel 15

### {4-[2-(4-Nitro-1,3-dioxo-1,3-dihydro-2H-isoindol-2-yl)ethyl]phenyl}acetonitril

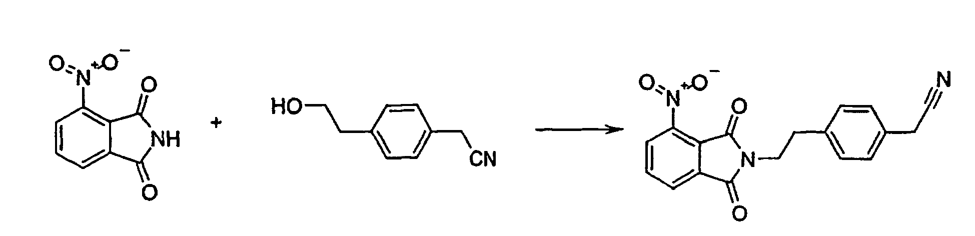

4.66 g (26.75 mmol) Diethylazodicarboxylat werden in 50 ml THF unter Argon vorgelegt. 2.57 g (13.38 mmol) 3-Nitrophthalimid, 2.37 g (14.71 mmol) [4-(2-Hydroxyethyl)phenyl]acetonitril und 7.02 g (26.75 mmol) Triphenylphosphin werden zugegeben, und es wird 20 h bei Raumtemperatur gerührt. Dann wird das Reaktionsgemisch auf Ether/wässrige Pufferlösung pH7 gegossen, die Phasen werden getrennt, die organische Phase wird über Magnesiumsulfat getrocknet, filtriert und einrotiert. Das Rohprodukt wird über Kieselgel chromatographiert (CH₂Cl₂). Es werden 3.08 g (69% d. Th.) der gewünschten Verbindung erhalten.
MS = 336.3 (M+H), R_{f} (SiO₂, CH2Cl2/EtOH =100/1) = 0.37.
Analog wurden dargestellt:

### Beispiel 16

Aus 3-Nitrophthalimid und 3-(2-Hydroxyethyl)benzonitril (Darstellung aus 2-(3-Nitrophenyl)ethanol über katalytische Reduktion der Nitrogruppe und anschliessende Sandmeyer Reaktion, vgl. G. Wagner, H. Vieweg, *Pharmazie; 37,* 1,**1982**; S. 13-16):

### 3-[2-(4-Nitro-1,3-dioxo-1,3-dihydro-2H-isoindol-2-yl)ethyl]benzonitril

R_{f} (SiO₂, CH₂Cl₂) = 0.23, rt (Methode 4) = 4.29 min.

### Beispiel 17

Aus *tert*-Butyl-1,3-dioxo-2,3,3a,4,7,7a-hexahydro-1*H*-isoindol-4-ylcarbamat und 3-(3-Hydroxy-1-propenyl)benzonitril (Darstellung aus 3-(3-Cyanophenyl)-2-propensäure (G. Wagner, C. Garbe, P. Richter, *Pharmazie; 28;* 11/12; **1973**; S. 724-729) mittels Boranreduktion):

### tert-Butyl-2-[(3-(3-cyanophenyl)-2-propenyl]-1,3-dioxo-2,3,3a,4,7,7a-hexahydro-1H-isoindol-4-ylcarbamat

MS = 408 (M+H), 352 (M+H - tert-Bu), rt (Methode 4) = 4.59 min.

### Beispiel 18

Aus *tert*-Butyl-1,3-dioxo-2,3,3a,4,7,7a-hexahydro-1*H*-isoindol-4-ylcarbamat und 3-(3-Hydroxy-1-propyl)benzonitril (Darstellung s. H. Vieweg, G. Wagner, *Pharmazie;* 37; 3; 1982; S. 178-182):

### tert-Butyl-2-[3-(3-cyanophenyl)propyl]-1,3-dioxo-2,3,3a,4,7,7a-bexabydro-1H-isoindol-4-ylcarbamat

MS = 410 (M+H), 354 (M+H - tert-Bu), 310 (M+H - tert-BuCO₂), rt (Methode 4) = 4.68 min.

### Beispiel 19

Aus *tert*-Butyl-1,3-dioxo-2,3,3a,4,7,7a-hexahydro-1*H*-isoindol-4-ylcarbamat und 3-(2-Hydroxyethyl)benzonitril:

### tert-Butyl-2-[2-(3-eyanophenyl)ethyl]-1,3-dioxo-2,3,3a,4,7,7a-hexahydro-1H-isoindol-4-ylcarbamat

MS = 396 (M+H), 340 (M+H - tert-Bu), 296 (M+H - tert-BuCO₂), rt (Methode 4) = 4.59 min.

### Beispiel 20

### 2-{4-[(4-Amino-1,3-dioxo-1,3-dihydro-2H isoindol-2-yl)methyl]phenyl}-acetonitril

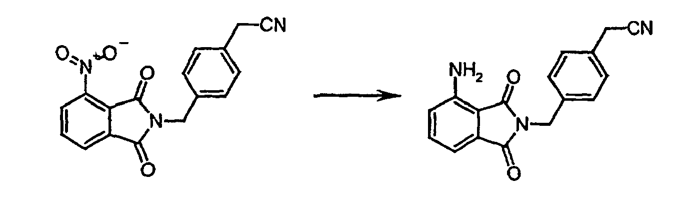

2.4 g (7.47 mmol) 2-{4-[(4-Nitro-1,3-dioxo-1,3-dihydro-2*H*-isoindol-2-yl)methyl]-phenyl}acetonitril werden in 130 ml eines Gemisches aus THF/EtOAc/EtOH (7/4/2) gelöst, mit 0.2 g Palladium auf Kohle (10%) versetzt und 6 h lang bei Raumtemperatur unter Wasserstoff-Atmosphäre (1.013 bar) gerührt. Dann wird das Reaktionsgemisch filtriert, das Lösungsmittel im Vakuum entfernt und das Rohprodukt über Kieselgel chromatographiert (CH₂Cl₂/EtOH = 100/0 bis 100/1). Es werden 1.78 g (82% d. Th.) der gewünschten Verbindung erhalten.

R_{f} (SiO₂, CH₂Cl₂/EtOH = 50/1) = 0.42, MS = 292 (M+H⁺), rt (Methode 4) = 3.77min.

Analog wurden durch Reduktion der Nitroverbindungen in THF/EtOAc/EtOH- oder EtOAc/EtOH-Gemischen folgende Amine dargestellt:

### Beispiel 21

### {3-[(4-Amino-1,3-dioxo-1,3-dihydro-2H-isoindol-2-yl)methyl]phenyl}acetonitril

MS = 292 (M+H), rt (Methode 4) = 3.85 min.

### Beispiel 22

### 4-(4-Amino-1,3-dioxo-1,3-dibydro-2H-isoindol-2-yl)butannitril

MS = 230 (M+H), rt (Methode 4) = 3.09 min.

### Beispiel 23

### 5-(4-Amino-1,3-dioxo-1,3-dihydro-2H-isoindol-2-yl)pentannitril

MS = 244 (M+H), rt (Methode 4) = 3.33 min.

### Beispiel 24

### 4-[2-(4-Amino-1,3-dioxo-1,3-dihydro-2H-isoindol-2-yl)ethyl]benzonitril

MS = 292 (M+H), rt (Methode 4) = 3.97 min.

### Beispiel 25

### {4-[2-(4-Amino-1,3-dioxo-1,3-dihydro-2H-isoindol-2-yl)ethyl]phenyl}acetonitril

MS = 306 (M+H), rt (Methode 4) = 3.98 min.

### Beispiel 26

### 3-[2-(4-Amino-1,3-dioxo-1,3-dihydro-2H-isoindol-2-yl)ethyl]benzonitril

MS = 292 (M+H), rt (Methode 4) = 3.98 min.

### Beispiel 27

### 4-[2-(4-Amino-1,3-dioxo-1,3,3a,4,7,7a-hexahydro-2H-isoindol-2-yl)ethyl]-benzonitril

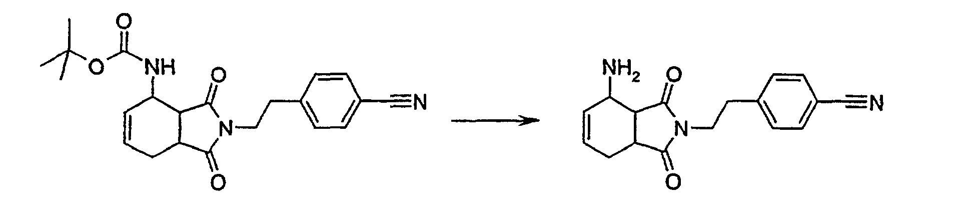

1.008 g (2.55 mmol) *tert*-Butyl-2-[2-(4-cyanophenyl)ethyl]-1,3-dioxo-2,3,3a,4,7,7a-hexahydro-1*H*-isoindol-4-ylcarbamat werden in 20 ml Dichlormethan gelöst und mit 0.79 ml (10.2 mmol) Trifluoressigsäure versetzt. Nach 4 h Rühren bei Raumtemperatur wird das Reaktionsgemisch einrotiert und mit EtOAc und gesättigter, wässriger NaHCO₃-Lsg. aufgearbeitet. Die organische Phase wird über Magnesiumsulfat getrocknet, filtriert und einrotiert. Das Rohprodukt wird an Kieselgel chromatographiert (CH₂Cl₂/EtOH = 50/1 + 0.5% wässrige NH₃-Lsg. (25%ig)). Es werden 530 mg (70% d. Th.) der gewünschten Verbindung erhalten. MS = 337 (M+H+MeCN), 296 (M+H),rt (Methode 4) = 2.04 min.

Analog wurden durch Umsetzung der entsprechenden *tert*-butoxycarbonylgeschützten Verbindungen mit Trifluoressigsäure in Dichlormethan die folgenden Amine dargestellt:

### Beispiel 28

### 3-[3-(4-Amino-1,3-dioxo-1,3,3a,4,7,7a-hexahydro-2H-isoindol-2-yl)-1-propenyl]benzonitril

MS = 349 (M+H+MeCN), 308 (M+H), rt (Methode 4) = 2.26 min.

### Beispiel 29

### 3-[3-(4-Amino-1,3-dioxo-1,3,3a,4,7,7a-hexahydro-2H-isoindol-2-yl)propyl]-benzonitril

MS = 310 (M+H), rt (Methode 4) = 2.28 min.

### Beispiel 30

### 3-[2-(4-Amino-1,3-dioxo-1,3,3a,4,7,7a-hexahydro-2H-isoindol-2-yl)ethyl]-benzonitril

MS = 296 (M+H), rt (Methode 4) = 2.04 min.

### Beispiel 31

### 5-Chlor-N-{2-[4-(cyanomethyl)benzyl]-1,3-dioxo-2,3-dihydro-1H-isoindol-4-yl}-2-thiophencarboxamid

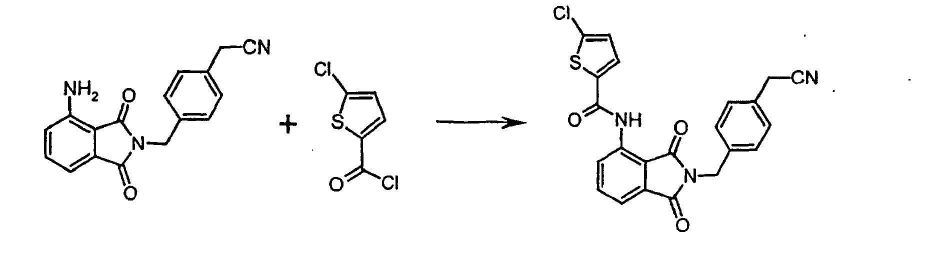

1.07 g (3.67 mmol) 2-{4-[(4-Amino-1,3-dioxo-1,3-dihydro-2*H*-isoindol-2-yl)-methyl]phenyl}-acetonitril, 1.33 g (7.35 mmol) 5-Chlor-2-thiophencarbonsäurechlorid (erhalten durch Umsetzung von 5-Chlor-2-thiophencarbonsäure mit SOCl₂) und 90 mg (0.74 mmol) 4-DMAP werden in 10 ml Pyridin 20 h bei Raumtemperatur gerührt. Das Reaktionsgemisch wird auf 2 N Salzsäure gegossen, und der entstehende Niederschlag wird abgesaugt, mit Wasser gewaschen und im Vakuum getrocknet. Es werden 1.57 g (98% d. Th.) der gewünschten Verbindung erhalten. R_{f} (SiO₂, CH₂Cl₂) = 0.25. Das Produkt kann ggf. durch Chromatografie an Kieselgel mit Methylenchlorid/Ethanol-Gemischen gereinigt werden.

Analog wurden durch Umsetzung der entsprechenden Amine mit 5-Chlor-2-thiophencarbonsäurechlorid dargestellt:

### Beispiel 32

### 5-Chlor-N-{2-[3-(cyanomethyl)benzyl]-1,3-dioxo-2,3-dihydro-1H-isoindol-4-yl}-2-thiophencarboxamid

MS = 436 (M+H), rt (Methode 4) = 5.27 min.

### Beispiel 33

### 5-Chlor-N-[2-(3-cyanopropyl)-1,3-dioxo-2,3-dihydro-1H-isoindol-4-yl]-2-thiophencarboxamid

MS = 374 (M+H), rt (Methode 4) = 4.80 min.

### Beispiel 34

### 5-Chlor-N-[2-(4-cyanobutyl)-1,3-dioxo-2,3-dibydro-1H-isoindol-4-yl]-2-thiophencarboxamid

MS = 388 (M+H), rt (Methode 4) = 5.03 min.

### Beispiel 35

### 5-Chlor-N-{2-[2-(4-cyanophenyl)ethyl]-1,3-dioxo-2,3-dihydro-1H-isoindol-4-yl}-2-thiophencarboxamid

MS = 436 (M+H), rt (Methode 4) = 5.42 min.

### Beispiel 36

### 5-Chlor-N-(2-{2-[4-(cyanomethyl)phenyl]ethyl}-1,3-dioxo-2,3-dihydro-1H-isoindol-4-yl)-2-thiophencarboxamid

R_{f} (SiO₂, CH₂Cl₂/EtOH = 100/1) = 0.5, rt (Methode 4) = 5.52 min.

### Beispiel 37

### 5-Chlor-N-{2-[2-(3-cyanophenyl)ethyl]-1,3-dioxo-2,3-dihydro-1H-isoindol-4-yl}-2-thiophencarboxamid

MS = 436 (M+H), rt (Methode 4) = 5.45 min.

### Beispiel 38

### 5-Chlor-N-{2-[2-(3-cyanophenyl)ethyl]-1,3-dioxo-2,3,3a,4,7,7a-hexahydro-1H-isoindol-4-yl}-2-thiophencarboxamid

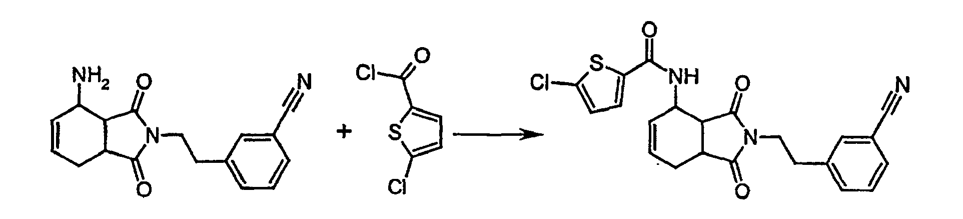

54 mg (0.183 mmol) 3-[2-(4-Amino-1,3-dioxo-1,3,3a,4,7,7a-hexahydro-2*H*-isoindol-2-yl)ethyl]benzonitril werden in 5 ml THF gelöst und bei 0°C mit 20.4 mg (0.2 mmol) Triethylamin und 36.4 mg (0.2 mmol) 5-Chlor-2-thiophencarbonsäurechlorid versetzt. Dann wird das Reaktionsgemisch für 2 h bei Raumtemperatur gerührt und anschließend auf Essigester und ges. wässr. NaHCO₃-Lsg. gegossen. Die organische Phase wird über Magnesiumsulfat getrocknet, filtriert und eingeengt. Das Rohprodukt wird an Kieselgel chromatographiert (CH₂Cl₂/EtOH = 100/0 - 100/1).
Es werden 58 mg (72% d. Th.) der gewünschten Verbindung erhalten.
MS = 440 (M+H), rt (Methode 4) = 4.59 min.

Die Reaktion kann alternativ auch in Dichlormethan statt THF durchgeführt werden.

Analog wurden durch Umsetzung der entprechenden Amine mit 5-Chlor-2-thiophencarbonsäurechlorid dargestellt:

### Beispiel 39

### 5-Chlor-N-{2-[2-(4-cyanophenyl)ethyl]-1,3-dioxo-2,3,3a,4,7,7a-hexahydro-1H isoindol-4-yl}-2-thiophencarboxamid

MS = 440 (M+H), R_{f} (SiO₂, Cyclohexan/Essigsäureethylester =1/1) = 0.27.

### Beispiel 40

### 5-Chlor-N-{2-[3-(3-cyanophenyl)-2-propenyl]-1,3-dioxo-2,3,3a,4,7,7a-hexahydro-1H-isoindol-4-yl}-2-thiophencarboxamid

MS = 452 (M+H), rt (Methode 4) = 4.59 min.

### Beispiel 41

### 5-Chlor-N -{2-[3-(3-cyanophenyl)propyl]-1,3-dioxo-2,3,3a,4,7,7a-hexahydro-1H-isoindol-4-yl}-2-thiophencarboxamid

MS = 454 (M+H), rt (Methode 4) = 4.68 min.

### Beispiel 42

### 5-Chlor-N-{2-[4-(cyanomethyl)benzyl]-3-hydroxy-1-oxo-2,3-dihydro-1H-isoindol-4-yl}-2-thiaphencarboxamid und 5-Chlor-N-{2-[4-(cyanomethyl)benzyl]-1-hydroxy-3-oxo-2,3-dihydro-1H-isoindol-4-yl}-2-thiophencarboxamid

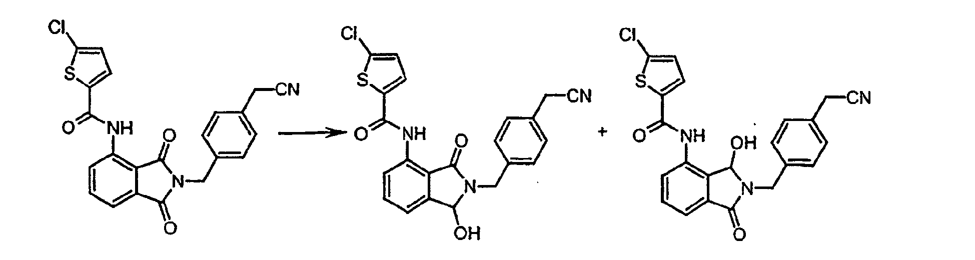

3 g (6.88 mmol) 5-Chlor-*N*-(2-[4-(cyanomethyl)benzyl]-1,3-dioxo-2,3-dihydro-1*H-*isoindol-4-yl}-2-thiophencarboxamid werden in einem Gemisch aus 200 ml Methanol und 400 ml Dichlormethan gelöst. Die Lösung wird mit 1.04 g (27.53 mmol) Natriumborhydrid versetzt und 4 h bei Raumtemperatur gerührt. Dann wird das Reaktionsgemisch vorsichtig auf 2 N Salzsäure gegossen. Die Phasen werden getrennt, die organische Phase wird über Magnesiumsulfat getrocknet, filtriert und das Lösungsmittel im Vakuum entfernt. Das Rohprodukt wird über Kieselgel chromatographiert (CH₂Cl₂/EtOH/konz. Ammoniaklsg. = 200/1/0.1 bis 20/1/0.1). Zuerst wird 5-Chlor-*N*-{2-[4-(cyanomethyl)benzyl]-1-hydroxy-3-oxo-2,3-dihydro-1*H*-isoindol-4-yl}-2-thiophencarboxamid eluiert. Es werden 1.07 g (36% d. Th.) dieser Verbindung erhalten.

R_{f} (SiO₂, CH₂Cl₂/EtOH/konz. Ammoniaklsg. = 50/1/0.01) = 0.17, MS = 438.3 (M+H⁺).

Als zweite Fraktion wird 5-Chlor-*N*-{2-[4-(cyanomethyl)benzyl]-3-hydroxy-1-oxo-2,3-dihydro-1*H*-isoindol-4-yl}-2-thiophencarboxamid eluiert. Es werden 0.85 g (28% d. Th.) dieser Verbindung erhalten.

R_{f} (SiO₂, CH₂Cl₂/EtOH/konz. Ammoniaklsg. = 50/1/0.01) = 0.11, MS = 438.3 (M+H⁺).

### Beispiel 43

### 5-Chlor-N-{2-[4-(cyanomethyl)benzyl]-1-oxo-2,3-dihydro-1H-isoindol-4-yl}-2-thiophencarboxamid

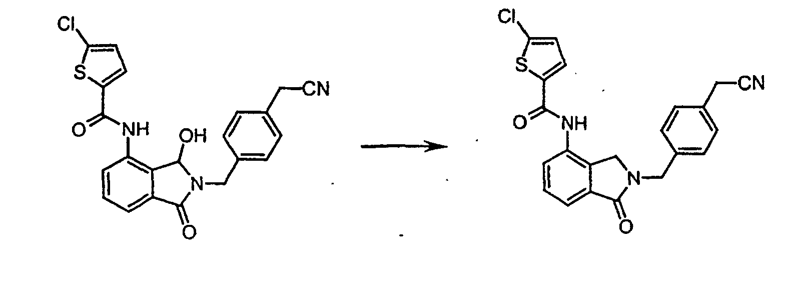

807 mg (1.84 mmol) 5-Chlor-*N*-{2-[4-(cyanomethyl)benzyl]-3-hydroxy-1-oxo-2,3- . dihydro-1*H*-isoindol-4-yl}-2-thiophencarboxamid werden in einem Gemisch aus 20ml Dichlormethan und 1.7 ml Trifluoressigsäure gelöst. Dann werden 429 mg (3.69 mmol) Triethylsilan zugegeben, und es wird über Nacht bei Raumtemperatur gerührt. Das Reaktionsgemisch wird mit Dichlormethan verdünnt, zweimal mit ges. NaHCO₃-Lsg und einmal mit 2 M Salzsäure gewaschen. Die organische Phase wird über Magnesiumsulfat getrocknet, filtriert und das Lösungsmittel wird im Vakuum entfernt. Es werden 765 mg (98% d. Th.) der gewünschten Verbindung erhalten. R_{f} (SiO₂, CH₂Cl₂/EtOH = 20/1) = 0.42, MS = 422.3 (M+H⁺).

### Beispiel 44

Analog kann 5-Chlor-*N*-{2-[4-(cyanomethyl)benzyl]-1-hydroxy-3-oxo-2,3-dihydro-1*H*-isoindol-4-yl}-2-thiophencarboxamid zu **5-Chlor-*N*-{2-[4-(cyanomethyl)-benzyl]-3-oxo-2,3-dihydro-1*H*-isoindol-4-yl}-2-thiophencarboxamid** umgesetzt werden.

R_{f} (SiO₂, CH₂Cl₂/EtOH =100/1) = 0.54, MS = 422.3 (M+H⁺).

### Beispiel 45

### N-{2-[4-(2-Amino-2-thioxoethyl)benzyl]-1,3-dioxo-2,3-dihydro-1H-isoindol-4-yl}-5-chlor-2-thiophencarboxamid

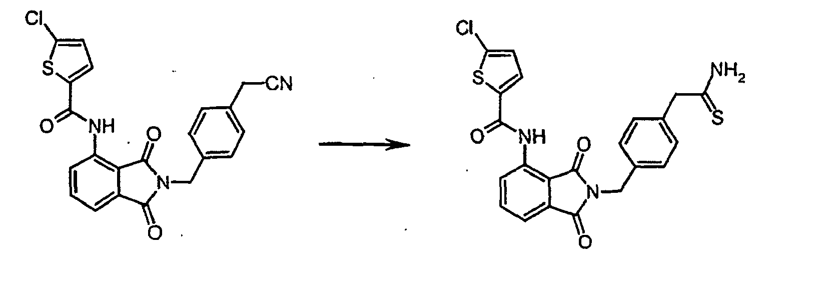

1.38 g (3.16 mmol) 5-Chlor-*N*-{2-[4-(cyanomethyl)benzyl]-1,3-dioxo-2,3-dihydro-1*H*-isoindol-4-yl}-2-thiophencarboxamid werden in 10 ml einer gesättigten Lösung von Schwefelwasserstoff in Pyridin gelöst. Die Lösung wird mit 2.56 g (25.31 mmol) Triethylamin versetzt und 20 h bei Raumtemperatur gerührt. Dann werden weitere 10 ml der gesättigten Schwefelwasserstoff Pyridin Lösung und 1.3 g Triethylamin zugegeben und weitere 20 h gerührt. Anschließend wird das Reaktionsgemisch mit EtOAc verdünnt, dreimal mit 2 N Salzsäure und einmal mit Pufferlösung pH7 gewaschen. Die organische Phase wird mit Magnesiumsulfat getrocknet, filtriert und im Vakuum eingedampft. Der Rückstand wird an Kieselgel chromatographiert. Es werden 0.87 g (59% d. Th.) der gewünschten Verbindung erhalten.
Rr(SiO₂, CH₂Cl₂/EtOH = 20/1) = 0.41, MS = 470 (M+H⁺).

### Beispiel 46

### Methyl-2-{4-[(4-{[(5-chlor-2-thienyl)carbonyl]amino}-1,3-dioxo-1,3-dihydro-2H isoindol-2-yl)methyl]phenyl}ethanimidothioat

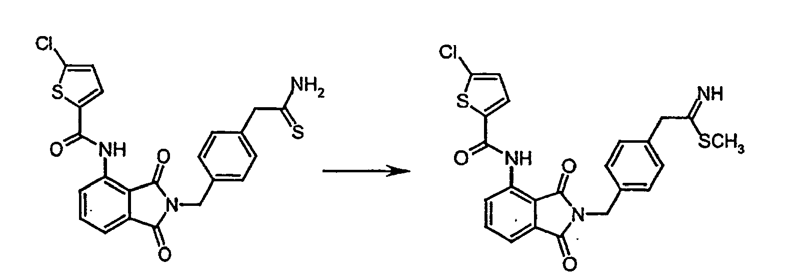

850 mg (1.81 mmol) *N*-{2-[4-(2-Amino-2-thioxoethyl)benzyl]-1,3-dioxo-2,3-dihydro-1*H*-isoindol-4-yl}-5-chlor-2-thiophencarboxamid werden in 150 ml Aceton gelöst und mit 5.134 g (36.17 mmol) Methyliodid versetzt. Das Gemisch wird unter Rühren für 2 h auf 60°C erwärmt und nach Abkühlen auf Raumtemperatur im Vakuum eingedampft. Das Rohprodukt wird ohne weitere Reinigung in der nächsten Stufe eingesetzt.

R_{f} (SiO₂, EtOAc/Cyclohexan =1/1) = 0.40.

### Beispiel 47

### N-{2-[4-(2-Amino-2-iminoethyl)benzyl]-1,3-dioxo-2,3-dihydro-1H-isoindol-4-yl}-5-chlor-2-thiophencarboxamid

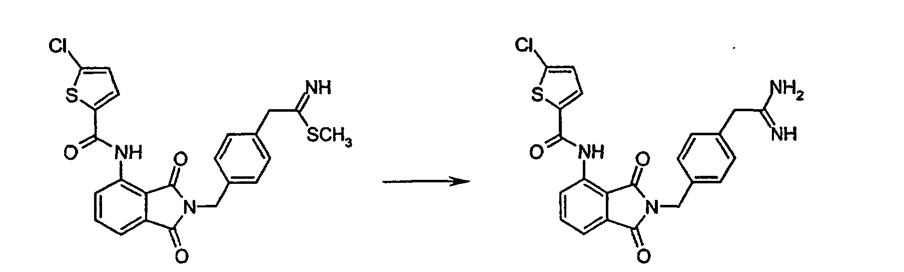

320 mg (0.661 mmol) Methyl-2-{4-[(4-{[(5-chlor-2-thienyl)carbonyl]amino}-1,3-dioxo-1,3-dihydro-2*H*-isoindol-2-yl)methyl]phenyl}ethanimidothioat werden in 40 ml Methanol suspendiert und mit 153 mg (1.98 mmol) Ammoniumacetat und 106 mg (1.98 mmol) Ammoniumchlorid versetzt. Das Gemisch wird über Nacht bei Raumtemperatur gerührt, mit Methanol und Methylenchlorid verdünnt bis eine klare Lösung entsteht und auf Kieselgel aufgezogen. Chromatographie an Kieselgel (CH₂Cl₂/EtOH = 10/1 bis 3/1) ergibt die gewünschte Verbindung. Das Produkt wird in Methanol und Methylenchlorid gelöst und mit Trifluoressigsäure versetzt. Entfernen des Lösungsmittel im Vakuum ergibt 298 mg (80% d. Th.) des gewünschten Produkts als Trifluoressigsäure-Salz.

Alternativ kann die Reinigung auch durch Chromatographie an einer RP8-Kieselgel Säule erfolgen (Wasser/Acetonitril = 4/1 + 0.1% Trifluoressigsäure bis 1/1 + 0.1% Trifluoressigsäure).
R_{f} (SiO₂, CH₂Cl₂/EtOH = 5/1) = 0.12, MS = 453.3 (M+H⁺).

### Beispiel 48

### 5-Chlor-N-(2-{4-[2-imino-2-(propylamino)ethyl]benzyl}-1,3-dioxo-2,3-dihydro-1H-isoindol-4-yl)-2-thiophencarboxamid

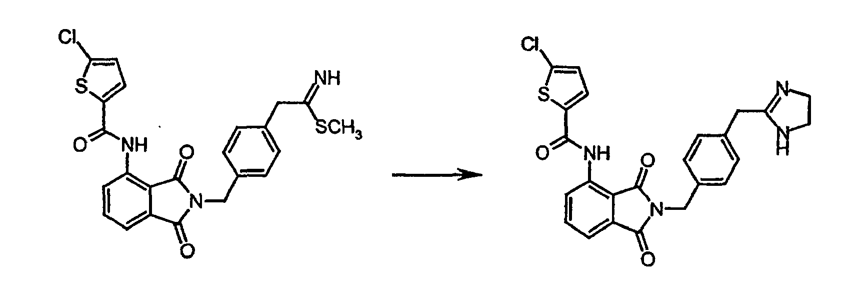

1.5 g (3.1 mmol) Methyl-2-{4-[(4-{[(5-chlor-2-thienyl)carbonyl]amino}-1,3-dioxo-1,3-dihydro-2*H*-isoindol-2-yl)methyl]phenyl}ethanimidothioat werden in 190 ml Methanol suspendiert und mit 280 mg (4.65 mmol) Ethylendiamin und 370 mg (6.2 mmol) Essigsäure versetzt. Das Gemisch wird über Nacht bei Raumtemperatur gerührt, mit Methanol und Methylenchlorid verdünnt bis eine klare Lösung entsteht und auf Kieselgel aufgezogen. Chromatographie an Kieselgel (CH₂Cl₂/EtOH = 10/1 bis 3/1) ergibt 570 mg (31% d. Th.) der gewünschten Verbindung. Das Produkt wird in Methanol und Methylenchlorid gelöst und mit Trifluoressigsäure versetzt. Entfemen des Lösungsmittel im Vakuum ergibt das Produkt als Trifluoressigsäure-Salz. Alternativ kann die Reinigung auch durch Chromatographie an einer RP8-Kieselgel Säule erfolgen (Wasser/Acetonitril = 4/1 + 0.1% Trifluoressigsäure bis 1/1 + 0.1% Trifluoressigsäure).
R_{f}(SiO₂, CH₂Cl₂/EtOH = 5/1) = 0.18, MS = 479.3 (M+H⁺).

Auf analoge Weise erhält man aus den entsprechenden Nitrilen durch Umsetzung mit H₂S/Pyridin, Methyliodid und Ammoniumsalzen bzw. von entsprechenden Aminen oder Diaminen die Verbindungen der folgenden Tabelle:

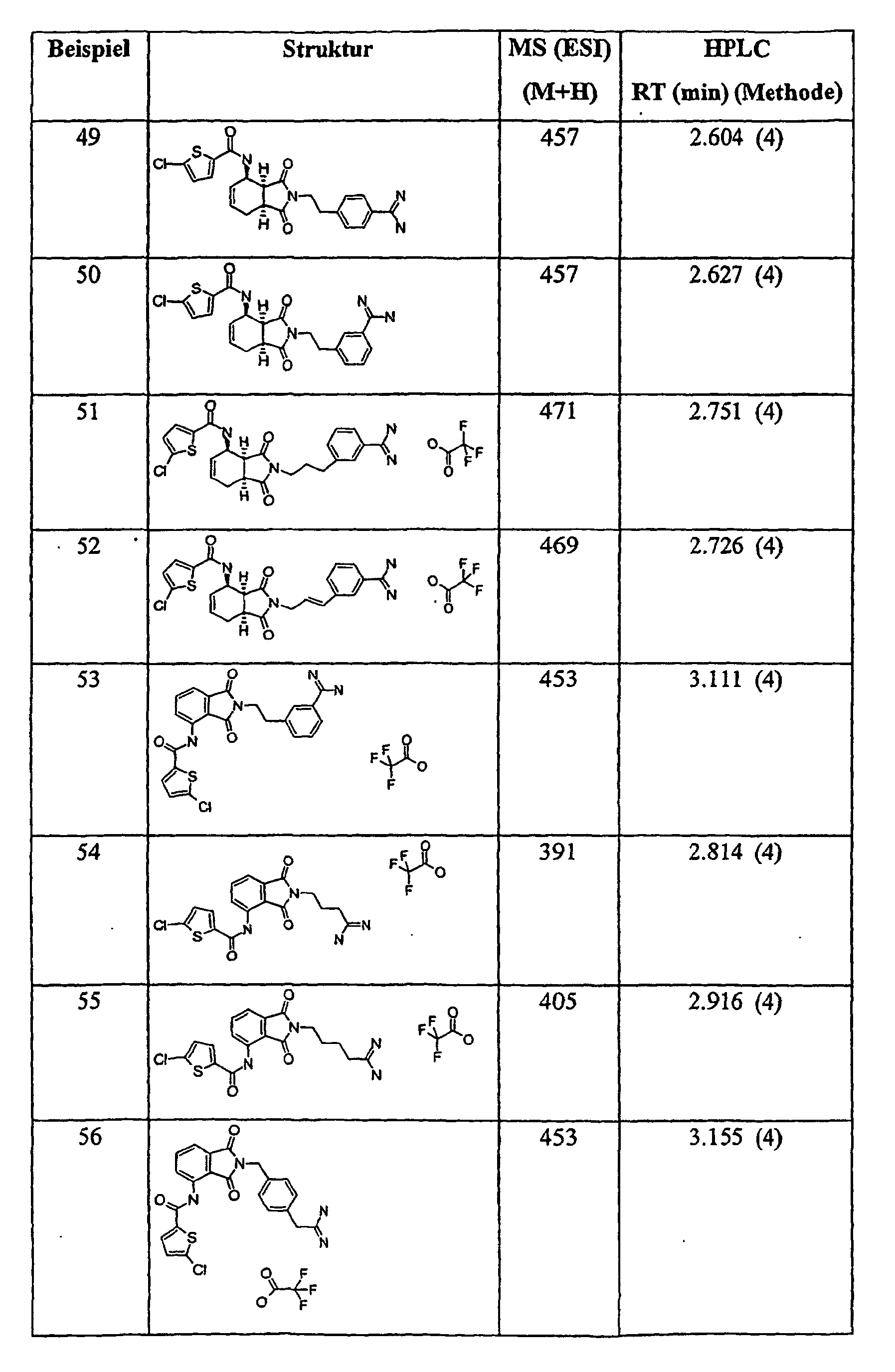

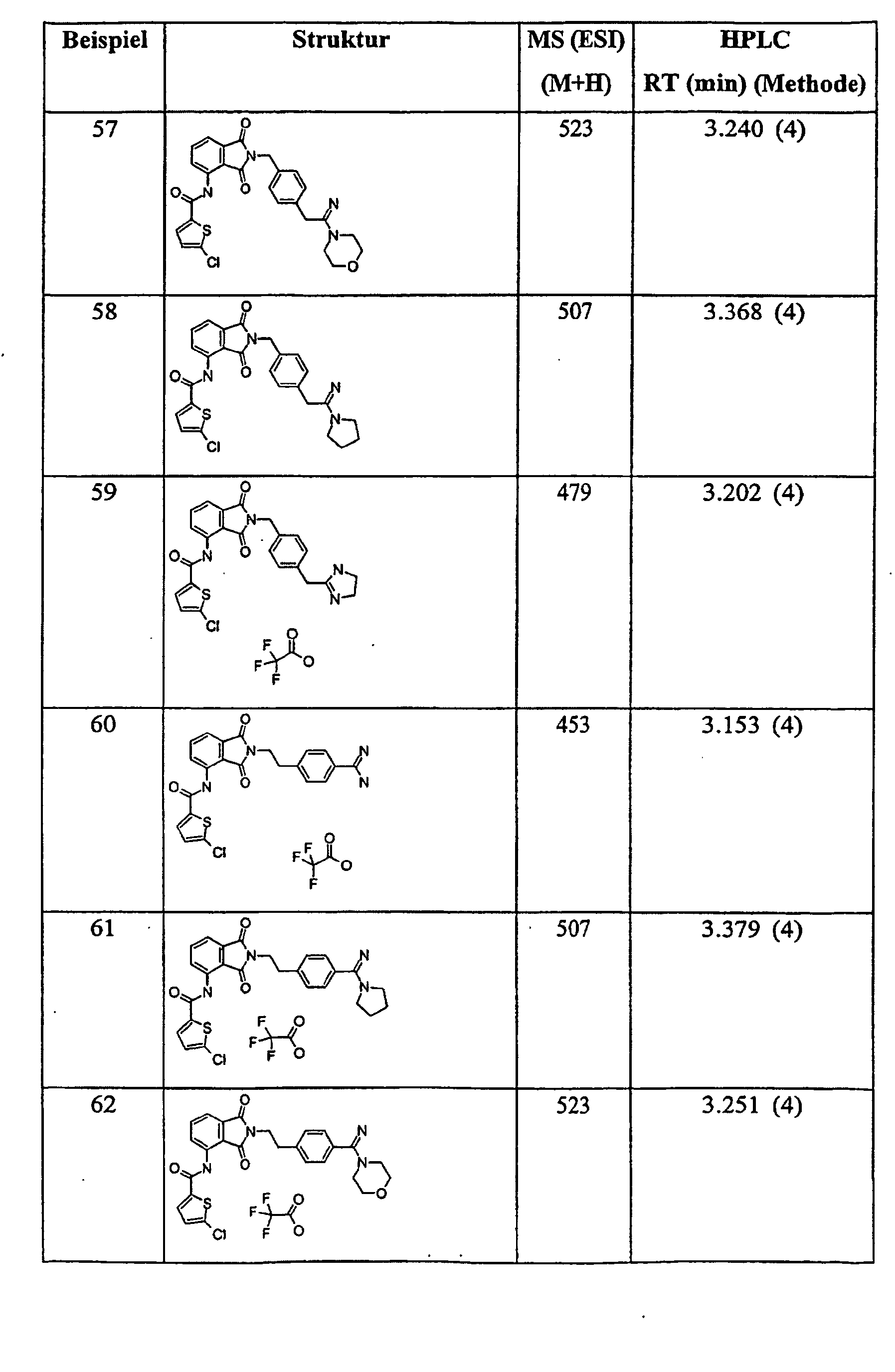

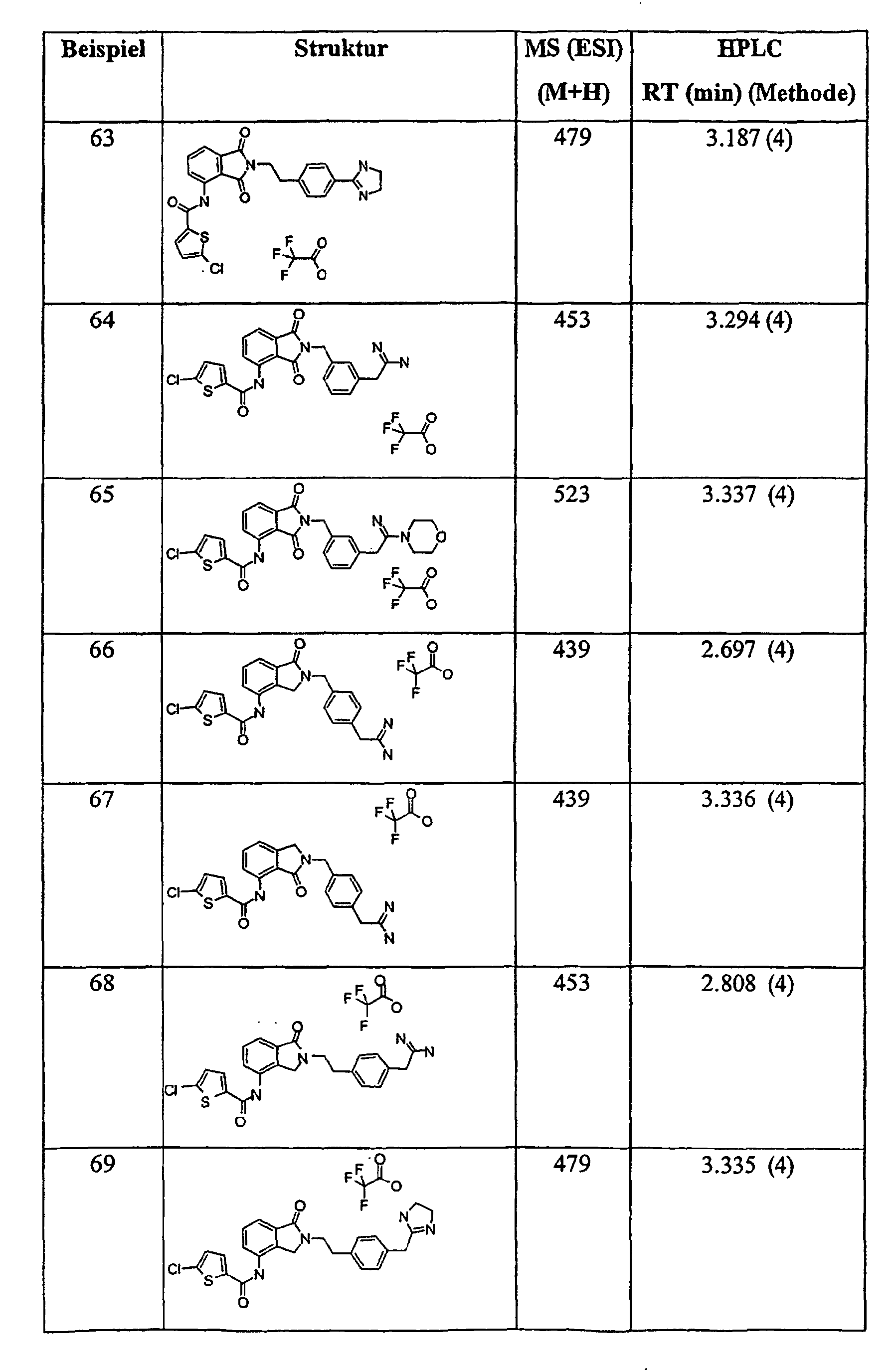

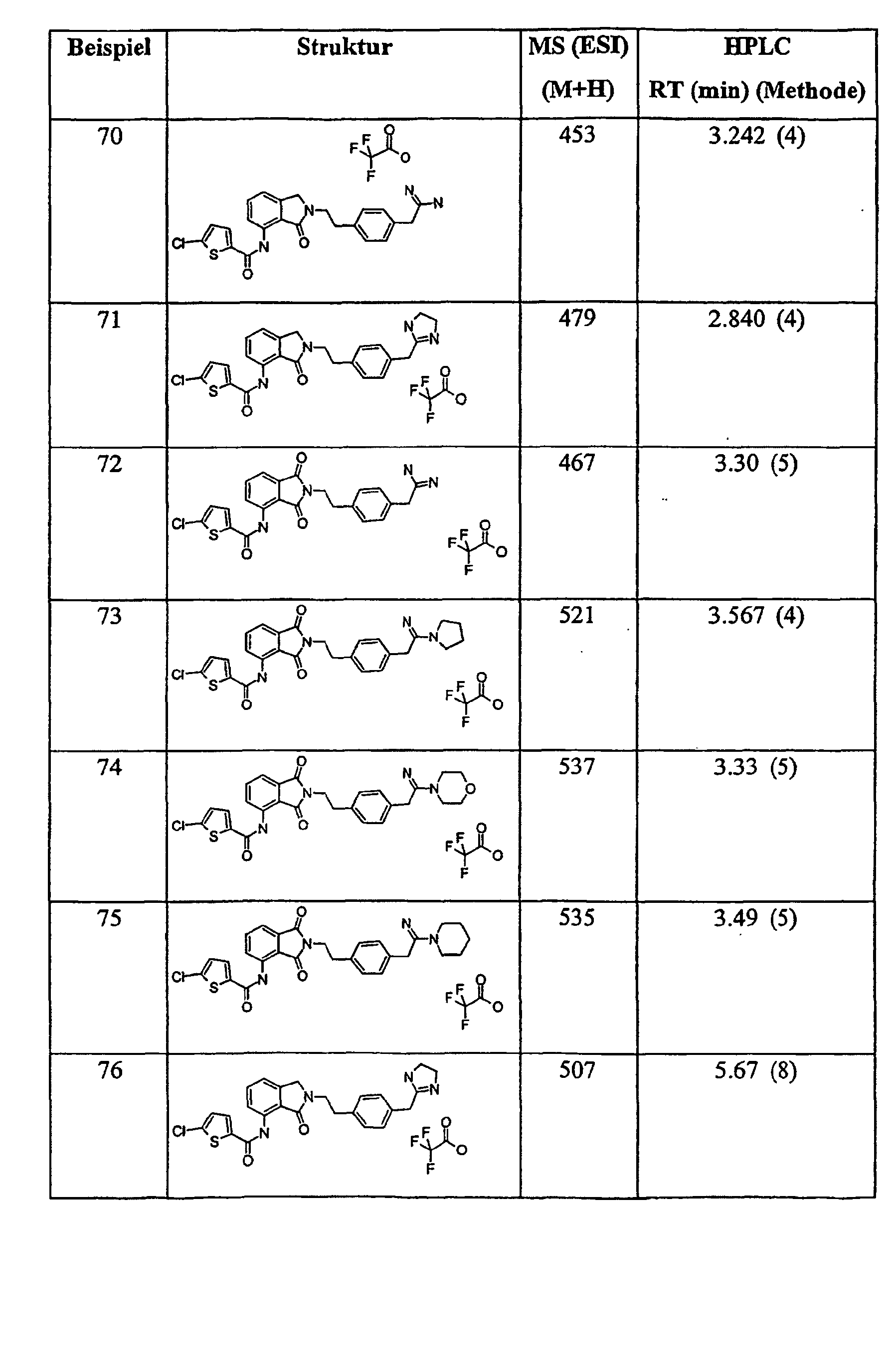

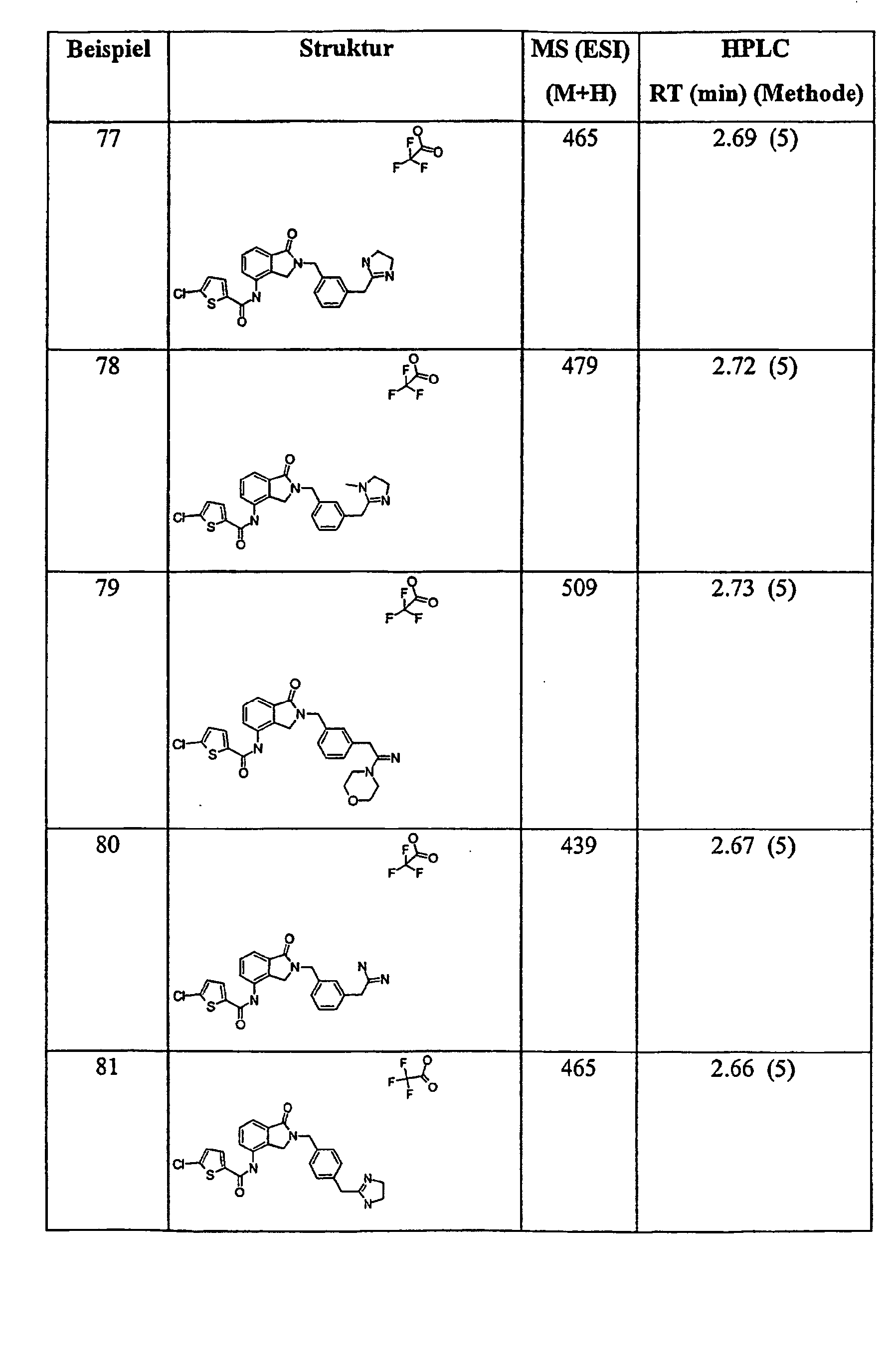

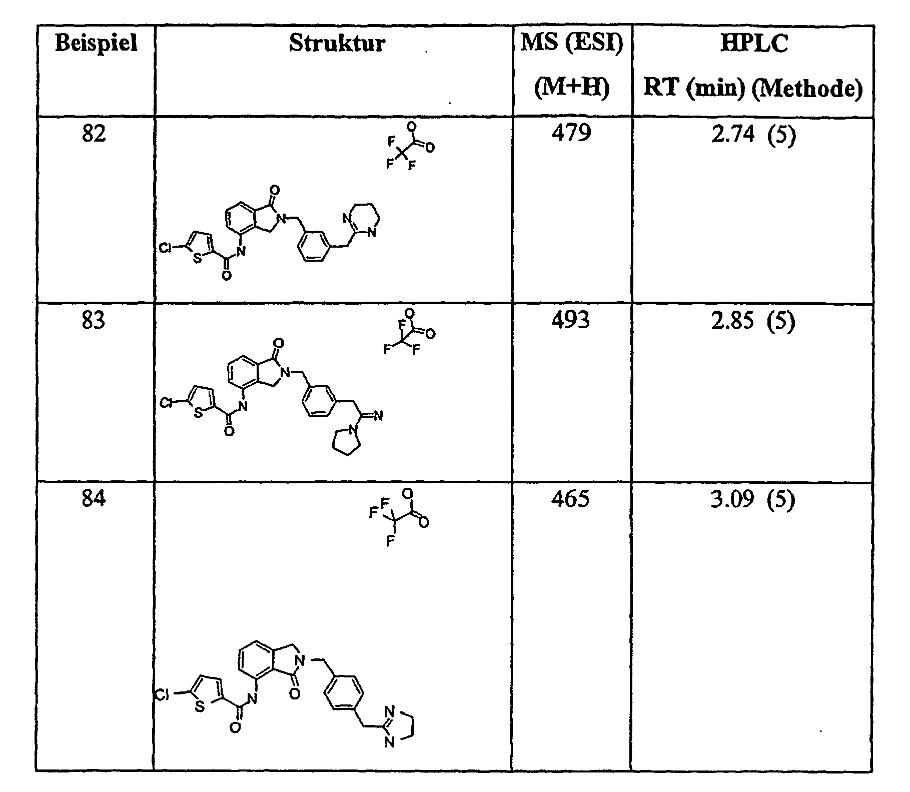

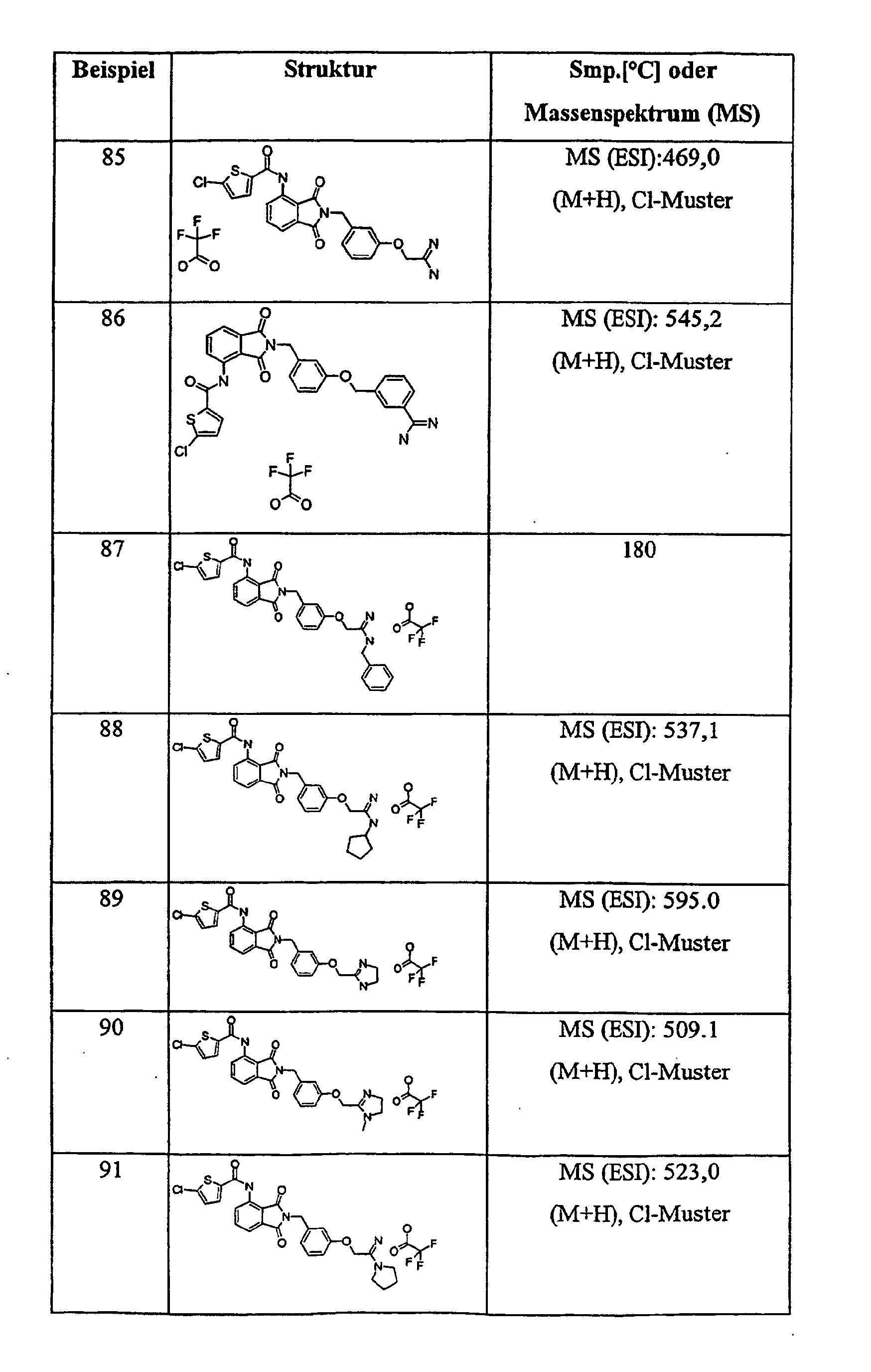

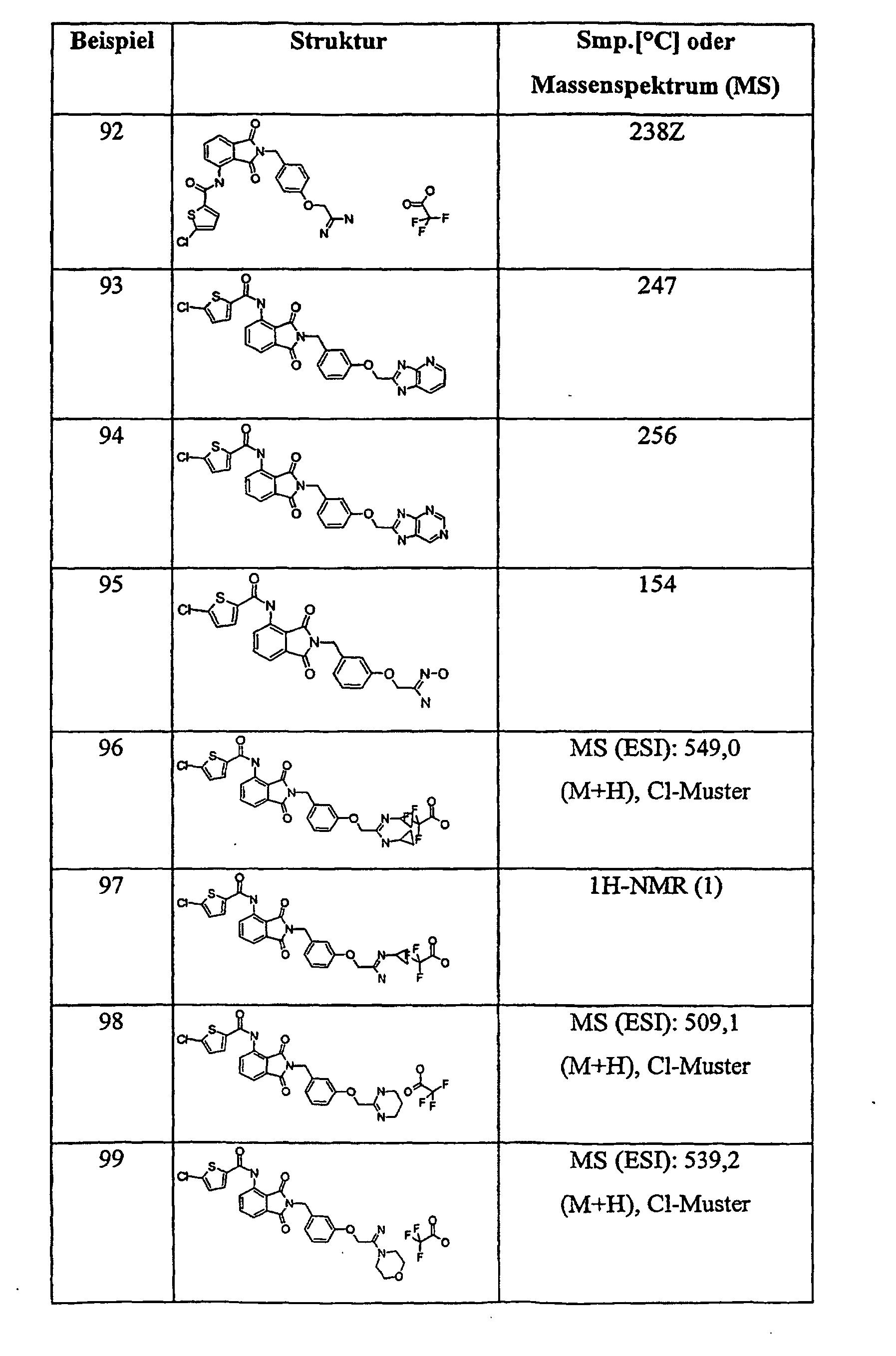

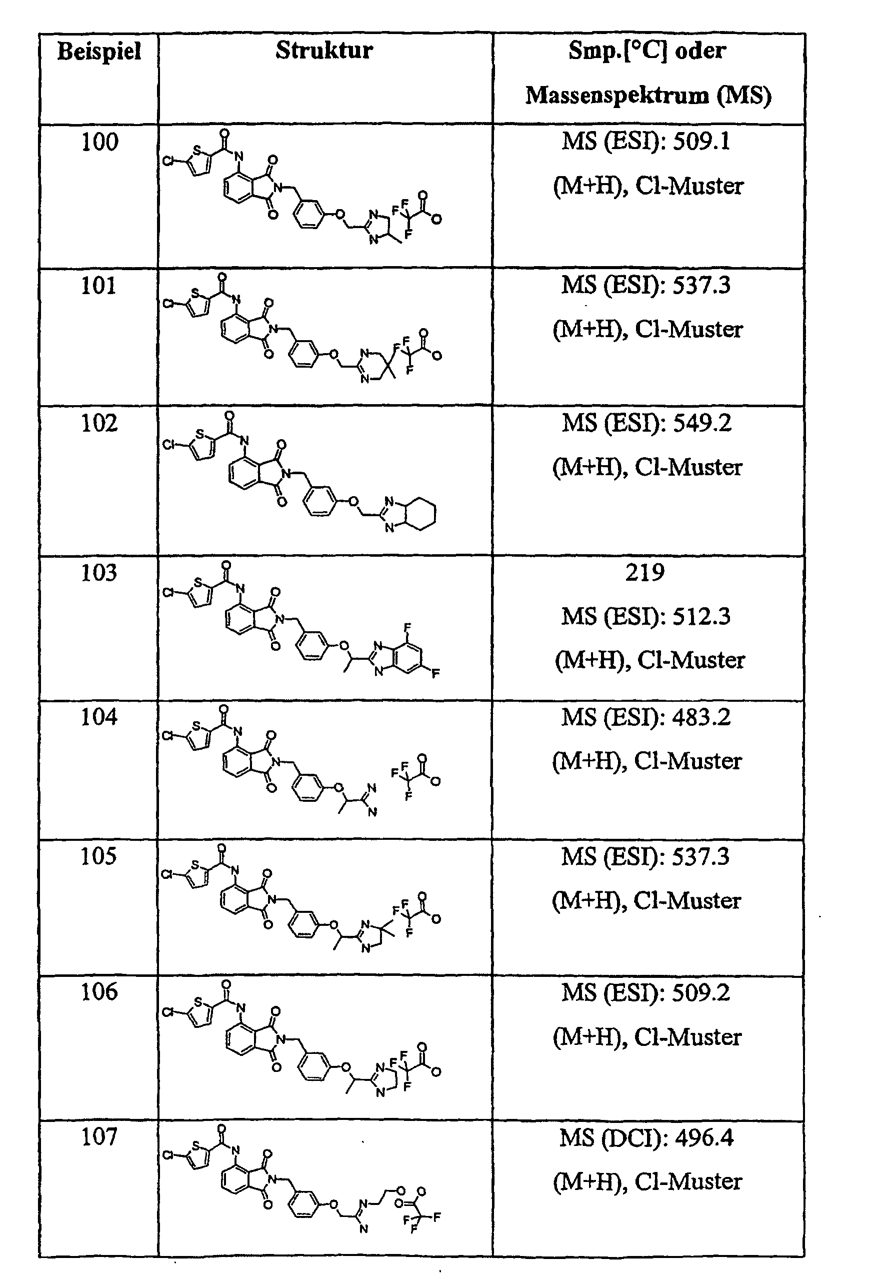

(1) ^{*1*}*H-NMR (d*^{*6*}*-,DMSO)* 0.6-0.7 (m, 2H), 0.8-0.9 (m, 2H), 2.6-2.7 (m, 1H), 4.8 (s, 2H), 4.85 (s, 2H), 6.85-7.0 (m, 2H), 7.05 (d, 1H), 7.25-7.4 (m, 2H), 7.7 (d, 1H), 7.8 (d, 1H), 7.9 (t, 1H), 8.3 (d, 2H), 9.2 (s, 1H), 9.5 (s, 1H), 9.9 (s, 1H), 10.45 (s, 1H).

### Beispiel 108

### N-(2-{4-[2-Amino-2-(hydroxyimino)ethyl]benzyl}-1,3-dioxo-2,3-dihydro-1H-isoindol-4-yl)-5-chlor-2-thiophencarboxamid

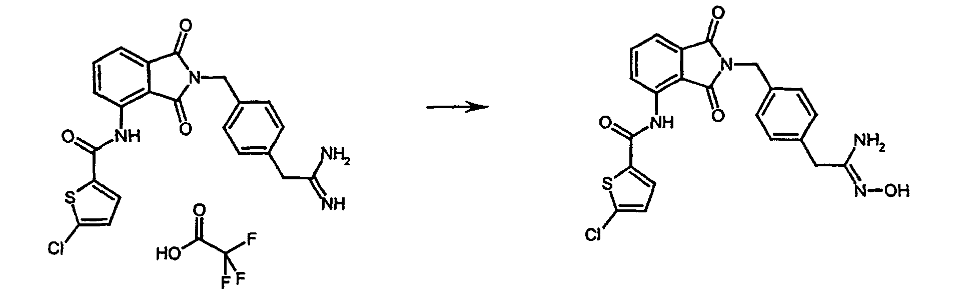

37 mg (0.07 mmol) *N*-{2-[4-(2-Amino-2-iminoethyl)benzyl]-1,3-dioxo-2,3-dihydro-1*H*-isoindol-4-yl}-5-chlor-2-thiophencarboxamid Trifluoroacetat werden in 3 ml Ethanol suspendiert, und es werden 0.09 ml (0.65 mmol) Triethylamin und 22.7 mg (0.33 mmol) Hydroxylammoniumchlorid zugegeben. Das Reaktionsgemisch wird 5 h bei Raumtemperatur gerührt und anschließend eingeengt. Der Rückstand wird mit Wasser verrührt und filtriert. Es werden 21 mg (69% d. Th.) des gewünschten Produkts als Feststoff erhalten.
MS = 469 (M+H), rt (Methode 4) = 3.29 min.

### Beispiel 109

### 4-[3-(Hydroxymethyl)phenoxy]-2-pyridincarboxamid

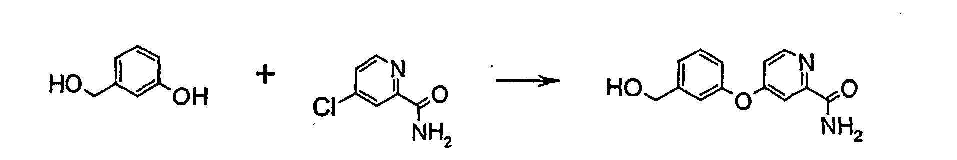

Man löst 3-Hydroxybenzylalkohol (7.93 g, 63.8 mmol) in 500 ml DMF, gibt Natriumethanolat (5.22 g, 76.6 mmol) hinzu, rührt 15 min unter Feuchtigkeitsausschluss und gibt anschließend 4-Chlorpyridin-2-carbonsäureamid (D. Varlet et al. *Heterocycles* **2000**, 53, 797-804) hinzu. Man rührt 70 Stunden bei 138°C und gibt nochmals eine Lösung von 4 g 3-Hydroxybenzaldehyd und 2.6 g Natriumethanolat in 100 ml DMF, die 15 min gerührt wird, hinzu. Man rührt 12 h bei 138°C und dampft anschließend im Vakuum ein. Der Rückstand wird in gesättigte KH₂PO₄-Lösung gegeben und mit Essigester extrahiert Die organische Phase wird mit Wasser gewaschen. Die vereinigten wässrigen Phasen werden nochmals mit Essigester extrahiert. Die vereinigten getrockneten organischen Phasen werden im Vakuum eingedampft und auf Kieselgel mit einem Toluol/Essigester-Gradienten chromatographiert. Man erhält 5.04 g (32.3% d.Th.) der Zielverbindung mit einem Smp. von 113°C und einem R_{f} (SiO₂, Essigester) von 0.49.

### Beispiel 110

Analog wurde **4-[3-(Hydroxymethyl)phenoxy]-*N*-methyl-2-pyridincarboxamid** dargestellt.

### Beispiel 111

### 4-{3-[(4-{[(5-Chlor-2-thienyl)carbonyl]amino}-1,3-dioxo-1,3-dihydro-2H-isoindol-2-yl)methyl]phenoxy}-2-pyridincarboxamid

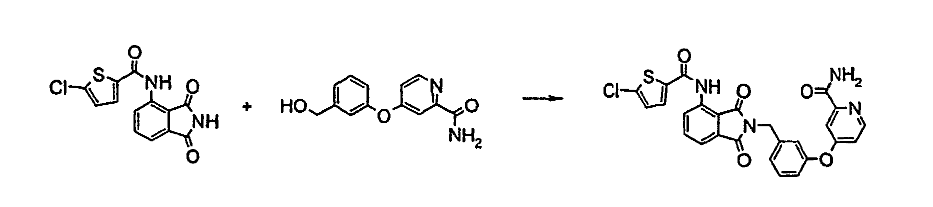

Man legt in THF unter Argon Triphenylphosphin (0.34 g, 1.3 mmol), 5-Chlor-*N-*(1,3-dioxo-2,3-dihydro-1*H*-isoindol-4-yl)-2-thiophencarboxaznid (0.2g, 0.65 mmol) und 4-[3-(Hydroxymethyl)phenoxy]-2-pyridincarboxamid (0.17 g, 0.71 mmol) vor und gibt Diethylazodicarboxylat (DEAD) (0.2 ml, 1.3 mmol) hinzu. Man rührt 1 h bei Raumtemperatur, gibt 1 g Kieselgel hinzu, dampft im Vakuum ein und chromatographiert auf Kieselgel mit einem Toluol/Essigester-Gradienten. Die gewünschte Fraktion wird aus Toluol umkristallisiert und mit Ether gewaschen. Die Kristalle enthalten 2 Moläquivalente Diethyl-1,2-hydrazinedicarboxylat.
Smp. 202°C, R_{f} (Toluol/Essigester = 1:1) = 0.46.

### Beispiel 112

Analog erhält man **4-{3-[(4-{[(5-Chlor-2-thienyl)carbonyl]amino}-1,3-dioxo-1,3-dihydro-2*H*-isoindol-2-yl)methyl]phenoxy}-*N*-methyl-2-pyridincarboxamid Trifluoracetat** nach Chromatographie auf Kromasil 100C18 und Elution mit Wasser/Acetonitril/1-proz.TFA = 24/70/6) in 27% Ausbeute:

### Beispiel 113

Analog erhält man aus 2-Amino-4-[3-(hydroxymethyl)phenoxy]pyridin (dargestellt durch Hoffmann-Abbau von 4-[3-(Hydroxymethyl)phenoxy]-2-pyridinecarboxamid mittels HOBr) nach 48-stündiger Reaktionszeit **N-(2-{3-[(2-Amino-4-pyridinyl)-oxy]benzyl}-1,3-dioxo-2,3-dihydro-1*H* isoindol-4-yl)-5-chlor-2-thiophencarboxamid**

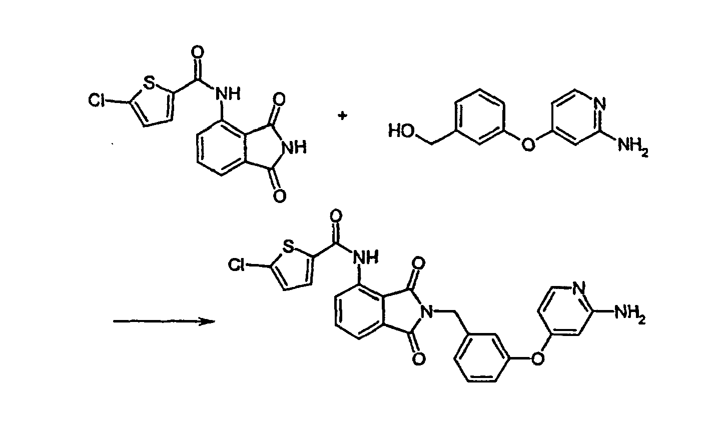

### Beispiel 114

### 3-(Hydroxymethyl)benzyl-tert-butylcarbamat

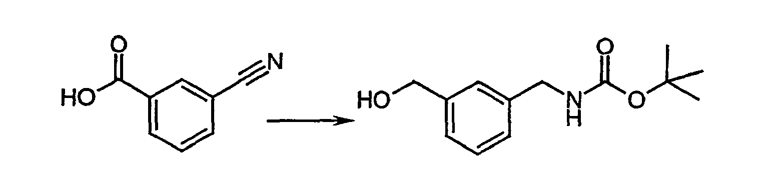

Zu einer eisgekühlten Lösung von 7.36 g (500 mmol) 3-Cyanobenzoesäure in 100 ml THF werden langsam 100 ml 1N BH₃-Lösung (100 mmol) in THF getropft. Nach 2 h wird die Kühlung entfernt und die Mischung auf Raumtemperatur erwärmt. Die Suspension wird über Nacht gerührt, bevor tropfenweise mit Methanol versetzt und eingeengt wird (der Vorgang wird zweimal wiederholt). Das Rohprodukt wird an Silicagel säulenfiltriert (Dichlormethan-Methanol-Triethylamin 85:10:5). Das erhaltene 3-Hydroxymethylbenzylamin wird in 150 ml 1,4-Dioxan gelöst, auf 0°C abgekühlt und mit 1.0 eq. Di-*tert*-butyldicarbonat (Boc-Anhydrid) und 1.1 eq. Natriumhydroxid in Form einer 1 molaren Lösung versetzt. Die Kühlung wird entfernt, und es wird 3 h bei Raumtemperatur nachgerührt, bevor eingeengt wird. Der Rückstand wird in Dichlormethan aufgenommen, mit 1N Salzsäure und ges. Natriumchlorid-Lösung gewaschen, über Magnesiumsulfat getrocknet, konzentriert und am Hochvakuum getrocknet. Ausbeute: 6.27 g (52.8% der Theorie);
MS (ESI): m/z (%) = 260 (M+Na, 17), 164 (100); HPLC (Methode 1): rt (%) = 6.73 (95).

Analog kann 4-(Hydroxymethyl)benzyl-*tert*-butyl-carbamat (vgl. *J. Chem. Soc. Perkin Trans 1*, **1999**, 1233) erhalten werden durch BH₃-Reduktion von 4-Cyanbenzoesäure in THF zu 4-Hydroxymethylbenzylamin und anschließende Umsetzung mit Di-*tert*-butyldicarbonat unter basischen Bedingungen.

### Beispiel 115

### [4-(2-Bromethyl)phenyl]methanol (vgl. WO 98/43956) erhalten durch BH₃₋Reduktion in THF von 4-(Brommethyl)-phenylessigsäure.

### Beispiel 116

**4-(Hydrozymethyl)phenethyl-*tert*-butyl-carbamat** ausgehend von 4-(Hydroxymethyl)phenethyl-benzylcarbamat (I. Fujii, R. A. Lerner, K. D. Janda, *J Amer. Chem. Soc.,* **1991**, 113, 8528) durch katalytische Hydrierung über 10% Palladium auf Kohle und nachfolgender Umsetzung mit Di-*tert*-butyldicarbonat unter basischen Bedingungen.

### Beispiel 117

**5-Brom-2-fluorbenzyl-*tert*-butylcarbamat** erhalten durch Umsetzung von 5-Brom-2-fluorbenzylamin (kann erhalten werden gemäß WO 97/16428) mit Di-*tert-*butyldicarbonat unter basischen Bedingungen.

### Beispiel 118

***Trans*-4-{[(*tert*-butoxycarbonyl)amino]methyl}cyclohezan-carbonsäure wird** ausgehend von *trans*-4-(Aminomethyl)-cyclohexancarbonsäure wie literaturbeschrieben erhalten *(J. Med Chem.,* **1986**, 29 4, 448-453).

### Beispiel 119

### tert-Butyl [4-trans-(hydrogymethyl)cyclohexyl)methylcarbamat

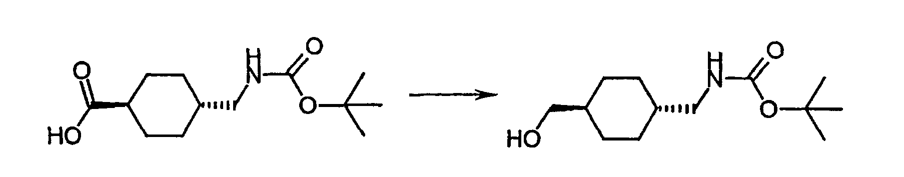

Zu einer eisgekühlten Lösung von 8.1 g (33.4 mmol) *trans-4-{[(tert-Butoxy*carbonyl)amino]methyl}cyclohexan-carbonsäure in 80 ml absolutem THF werden 33.5 ml (33.5 mmol) Boran-THF-Komplex als 1molare Lösung in THF getropft. Die Mischung wird auf Raumtemperatur erwärmt und weitere 3 h gerührt, bevor vorsichtig tropfenweise Methanol hinzugefügt wird. Die Mischung wird eingeengt, der Rückstand in Dichlormethan aufgenommen, mit 1N Natriumhydroxid-Lösung gewaschen, über Magnesiumsulfat getrocknet und konzentriert. Ausbeute 7.21 g (88.6% der Theorie);
MS (DCI, NH₄): m/z (%) = 261 (M+NH₄, 100).

### Beispiel 120

### cis-[3-(Hydroxymethyl)cyclohexyl]-acetonitril

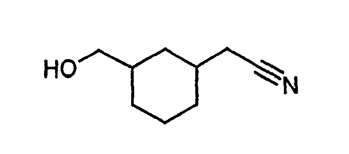

Zu einer Lösung von 600 mg (3.81 mmol) *cis*-1,3-Bishydroxymethylcyclohexan *[Bioorg. Med. Chem. Lett,* **1996**, 1589-1594] und 0.8 ml Triethylamin in 25 ml absolutem Dichlormethan werden bei 0°C 0.325 ml Methansulfonsäure (4.2 mmol) getropft. Nach 15 min bei 0°C wird auf Raumtemperatur erwärmt und 2 h gerührt, bevor im Vakuum eingeengt wird.

Vom öligen Rückstand werden 330 mg in 3 ml DMSO gelöst und mit 106 mg (1.63 mmol) Kaliumcyanid versetzt. Die Mischung wird 24 h bei 90°C gerührt. Nach Abkühlen wird mit Dichlormethan verdünnt, mit gesättigter Natriumhydrogencarbonat-Lösung und Natriumchlorid-Lösung gewaschen, über Magnesiumsulfat getrocknet und eingeengt. Ausbeute: 207 mg;
GC-MS 10.03 (100) mit m/z (%) =171 (M+NH₄, 100).

### Beispiel 121

### N-{2-[3-(Brommethyl)benzyl]-1,3-diozo-2,3-dihydro-1H-isoindol-4-yl}-5-chlor-2-thiophencarboxamid

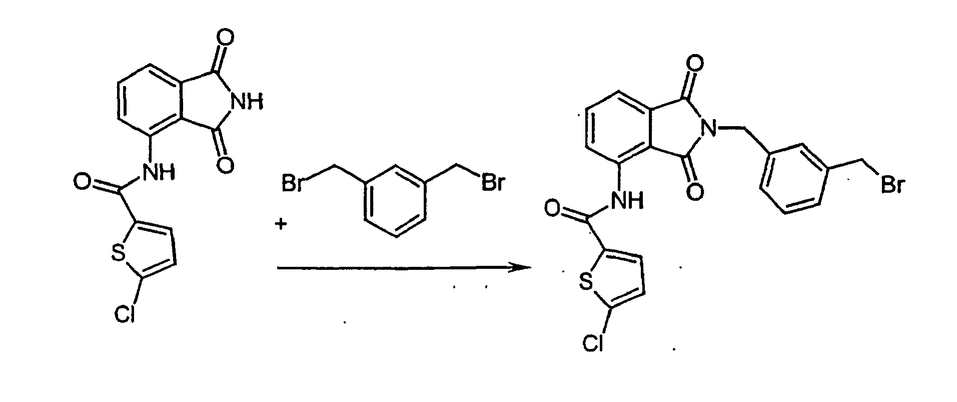

Zu einer Suspension von 2.91 g (9.5 mmol) 5-Chlor-*N*-(1,3-dioxo-2,3-dihydro-1*H-*isoindol-4-yl)-2-thiophencarboxamid in 30 ml DMF werden 1.31 g (9.5 mmol) Kaliumcarbonat gegeben. Die Suspension wird 30 min kräftig gerührt, bevor 8.78 g (33.3 mmol) α,α-Dibrom-*m*-Xylol, gelöst in 20 ml DMF auf einmal zugegeben werden. Die Suspension wird 5 h bei Raumtemperatur kräftig gerührt, bevor am Hochvakuum eingeengt wird. Der Rückstand wird in Dichlormethan aufgenommen, mit Wasser und ges. Natriumchlorid-Lösung gewaschen, über Magnesiumsulfat getrocknet und eingeengt. Das Rohprodukt wird mit Ether verrieben und filtriert. Der Feststoff wird weiter durch Säulenfiltration an Silicagel (Dichlormethan) gereinigt. Ausbeute: 2.94 g (63.2% der Theorie);
MS (EI, pos): m/z (%) = 488/490 (M⁺, 30); HPLC (Methode 1): rt (%) = 3.62 (100).

### Allgemeine Arbeitsvorschrift (I) zur Darstellung von Aminderivaten über Alkylierungen ausgehend von N-{2-[3-(Brommethyl)benzyl]-1,3-diozo-2,3-dihydro-1H-isoindol-4-yl}-5-chlor-2-thiophencarboxamid

Zu einer Lösung oder Suspension von *N*-{2-[3-(Brommethyl)benzyl]-1,3-dioxo-2,3-dihydro-1*H* isoindol-4-yl}-5-chlor-2-thiophencarboxamid in 1,2-Dichlorethan oder 1,4-Dioxan werden 3 bis 5 eq. Amin-Derivat gegeben. Die Mischung wird 2 bis 10 h bei Raumtemperatur oder 2 bis 5h bei 80°C gerührt, bevor nach Abkühlen auf Raumtemperatur mit Dichlormethan verdünnt und filtriert wird. Der Feststoff wird am Hochvakuum getrocknet. Aus dem so erhaltenen Hydrobromid des Produkts kann das freie Aminderivat gewonnen werden: Nach Zugabe von größeren Mengen von Dichormethan/Methanol-Gemischen wird die organische Phase mit ges. Natriumhydrogencarbonat-Lösung gewaschen, über Magnesiumsulfat getrocknet und eingeengt. Der Rückstand wird aus Dichlormethan-Ether Gemischen kristallisiert.

Nach allgemeiner Arbeitsvorschrift I können folgende Amin-/Hydrazinderivate ausgehend von *N*- {2-[3-(Brommethyl)benzyl]-1,3-dioxo-2,3-dihydro-1*H*-isoindol-4-yl}-5-chlor-2-thiophencarboxamid erhalten werden:

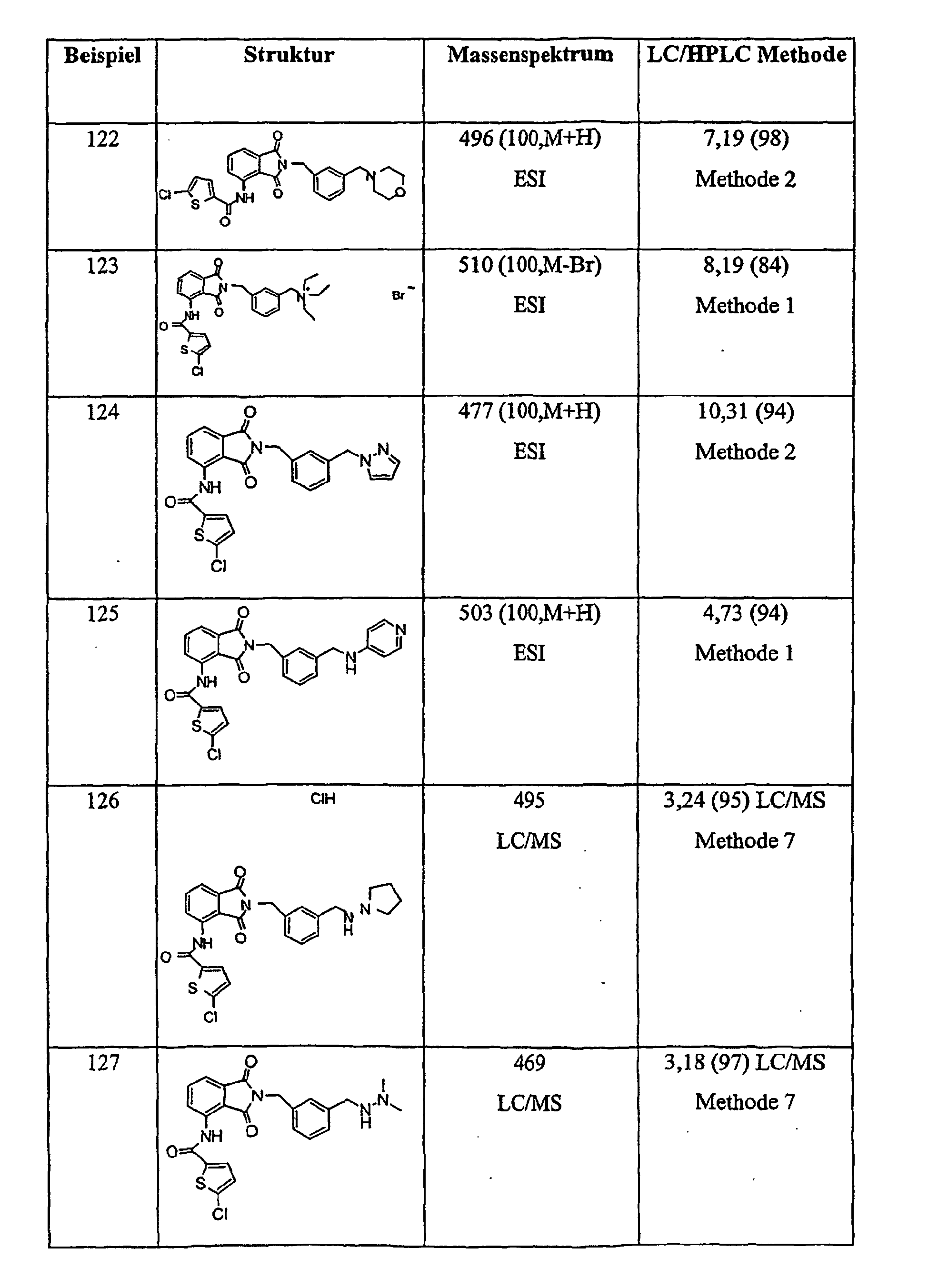

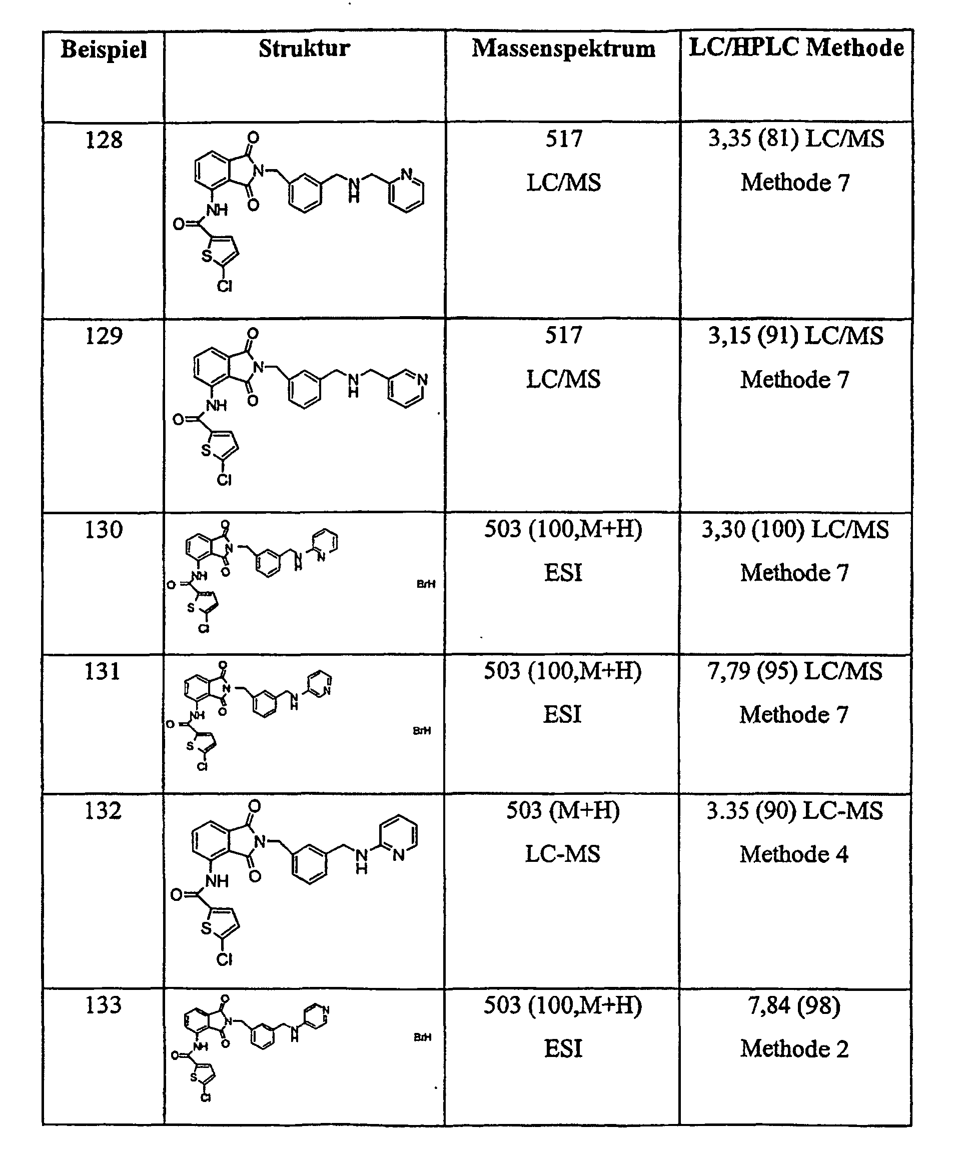

### Beispiel 134

### tert-Butyl-3-[(4-{[(5-chlor-2-thienyl)carbonyl]amino}-1,3-dioxo-1,3-dihydro-2H isoindol-2-yl)methyl]benzylcarbamat

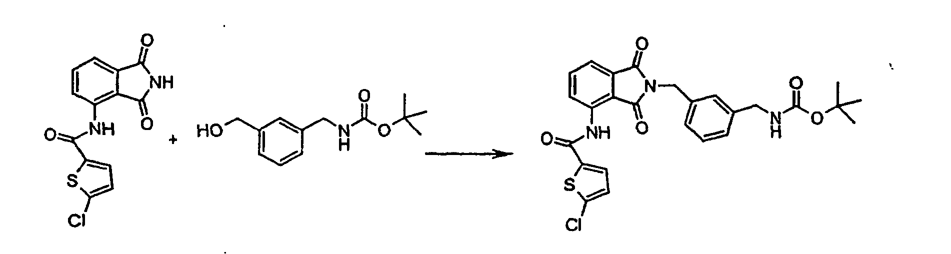

Zu einer wassergekühlten Mischung aus 3.37 g (11 mmol) 5-Chlor-*N*-(1,3-dioxo-2,3-dihydro-1*H*-isoindol-4-yl)-2-thiophencarboxamid, 3.26 g (13.8 mmol) *tert*-Butyl-3-(hydroxymethyl)benzylcarbamat und 4.04 g (15.4 mmol) Triphenylphosphin in 60 ml absolutem THF werden 2.41 ml (15.4 mmol) Azodicarbonsäurediethylester (DEAD) getropft. Die Reaktionsmischung wird 90 min kräftig bei Raumtemperatur gerührt, bevor auf ca. 30 ml Volumen eingeengt wird. Der Feststoff wird mit 300 ml Ether versetzt und filtriert. Aus der Mutterlauge wird eine 2. Charge durch erneute Kristallisation erhalten. Das Produkt wird am Hochvakuum getrocknet. Alternativ kann das Produkt durch Säulenchromatographie an Silicagel (Dichlormethan/Methanol-Gemisch oder Cyclohexan/Essigester-Gemisch) gereinigt werden. Ausbeute: 4.79 g (82.8% der Theorie);
MS (ESI): m/z (%) = 526 (M+H, 20); HPLC (Methode 1): rt (%) = 9.69 (98.6).

Auf analoge Weise können ausgehend von 5-Chlor-*N*-(1,3-dioxo-2,3-dihydro-1*H-*isoindol-4-yl)-2-thiophencarboxamid und entsprechenden Alkoholen folgende Verbindungen erhalten werden:

### Beispiel 135

### N-[2-(3-Aminobenzyl)-1,3-dioxo-2,3-dihydro-1H-isoindol-4-yl]-5-chlor-2-thiophencarboxamid

MS (DCI, NH₃): m/z (%) = 429 (M+NH₄, 100), 412 (M+H, 20); HPLC (Methode 1): rt(%)= 7.88 (97.3).

### Beispiel 136

### N-{2-[4-(Brommethyl)phenethyl]-1,3-dioxo-2,3-dihydro-1H-isoindol-4-yl}-5-chlor-2-thiophencarboxamid

MS (DCI, NH₃): m/z (%) = 519/522 (M+NH₄, 40), 442 (40), ); HPLC (Methode 1): rt (%) = 8.85 (42), 10.19 (42).

### Beispiel 137

### tert-Butyl-{4-trans-[(4-{[(5-chlor-2-thienyl)carbonyl]amino}-1,3-dioxo-1,3-dihydro-2H-isoindol-2-yl)methyl]cyclohexyl}methyl-carbamat

MS (ESI neg): m/z (%) = 530 ([M-H]⁻, 100); HPLC (Methode 1): rt (%) = 5.85 (98).

### Beispiel 138

### 5-Chlor-N-(2-{[cis-3-(cyanomethyl)cyclohexyl]methyl}-1,3-dioxo-2,3-dihydro-1H-isoindol-4-yl)-2-thiophencarboxamid

MS (DCI, NH₄): m/z (%) = 464 (M+Na, 42), 442 (M+H, 40); HPLC (Methode 2): rt (%) = 5.49 (100).

### Beispiel 139

### 5-Chlor-N-(1,3-dioxo-2-{4-[(4-pyridinylamino)methyl]phenethyl}-2,3-dihydro-1H-isoindol-4-yl)-2-thiophencarboxamid

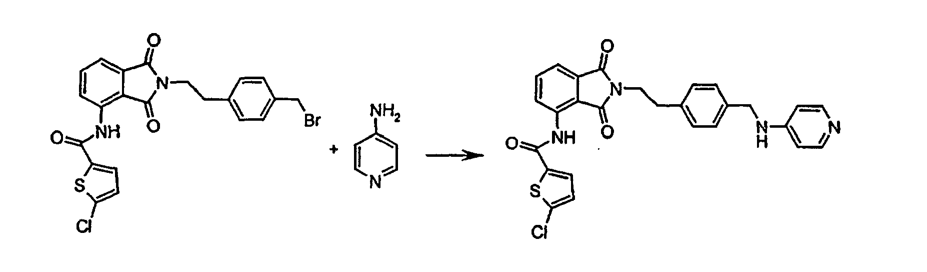

Zu einer Lösung von 37.4 mg (0.4 mmol) 4-Aminopyridin in 4 ml 1,2-Dichlorethan werden portionsweise 50 mg (0.1 mmol) *N*-{2-[4-(Brommethyl)phenethyl]-1,3-dioxo-2,3-dihydro-1*H*-isoindol-4-yl} -5-chlor-2-thiophencarboxamid eingetragen. Die Mischung wird über Nacht bei Raumtemperatur gerührt, bevor mit Dichlormethan/Methanol (98:2) verdünnt, mit ges. Natriumhydrogencarbonat-Lösung gewaschen, über Magnesiumsulfat getrocknet und eingeengt wird. Der Rückstand wird mit Dichlormethan/Ether kristallisiert, der Feststoff abgesaugt und am Hochvakuum getrocknet. Ausbeute: 25.1 mg (37.2% der Theorie); MS (ESI): m/z (%) = 517 (M+H, 100); ); LC-MS (Methode 7): rt (%) = 3.42 (77).

### Beispiel 140

### N-{2-[3-(Aminomethyl)benzyl]-1,3-dioxo-2,3-dihydro-1H-isoindol-4-yl}-5-chlor-2-thiophencarboxamid

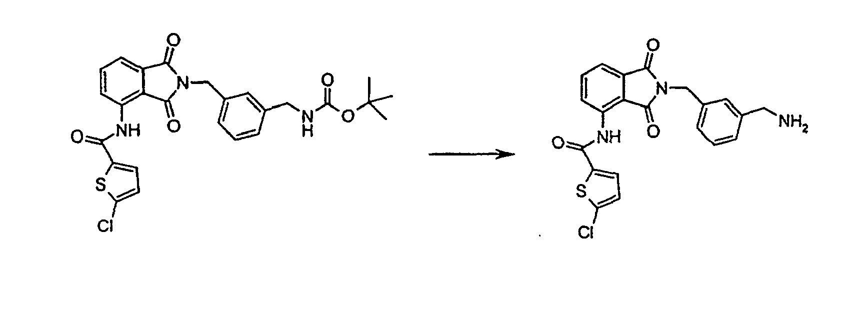

Zu einer eisgekühlten Mischung von 3.95 g (7.5 mmol) *tert*-Butyl-3-[(4-{[(5-chlor-2-thienyl)carbonyl]amino}-1,3-dioxo-1,3-dihydro-2*H*-isoindol-2-yl)methyl]benzylcarbamat in 40 ml Chloroform werden 40 ml einer Mischung aus TFA und Wasser (9:1) getropft. Die Eiskühlung wird entfernt und die Lösung wird 2 h bei Raumtemperatur gerührt, bevor eingeengt wird. Die Rückstand wird in Dichlormethan/Methanol (98:2) aufgenommen und die Mischung mit ges. Natriumhydrogencarbonat-Lösung gewaschen, über Magnesiumsulfat getrocknet und eingeengt. Ausbeute: 3.22 g (97.8% der Theorie);

MS (ESI): m/z (%) = 426 (M+H, 95), 409 (100); HPLC (Methode 1): rt (%) = 7.98 (98.1).

Auf analoge Weise können ausgehend von entsprechenden Boc-geschützten Vorläufern folgende Verbindungen erhalten werden:

### Beispiel 141

### N-{2-[4-(Aminomethyl)benzyl]-1,3-diozo-2,3-dihydro-1H-isoindol-4-yl}-5-chlor-2-thiophencarboxamid

MS (DCI, NH₃): m/z (%) = 426 (M+H, 100); HPLC (Methode 1): rt (%) = 8.05 (98.3).

### Beispiel 142

### N-{2-(4-(2-Aminoethyl)benzyl]-1,3-dioxo-2,3-dihydro-1H-isoindol-4-yl}-5-chlor-2-thiophenecarboxamid

MS (ESI): m/z (%) = 440 (M+H, 100); HPLC (Methode 1): rt (%) = 4.60 (94.2).

### Beispiel 143

### N-[2-(3-{[(Aminocarbonyl)amino]methyl}benzyl)-1,3-dioxo-2,3-dihydro-1H-isoindol-4-yl]-5-chlor-2-thiophencarboxamid

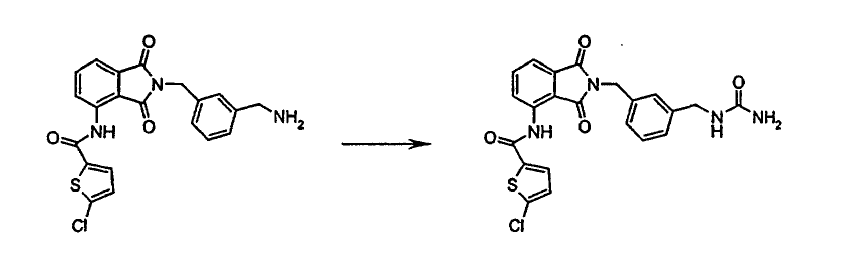

Zu einer Mischung von 50 mg (0.12 mmol) *N*-{2-[3-(Aminomethyl)benzyl]-1,3-dioxo-2,3-dihydro-1*H*-isoindol-4-yl}-5-chlor-2-thiophencarboxamid in 2 ml trockenem Dichlormethan werden 0.16 ml (1.2 mmol) Trimethylsilylisocyanat getropft. Die Suspension wird über Nacht bei Raumtemperatur gerührt, bevor eingeengt und am Hochvakuum getrocknet wird. Der Rückstand wird mit Ether versetzt und das Produkt als Feststoff durch Filtration erhalten. Ausbeute: 50 mg (90.8% der Theorie);

MS (DCI, NH₃): m/z (%) = 469 (M+H, 33); 426 (100); HPLC (Methode 1): rt (%) = 8.50 (95.8).

Auf analoge Weise können ausgehend von entsprechenden Amin-Derivaten mit Trimethylisocyanat folgende Verbindungen erhalten werden:

### Beispiel 144

### N-[2-(4-{[(Aminocarbonyl)amino]methyl}benzyl)-1,3-dioxo-2,3-dihydro-1H-isoindol-4-yl]-5-chlor-2-thiophencarboxamid

MS (DCI, NH₃): m/z (%) = 486 (M+NH₄, 100), 426 (95) ; HPLC (Methode 1): rt (%) = 8.51 (98.5).

### Beispiel 147

### N-[2-(4-{2-[(Aminocarbonyl)amino]ethyl}benzyl)-1,3-dioxo-2,3-dihydro-1H-isoindol-4-yl]-5-chlor-2-thiophencarboacamid

MS (DCI, NH₃): m/z (%) = 483 (M+H, 83), 440 (100); HPLC (Methode 1): rt (%) = 4.76 (98.8).

### Allgemeine Arbeitsvorschrift (II-a) zur Darstellung von Harnstoffderivaten ausgehend von N-{2-[3-(Aminomethyl)benzyl]-1,3-dioxo-2,3-dihydro-1H-isoindol-4-yl}-5-chlor-2-thiophencarboxamid und Isocyanaten

Zu einer Lösung von 2 bis 3 eq. Isocyanat in absolutem Dichlormethan (ca. 0.4 mol/l) wird bei Raumtemperatur eine Lösung von 1 eq. Aminderivat in absolutem Dichlormethan (ca. 0.05 mol/l) getropft. Die Mischung wird 30 min bis 14 h bei Raumtemperatur gerührt, bevor mit Dichlormethan/Ether-Gemischen verdünnt wird. Der Kristallbrei wird abgesaugt und am Hochvakuum getrocknet. Gegebenenfalls erfolgt eine Nachreinigung des Produkts durch Kristallisation aus Dichlormethan/Ether oder Säulenfiltration an Silicagel (Dichlormethan/Methanol-Gemische).

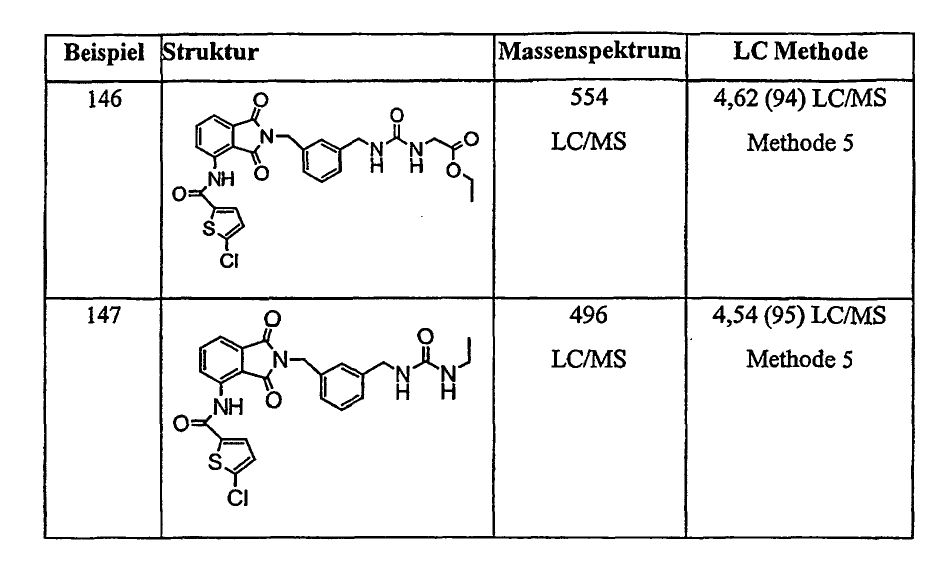

### Allgemeine Arbeitsvorschrift (II-b) zur Darstellung von Harnstoffderivaten ausgehend von N-[2-(3-Aminobenzyl)-1,3-dioxo-2,3-dihydro-1H-isoindol-4 yl]-5-chlor-2-thiophen-carboxamid und Isocyanaten

Zu einer Suspension des Anilins in absolutem Dichlormethan werden 3 bis 15 eq. Isocyanat gegeben. Die Mischung wird 14 bis 48 h bei Raumtemperatur gerührt, bevor nach Zugabe von Ether filtriert wird. Das Rohprodukt wird durch Kristallisation mit Dichlormethan/Ether-Gemischen oder durch präparative Dünnschicht-Chromatographie (Dichlormethan-Methanol-Gemische) gereinigt.

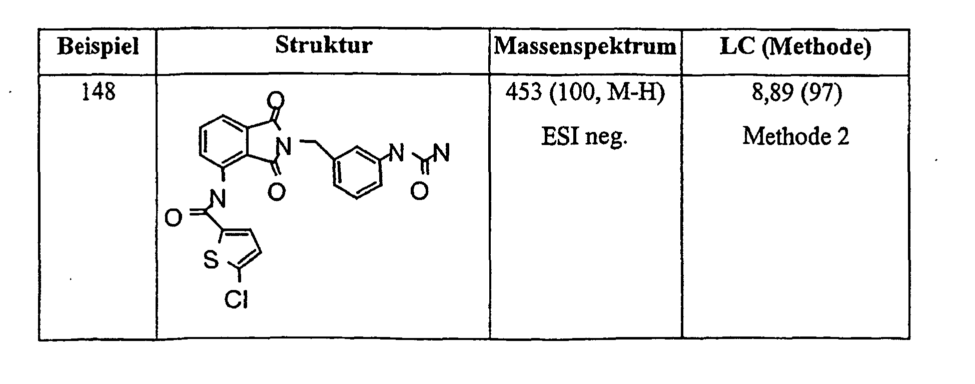

### Beispiel 149

### 5-Chlor-N-{2-[3-(isocyanatomethyl)benzyl]-1,3-dioxo-2,3-dihydro-1H-isoindol-4-yl}-2-thiophenearboxamid

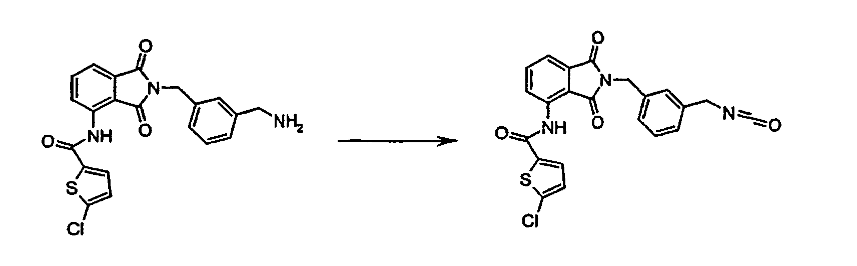

Zu einer eisgekühlten Lösung von 0.12 ml (0.96 mmol) Diphosgen in 7.5 ml Dichlormethan tropft man eine Lösung aus 430 mg (1.0 mmol) *N*-{2-[3-(Aminomethyl)benzyl]-1,3-dioxo-2,3-dihydro-1*H*-isoindol-4-yl}-5-chlor-2-thiophencarboxamid und 433 mg (2.0 mmol) 1,8-Bis-(dimethylamino)-naphthalin in 222 ml Dichlormethan. Nach 5 min bei 0°C wird die Mischung auf Raumtemperatur erwärmt und 35 min gerührt, bevor 40 ml Ether zugesetzt werden. Es wird vom Unlöslichen abfiltriert und das Filtrat eingeengt und am Hochvakuum getrocknet. Der erhaltene Feststoff wird ohne weitere Aufreinigung unverzüglich weiter umgesetzt. Ausbeute: 454 mg (99.5% der Theorie).

### Beispiel 150

Auf analoge Weise kann ausgehend von *N*-{2-[4-(Aminomethyl)benzyl]-1,3-dioxo-2,3-dihydro-1*H*-isoindol-4-yl}-5-chlor-2-thiophencarboxamid erhalten werden:

### 5-Chlor-N-{2-[4-(isocyanatomethyl)benzyl]-1,3-dioxo-2,3-dihydro-1H-isoindol-4-yl}-2-thiophencarboxamid

MS (DCI, NH₃): m/z (%) = 469 (M+NH₄, 30).

### Allgemeine Arbeitsvorschrift (III-a) zur Darstellung von Harnstoffderivaten ausgehend von 5-Chlor-N-{2-[3-(isocyanatomethyl)benzyl]-1,3-dioxo-2,3-dihy-dro-1H-isoindol-4-yl}-2-thiophencarboxamid

Eine Lösung des Isocyanats (1 eq.) in einem Gemisch von absolutem Dichlormethan und DMF wird zu einer Lösung von 2 bis 5 eq. des entsprechenden Amin- bzw. Hydrazin-Derivates in absolutem Dichlormethan getropft. Liegen Amin- bzw. Hydrazinderivat als Hydrochloride vor, werden zusätzlich ca. 5 eq. Triethylamin hinzugefügt. Die Mischung wird ca. 16 h bei Raumtemperatur gerührt. Nach Zugabe von Dichlormethan/Ether-Gemischen wird das Rohprodukt als Feststoff durch Filtration gewonnen. Das Rohprodukt wird durch Kristallisation mit Dichlormethan/Ether-Gemischen oder durch Chromatographie an Silicagel (Dichlormethan-Methanol-Gemische) gereinigt.

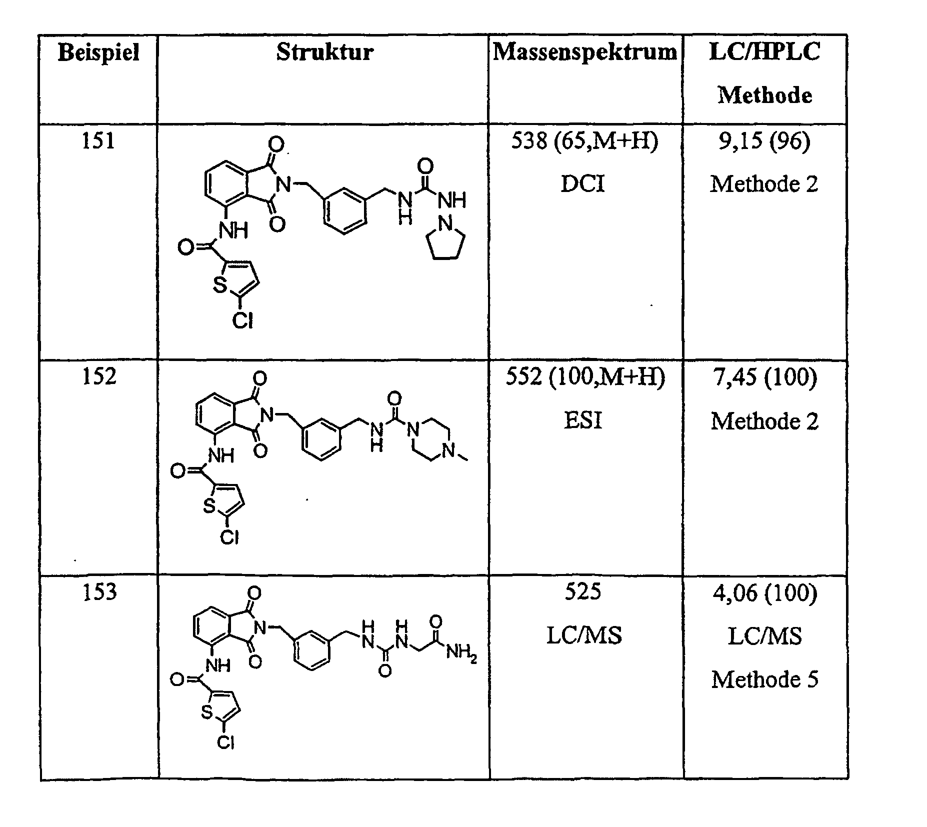

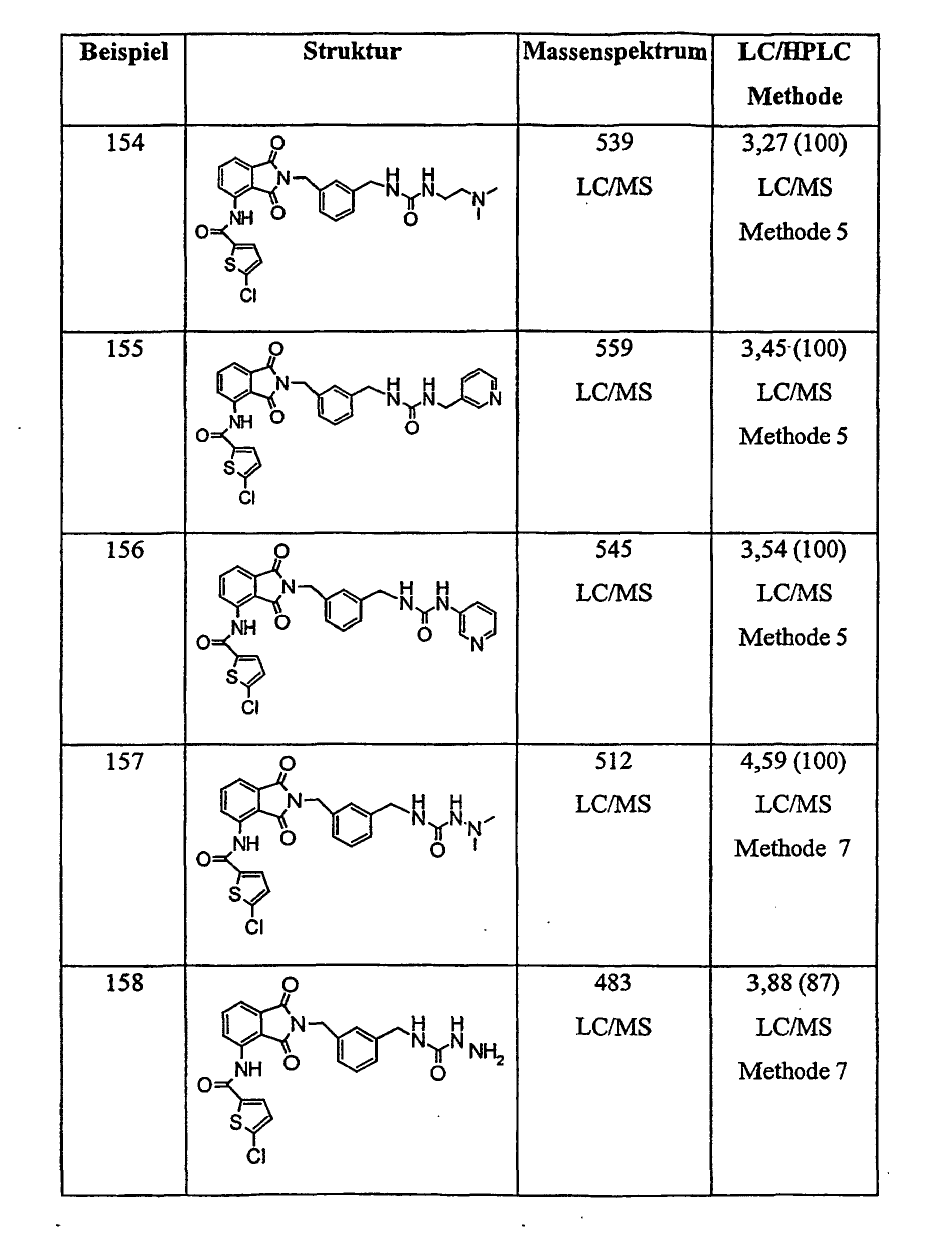

### Allgemeine Arbeitsvorschrift (III-b) zur Darstellung von Harnstoffderivaten ausgehend von 5-Chlor-N-{2-[4-(isocyanatomethyl)benzyl]-1,3-dioxo-2,3-dihydro-1H-isoindol-4-yl}-2-thiophenearboxamid

Eine Lösung des Isocyanats (1 eq.) in einem Gemisch von absolutem Dichlormethan und DMF wird zu einer Lösung von 2 bis 5 eq. des entsprechenden Amin- bzw. Hydrazin-Derivates in absolutem Dichlormethan getropft. Liegen Amin- bzw. Hydrazinderivat als Hydrochloride vor, werden zusätzlich ca. 5 eq. Triethylamin hinzugefiigt. Die Mischung wird ca. 16 h bei Raumtemperatur gerührt. Nach Zugabe von Dichlormethan/Ether-Gemischen wird das Produkt als Feststoff durch Filtration gewonnen und am Hochvakuum getrocknet. Gegebenenfalls wird Produkt durch Kristallisation mit Dichlormethan/Ether-Gemischen oder durch Chromatographie an Silicagel (Dichlormethan-Methanol-Gemische) weiter aufgereinigt.

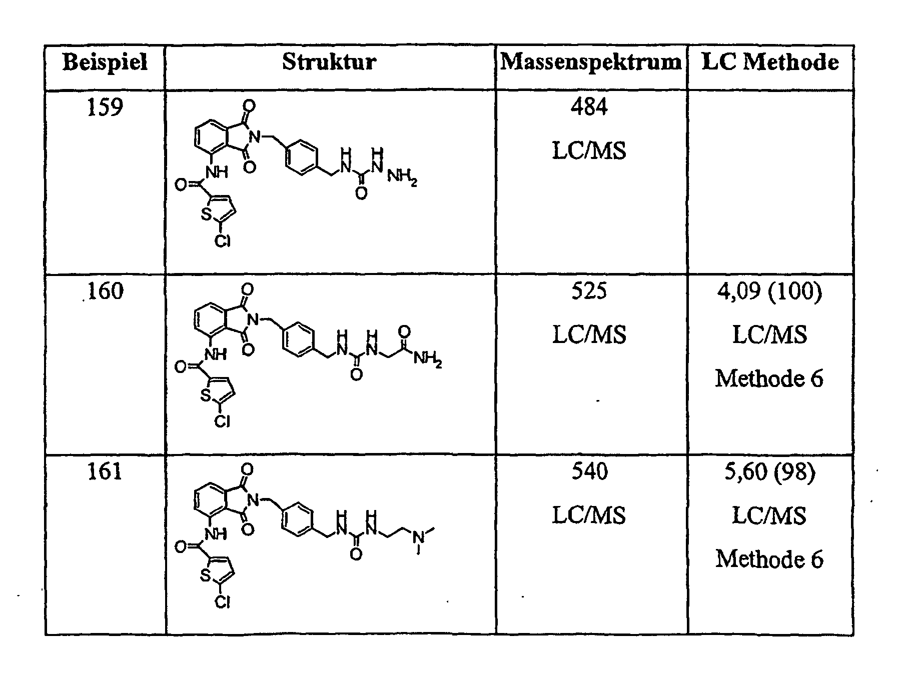

### Beispiel 162

### N-(2-{4-[2-(Acetylamino)ethyl]benzyl}-1,3-dioxo-2,3-dihydro-1H-isoindol-4-yl)-5-chlor-2-thiophencarboxamid

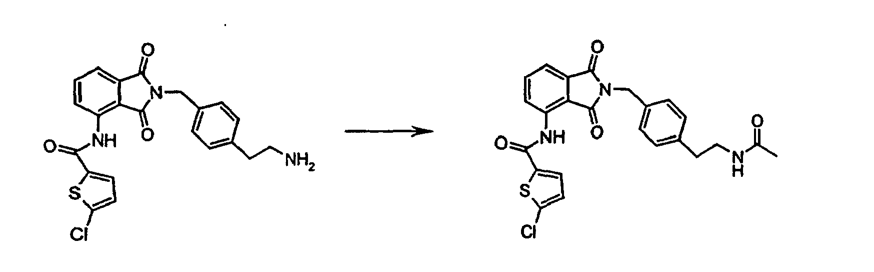

Zu einer Lösung von 40 mg (0.09 mmol) *N-*{2-[4-(2-Aminoethyl)benzyl]-1,3-dioxo-2,3-dihydro-1*H*-isoindol-4-yl}-5-chlor-2-thiophencarboxamid in 2 ml absolutem THF werden 0.04 ml (0.46 mmol) Pyridin und 0.02 ml (0.18 mmol) Essigsäureanhydrid getropft. Die Lösung wird nach 2 h bei Raumtemperatur konzentriert und der Rückstand in Essigester aufgenommen. Man wäscht zweimal mit Wasser, trocknet über Magnesiumsulfat, engt ein und trocknet am Hochvakuum. Ausbeute: 35.2 mg (80.3% der Theorie);

MS (DCI, NH₃): m/z (%) = 499 (M+NH₄, 100); HPLC (Methode 1): rt (%) = 490 (100).

### Beispiel 163

### tert-Butyl-3-[(4-{[(5-chlor-2-thienyl)carbonyl]amino}-3-hydroxy-1-oxo-1,3-dihydro-2H-isoindol-2-yl)methyl]benzylcarbamat und tert-Butyl-3-[(4-{[(5-chlor-2-thienyl)carbonyl]amino}-1-hydroxy-3-oxo-1,3-dihydro-2H isoindol-2-yl)methyl]benzylcarbamat

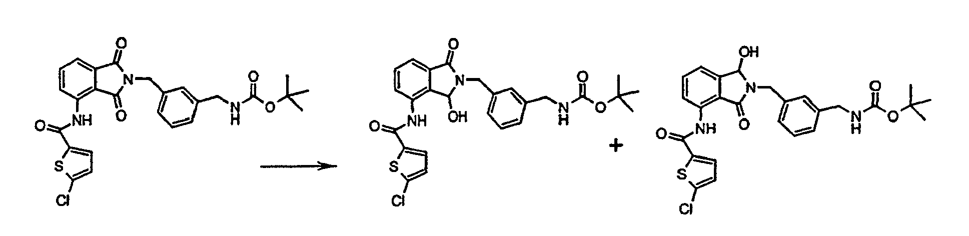

Zu einer Lösung von 620 mg (1.18 mmol) *tert*-Butyl-3-[(4-{[(5-chlor-2-thienyl)carbonyl]amino}-1,3-dioxo-1,3-dihydro-2*H* isoindol-2-yl)methyl]benzylcarbamat in 25 ml Methanol und 25 ml Dichlormethan werden portionsweise 55 mg (1.41 mmol) Natriumborhydrid gegeben. Die Lösung wird 3 h bei Raumtemperatur gerührt, bevor vorsichtig mit 1N Salzsäure-Lösung leicht angesäuert wird. Es wird Wasser zugesetzt und zweimal mit Dichlormethan extrahiert. Die vereinigten organischen Phasen werden mit Magnesiumsulfat getrocknet und eingeengt. Das Produktgemisch wird durch Säulenchromatographie an Silicagel (Dichlormethan/Methanol 98:2) aufgetrennt. Die Zuordnung der isomeren Produkte erfolgt durch hochaufgelöste NMR-Spektroskopie. Erhalten werden:

220 mg ***tert*-Butyl-3-[(4-{[(5-chlor-2-thienyl)carbonyl]amino}-3-hydroxy-1-oxo-1,3-dihydro-2*H*-isoindol-2-yl)methyl]benzylcarbamat** (66% Reinheit, 23.2% der Theorie). MS (LC-MS): m/z (%) = 510 (M-H₂O, 100); HPLC (Methode 1): rt (%) = 8.45 (66%).

300 mg ***tert*-Butyl-3-[(4-{[(5-chlor-2-thienyl)carbonyl]amino}-1-hydroxy-3-oxo-1,3-dihydro-2*H*-isoindol-2-yl)methyl]benzylcarbamat** (45.9% der Theorie). MS (LC-MS m/z (%) = 510 (M-H₂O, 100); HPLC (Methode 1): rt (%) = 9.09 (95).

### Beispiel 164

### N-{2-[3-(Aminomethyl)benzyl]-1-oxo-2,3-dihydro-1H-isoindol-4-yl}-5-chlor-2-thiophencarboxamid

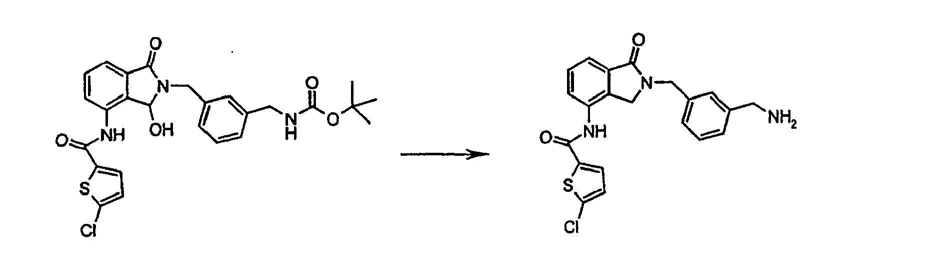

Zu einer Lösung von 181 mg (66% Reinheit, 0.22 mmol) *tert*-Butyl-3-[(4-{[(5-chlor-2-thienyl)carbonyl]amino)-3-hydroxy-1-oxo-1,3-dihydro-2*H*-isoindol-2-yl)methyl]-benzylcarbamat in 4.5 ml Dichlormethan werden 0.21 ml (2.7 mmol) TFA und 0.07ml (0.45 mmol) Triethylsilan getropft. Die Lösung wird über Nacht bei Raumtemperatur gerührt, bevor eingeengt und am Hochvakuum getrocknet wird. Der Rückstand wird in Dichlormethan/Methanol aufgenommen, mit 1N Natriumhydroxid-Lösung gewaschen, über Magnesiumsulfat getrocknet und eingeengt. Der Rückstand wird mit Ether versetzt und abgesaugt, man trocknet das Produkt am Hochvakuum. Ausbeute: 84 mg (90.7% der Theorie);
MS (DCI, NH₃): m/z (%) = 412 (M+H,100); HPLC (Methode 2): rt (%) = 6.25 (80).

### Beispiel 165

Auf analoge Art lässt sich ***N*-{2-[3-(Aminomethyl)benzyl]-3-oxo-2,3-dihydro-1*H-*isoindol-4-yl}-5-chlor-2-thiophencarboxamid** ausgehend von *tert*-Butyl-3-[(4-{[(5-chlor-2-thienyl)carbonyl]amino}-1-hydroxy-3-oxo-1,3-dihydro-2*H*-isoindol-2-yl)methyl]benzylcarbamat darstellen.

MS (ESI): m/z (%) = 412 (M+H, 100); HPLC (Methode 2): rt (%) = 7.17 (87).

### Beispiel 166

### N-[2-(3-{[(Aminocarbonyl)amino]methyl}benzyl)-1-oxo-2,3-dihydro-1H-isoindol-4-yl]-5-chlor-2-thiophencarboxamid

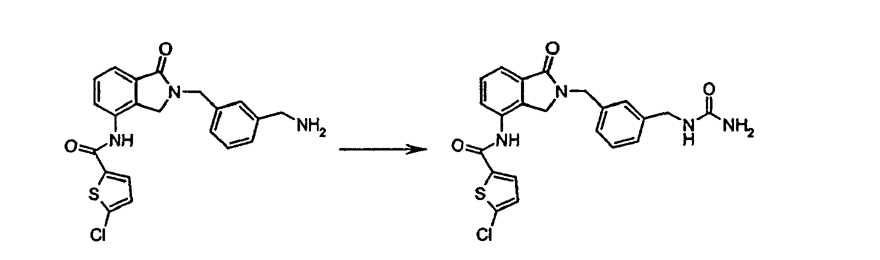

Zu einer Lösung von 50 mg (0.12 mmol) *N*-{2-[3-(Aminomethyl)benzyl)-1-oxo-2,3-dihydro-1*H*-isoindol-4-yl}-5-chlor-2-thiophencarboxamid in 2 ml absolutem Dichlormethan werden 0.08 ml (0.61 mmol) Trimethylsilylisocyanat getropft. Die Mischung wird über Nacht bei Raumtemperatur gerührt, bevor Ether zugegeben wird. Der Feststoff wird abgesaugt, mit Ether nachgewaschen und am Hochvakuum getrocknet. Ausbeute: 44.6 mg (64.6% der Theorie);

MS (DCI, NH₃): m/z (%) = 427 (M+NH₄, 53), 412 (100); HPLC (Methode 2): rt (%) = 7.13 (80).

### Beispiel 167

Auf analoge Art lässt sich ***N*-[2-(3-{[(Aminocarbonyl)amino]methyl}benzyl)-3-oxo-2,3-dihydro-1*H*-isoindol-4-yl]-5-chlor-2-thiophencarboxamid** ausgehend von *N*-{2-[3-(Aminomethyl)benzyl]-3-oxo-2,3-dihydro-1*H*-isoindol-4-yl}-5-chlor-2-thiophencarboxamid darstellen.

MS (DCI, NH₃): m/z (%) = 472 (M+NH₄,100) 455 (M+H, 80); HPLC (Methode 2): rt (%) = 8.52 (85).

### Allgemeine Arbeitsvorschrift (IV-a) zur Reduktion von Phthalimiden zu substituierten 5-Chlor-N-(3-hydrozy-1-oxo-2,3-dihydro-1H-isoindol-4-yl)-2-thiophencarboxamid Derivaten und den regioisomeren 5-Chlor-N-(1-hydrozy-3-oxo-2,3-dihydro-1H-isoindol-4-yl)-2-thiophen-carboxamid Derivaten

Eine Suspension entsprechend substituierter Phthalimide (1 eq.) in einem Gemisch aus Methanol und Dichlormethan (1:1) wird portionsweise bei 0°C mit Natriumborhydrid (1.5 bis 2.5 eq.) versetzt. Nach Ende der Zugabe wird 1.5 bis 3 h bei Raumtemperatur gerührt, bevor die Mischung mit 1 N Salzsäure-Lösung leicht angesäuert und mit Dichlormethan verdünnt wird. Es wird mit ges. Natriumhydrogencarbonat-Lösung und ges. Natriumchlorid-Lösung gewaschen, über Magnesiumsulfat getrocknet und konzentriert. Das Produktgemisch kann entweder durch Säulenchromatographie an Silicagel (Dichlorrnethanfmethanol-Gemische) oder durch präparative RP-HPLC getrennt und gereinigt werden. Bei längerem Kontakt von Hydroxy-oxo-2,3-dihydro-1*H*-isoindol-4-yl Derivaten mit Methanol und Silicagel kann eine Umsetzung zu entsprechend Methoxy-substituierten Derivaten erfolgen.

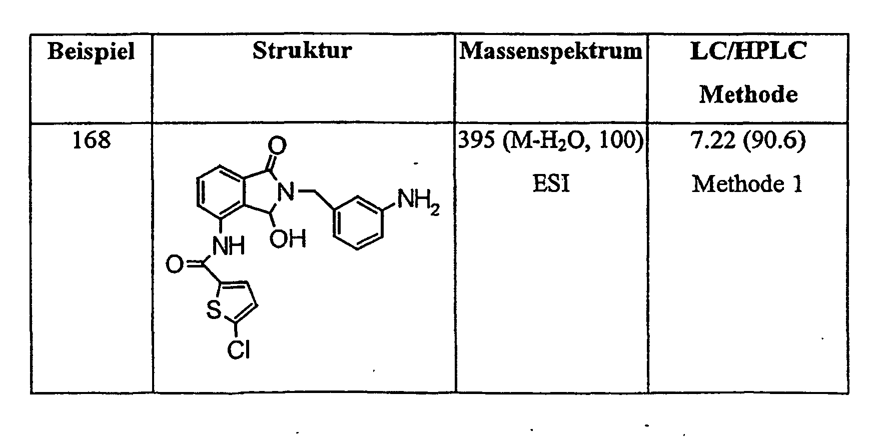

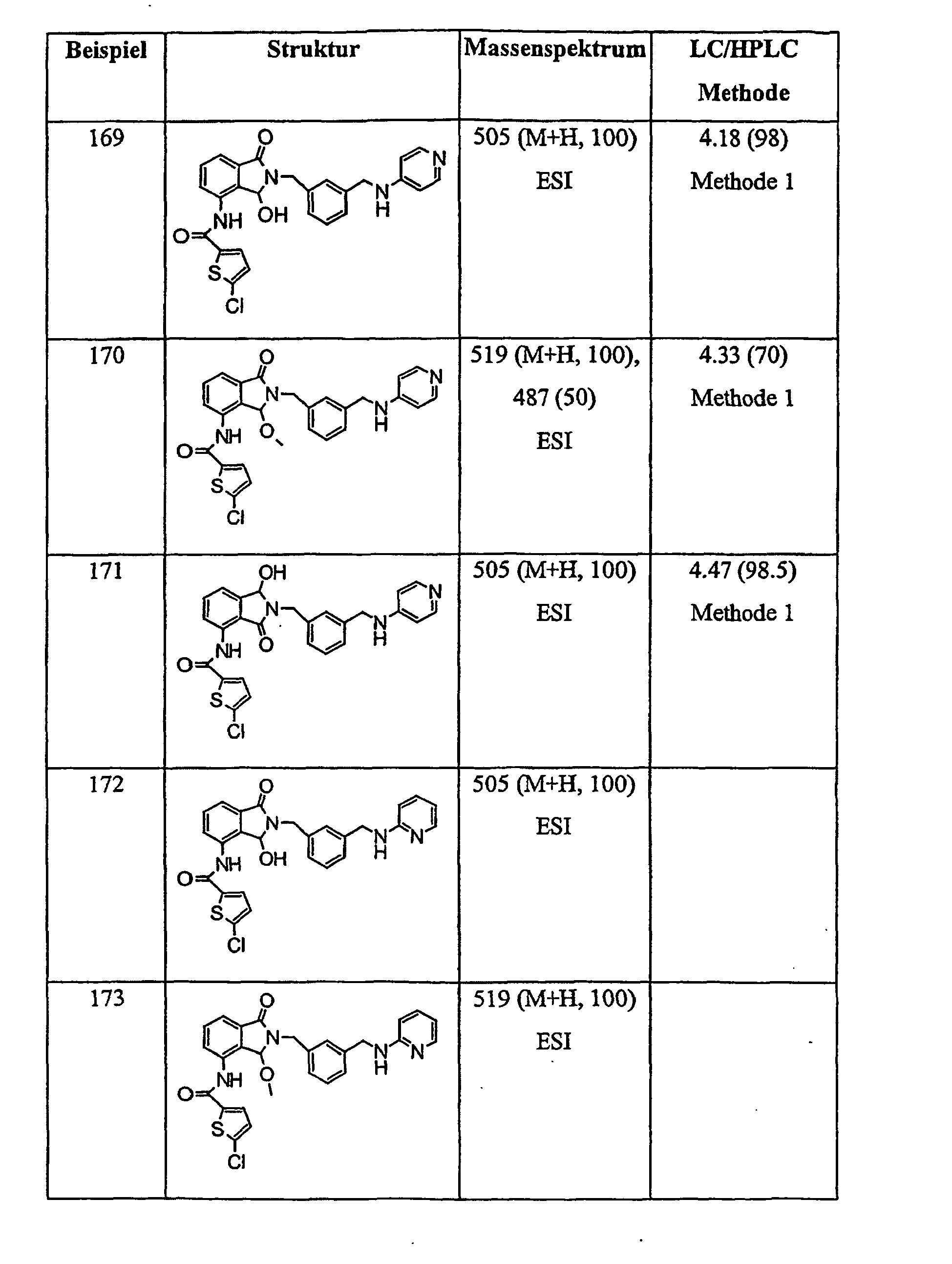

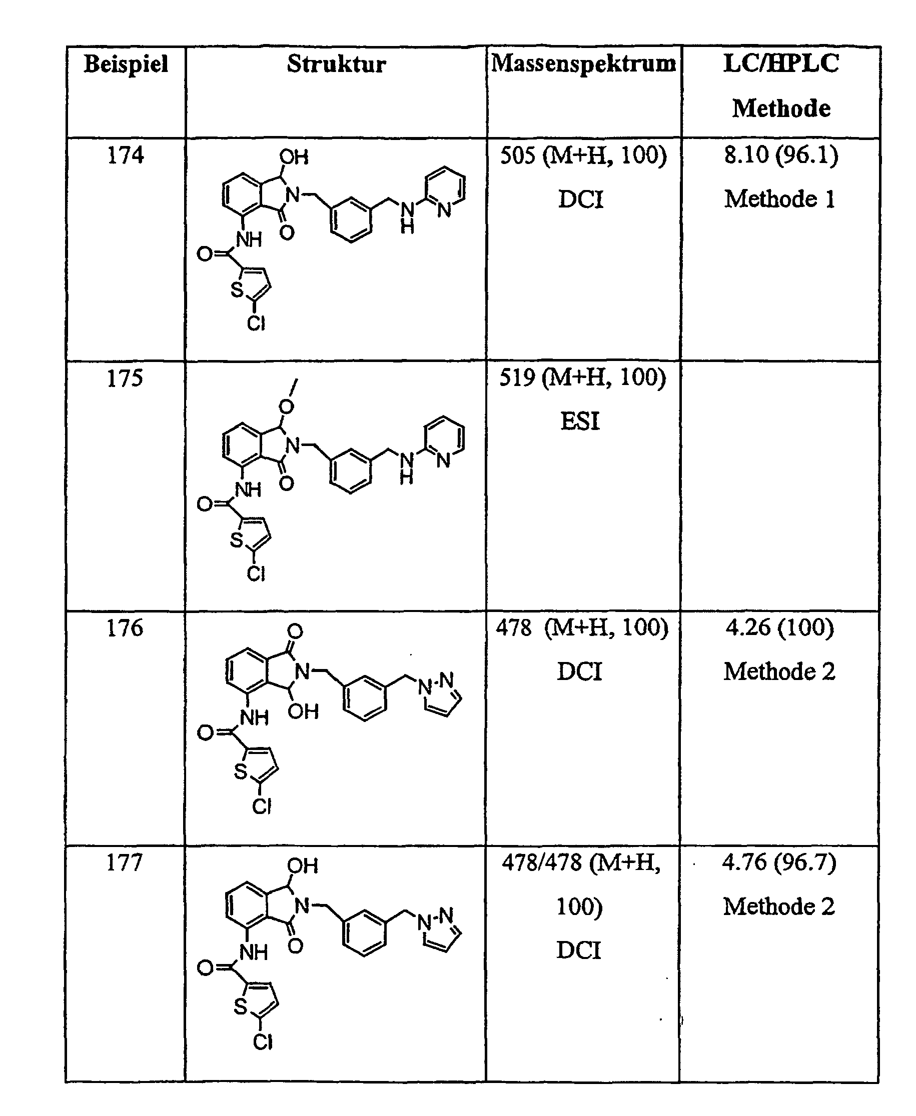

### Allgemeine Arbeitsvorschrift (IV-b) zur Darstellung von substituierten 5-Chlor-N-(1-oxo-2,3-dihydro-1H-isoindol-4-yl)-2-thiophencarboxamid Derivaten und den regioisomeren 5-Chlor-N-(3-oxo-2,3-dihydro-1H-isoindol-4-yl)-2-thiophencarboxamid Derivaten ausgehend von entsprechenden Hydroxy-oxo-2,3-dihydro-1H-isoindol-4-yl Derivaten

Zu einer Lösung eines Hydroxy-oxo-2,3-dihydro-1*H*-isoindol-4-yl Derivats in Dichlormethan (ca. 0.1 mol/l) werden bei Raumtemperatur tropfenweise TFA (ca. 12 eq.) und Triethylsilan (1.5 bis 2 eq.) gegeben. Die Mischung wird 3 bis 16 h bei Raumtemperatur gerührt, bevor zur Trockne eingeengt wird. Der Rückstand wird in Dichlormethan aufgenommen und mit ges. Natriumhydrogencarbonat-Lösung oder 1N Natriumhydroxid-Lösung gewaschen, über Magnesiumsulfat getrocknet und konzentriert. Gegebenenfalls kann das Produkt entweder durch Kristallisation mit Ether/Dichlormethan-Gemsichen oder durch Chromatographie an Silicagel (Dichlormethan/Methanol-Gemische) gereinigt werden.

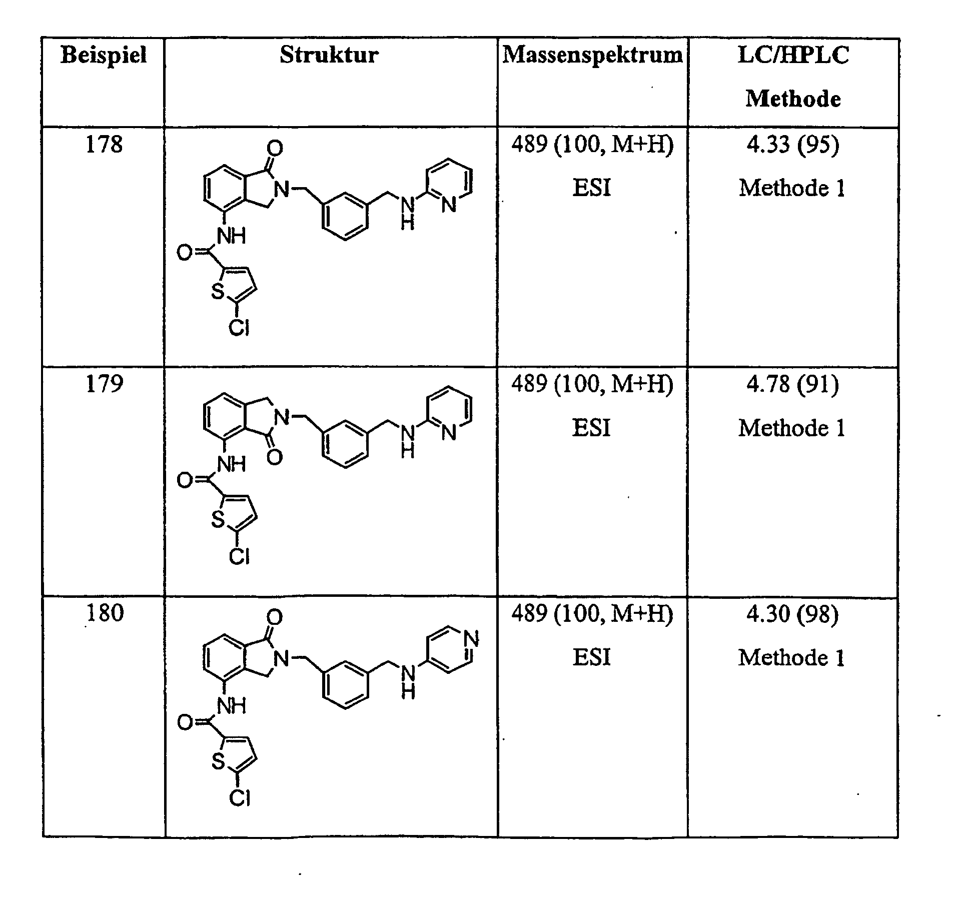

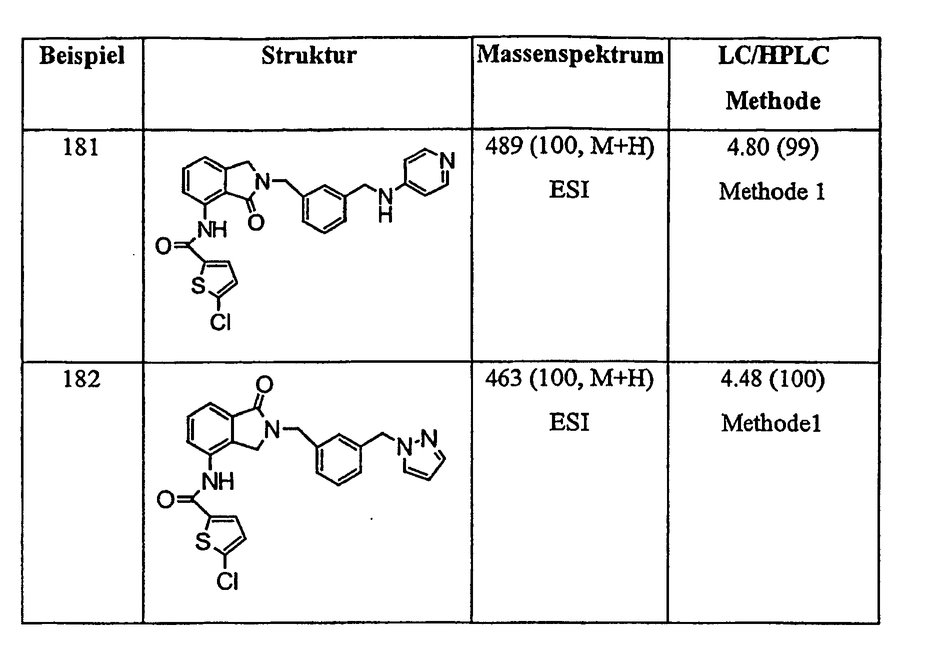

### Beispiel 183

### 2-{3-[(3-Hydroxy-4-nitro-1-oxo-1,3-dihydro-2H-isoindol-2-yl)methyl]phenyl}-acetonitril

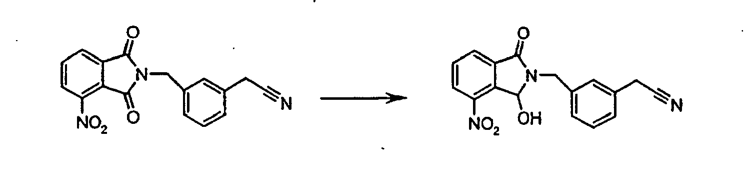

In eine auf -15 °C abgekühlte Suspension von 10.95 g (34.1 mmol) 2-{3-[(4-Nitro-1,3-dioxo-1,3-dihydro-2*H*-isoindol-2-yl)methyl]phenyl}acetonitril in einem Gemisch von 180 ml Dichlormethan und 180 ml Methanol werden portionsweise 1.81 g (47.7 mmol) Natriumborhydrid eingetragen. Die Mischung wird über 2 h auf Raumtemperatur erwärmt, bevor die Lösung mit 1N Salzsäure-Lösung vorsichtig leicht angesäuert wird. Nach Einengen auf ca. 100 ml wird der Rückstand in Dichlormethan/Methanol (98:2) aufgenommen, mit Wasser und ges. Natriumchlorid-Lösung gewaschen, über Magnesiumsulfat getrocknet und konzentriert (auf ca. 100 ml). Der Kristallbrei wird mit Dichlormethan versetzt, abfiltriert und der Feststoff am Hochvakuum getrocknet. Ausbeute: 6.8 g (61.7% der Theorie);

MS (DCI, NH₃): m/z (%) = 341 (M+NH₄, 100); HPLC (Methode 2): rt (%) = 6.99 (94.1).

### Beispiel 184

### 2-{3-[(4-Amino-3-hydroxy-1-ozo-1,3·dihydro-2H-isoindol-2-yl)methyl]phenyl}-acetonitril

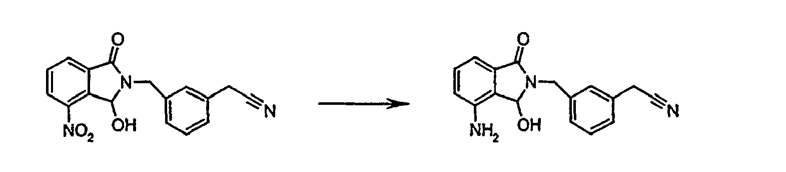

Zu einer Suspension von 6.47 g (20 mmol) 2-{3-[(3-Hydroxy-4-nitro-1-oxo-1,3-dihydro-2*H*-isoindol-2-yl)methyl]phenyl}acetonitril in einem Gemisch aus Methanol (100 ml), THF (100 ml) und Essigester (100) werden unter Argon 1.5 g Palladium auf Kohle (10%) gegeben. Die Mischung wird unter H₂-Atmosphäre (Normaldruck) über Nacht kräftig gerührt. Die Reaktionsmischung wird über Celite filtriert und eingeengt. Das Produkt wird am Hochvakuum getrocknet. Ausbeute: 5.89 g (90.3% der Theorie, ca. 90% Reinheit);

MS (DCI, NH₃): m/z (%) = 311 (M+NH₄, 100); HPLC (Methode 1): rt (%) = 3.36 (88).

### Beispiel 185

### 2-{3-[(4-Amino-1-oxo-1,3-dihydro-2H-isoindol-2-yl)methyl]phenyl}acetonitril

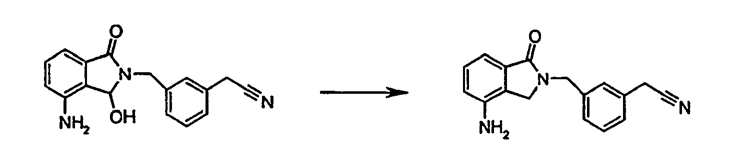

Zu einer Suspension von 5.87 g (20 mmol, ca 90% Reinheit) 2-{3-[(4-Amino-3-hydroxy-1-oxo-1,3-dihydro-2*H*-isoindol-2-yl)methyl]phenyl}acetonitril in 200 ml Dichlormethan werden bei Raumtemperatur zunächst 8 ml (50 mmol) Triethylsilan, dann tropfenweise 20 ml (260 mmol) TFA gegeben. Die Mischung wird 4 h bei Raumtemperatur gerührt, bevor konzentriert und am Hochvakuum getrocknet wird. Der Rückstand wird in Dichlormethan aufgenommen, mit ges. Natriumhydrogencarbonat-Lösung und ges. Natriumchlorid-Lösung gewaschen, über Magnesiumsulfat getrocknet und auf ca. 50 ml konzentriert. Der Kristallbrei wird mit ca. 100 ml Ether versetzt und nach ca. 2 h Rühren filtriert. Ausbeute: 4.36 g (78.7% der Theorie);

MS (EI pos): m/z (%) = 277 (M⁺, 100); HPLC (Methode 1): rt (%) = 3.36 (90).

### Beispiel 186

### 5-Chlor-N-{2-[3-(cyanomethyl)benzyl]-1-oxo-2,3-dihydro-1H-isoindol-4-yl}-2-thiophencarboxamid

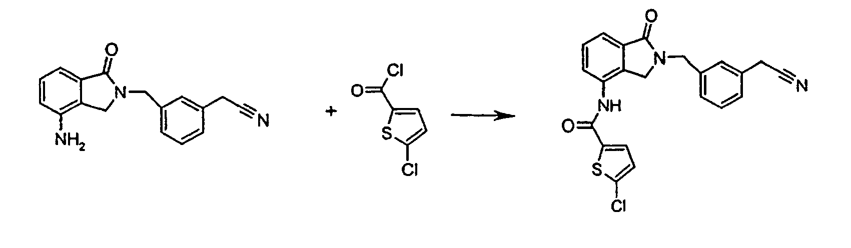

Zu einer Suspension von 2.77 g (10 mmol) 2-{3-[(4-Amino-1-oxo-1,3-dihydro-2*H-*isoindol-2-yl)methyl]phenyl}acetonitril in 30 ml absolutem THF werden 3.24 ml (40 mmol) Pyridin und 36.6 mg (0.3 mmol) 4-Dimethylaminopyridin gegeben. Die Mischung wird auf 0°C abgekühlt und tropfenweise mit 2.44 g (12.5 mmol) 5-Chlorthiophen-2-carbonsäurechlorid versetzt. Nach Ende der Zugabe wird die Eiskühlung entfernt und die Mischung auf Raumtemperatur erwärmt. Nach 1.5 h wird mit Dichlormethan verdünnt, mit ges. Natriumhydrogencarbonat- und ges. Natriumchlorid-Lösung gewaschen, über Magnesiumsulfat getrocknet und eingeengt. Der Rückstand wird in wenig Dichlormethan aufgenommen und mit Ether zur Kristallisation gebracht. Der Kristallbrei wird 2 h gerührt, bevor abgesaugt und am Hochvakuum getrocknet wird. Ausbeute: 3.83 g (90.8% der Theorie);

MS (DCI, NH₃): m/z (%) = 444 (M+Na, 32), 422 (M+H, 100); HPLC (Methode 1): rt (%) = 4.44 (95).

### Allgemeine Arbeitsvorschrift (V) zur Anilidbildung ausgehend von 2-{3-[(4-Amino-1-oxo-1,3-dihydro-2H-isoindol-2-yl)methyl]phenyl}acetonitril und in situ generierten Säurechloriden

Zu einer Mischung der entsprechenden Carbonsäure (2.0 eq) in absolutem Dichlormethan (ca. 0.25 mol/l) wird bei Raumtemperatur eine Stammlösung von N-(1-Chlor-2-methyl-1-propenyl)-*N,N*-dimethylamin (2.4 eq.) in absolutem Dichlormethan (ca. 2.5 mol/l) getropft. Nach 15 bis 30 min bei Raumtemperatur wird leq. 2-{3-[(4-Amino-1-oxo-1,3-dihydro-2*H*-isoindol-2-yl)methyl]phenyl}acetonitril hinzugefügt und die Mischung 1.5 bis 3 h bei Raumtemperatur intensiv gerührt, bevor nach Verdünnen mit absolutem Dichlormethan ca. 5 eq polymergebundenes Trisamin (PS-Trisamin, ca. 4 mmol/g) zugesetzt werden. Die Mischung wird ca. 2 h geschüttelt, filtriert und das Filtrat eingeengt. Das Produkt kann entweder durch Kristallisation mit Dichlormethan/Ether-Gemischen oder durch Chromatographie an Silicagel (Dichlormethan/Methanol-Gemische) gereinigt werden.

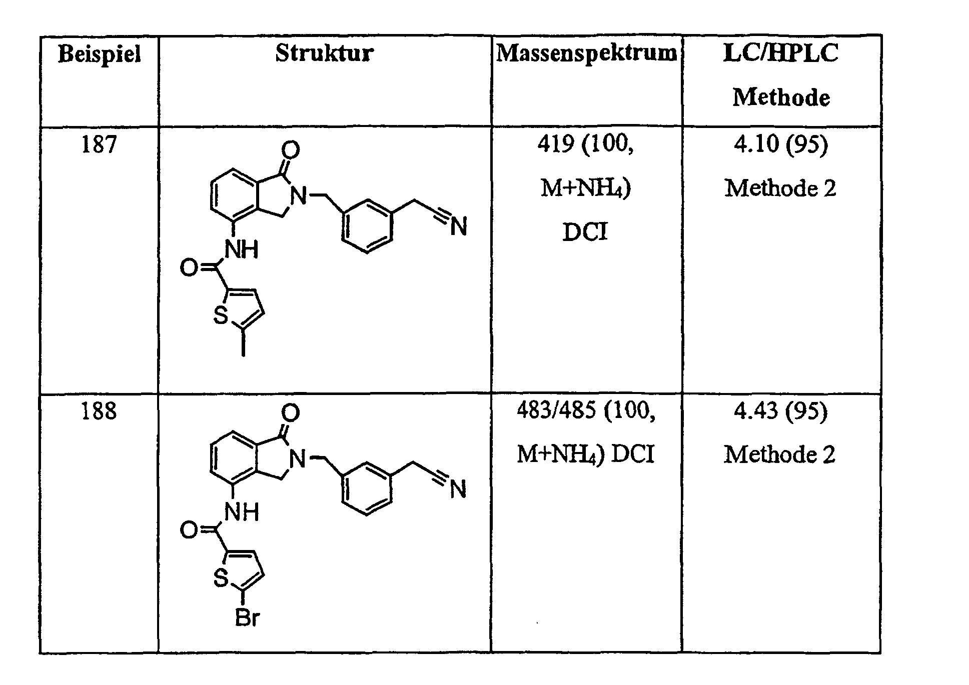

### Beispiel 189

### N-(2-{3-[2-Amino-2-(hydroxyimino)ethyl]benzyl}-1-oxo-2,3-dihydro-1H-isoindol-4-yl)-5-chlor-2-thiophencarboxamid

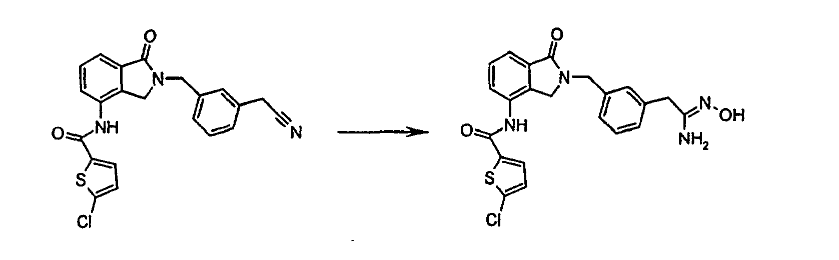

Eine Mischung von 436 mg (1.0 mmol) 5-Chlor-*N*-{2-[3-(cyanomethyl)benzyl]-1-oxo-2,3-dihydro-1*H*-isoindol-4-yl}-2-thiophencarboxamid in 5 ml 2-Propanol wird mit 90 mg ( 1.3 mmol) Hydroxylamin Hydrochlorid und 0.2 ml (1.4 mmol) Triethylamin versetzt und für 30 min auf Rückfluss erhitzt. Nach Abkühlen auf Raumtemperatur wird mit Wasser versetzt und der ausgefallene Feststoff abgesaugt. Das Produkt wird durch Chromatographie an Silicagel isoliert. Ausbeute: 45.3 mg (10% der Theorie);

MS (ESI): m/z (%) = 455 (M+H, 100); HPLC (Methode 1): rt (%) = 4.03 (90).

### Allgemeine Arbeitsvorschrift (VI) (Suzuki-Kupplung) zur Darstellung von Biphenyl-Derivaten

Eine Lösung von 1 eq. Arylbromid in 1,2-Dimethoxyethan (ca. 0.2 bis 0.5 mol/l) und Dichlorobis(triphenylphosphin)-palladium(II) (0.05 eq.) wird unter Argon mit 1.2 eq. Boronsäure und 2.5 eq Natriumcarbonat in Form einer 2 molaren wässrigen Lösung versetzt. Die Mischung wird für 1 h bis 5 h auf Rückfluss erhitzt und nach Abkühlen mit Wasser versetzt. Es wird mit Essigester extrahiert und die organische Phase mit ges. Natriumchlorid-Lösung gewaschen, über Magnesiumsulfat getrocknet und eingeengt. Das Produkt wird durch Chromatographie an Silicagel (Dichlormethan, Dichlormethan/Methanol oder Essigester/Cyclohexan-Gemische) isoliert und gereinigt.

Gemäß allgemeiner Arbeitsvorschrift (Suzuki-Kupplung) können ausgehend von 3-Hydroxymethylphenylboronsäure (kann hergestellt werden gemäß WO 97/36893 bzw. WO 95/29682) und Arylbromiden folgende Biphenyl-Derivate erhalten werden:

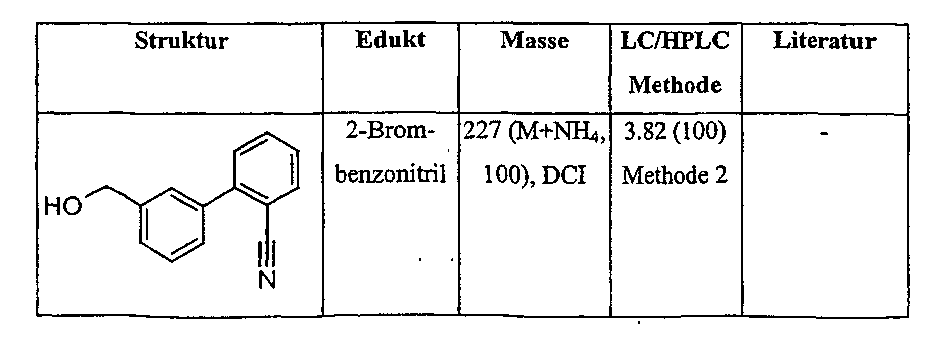

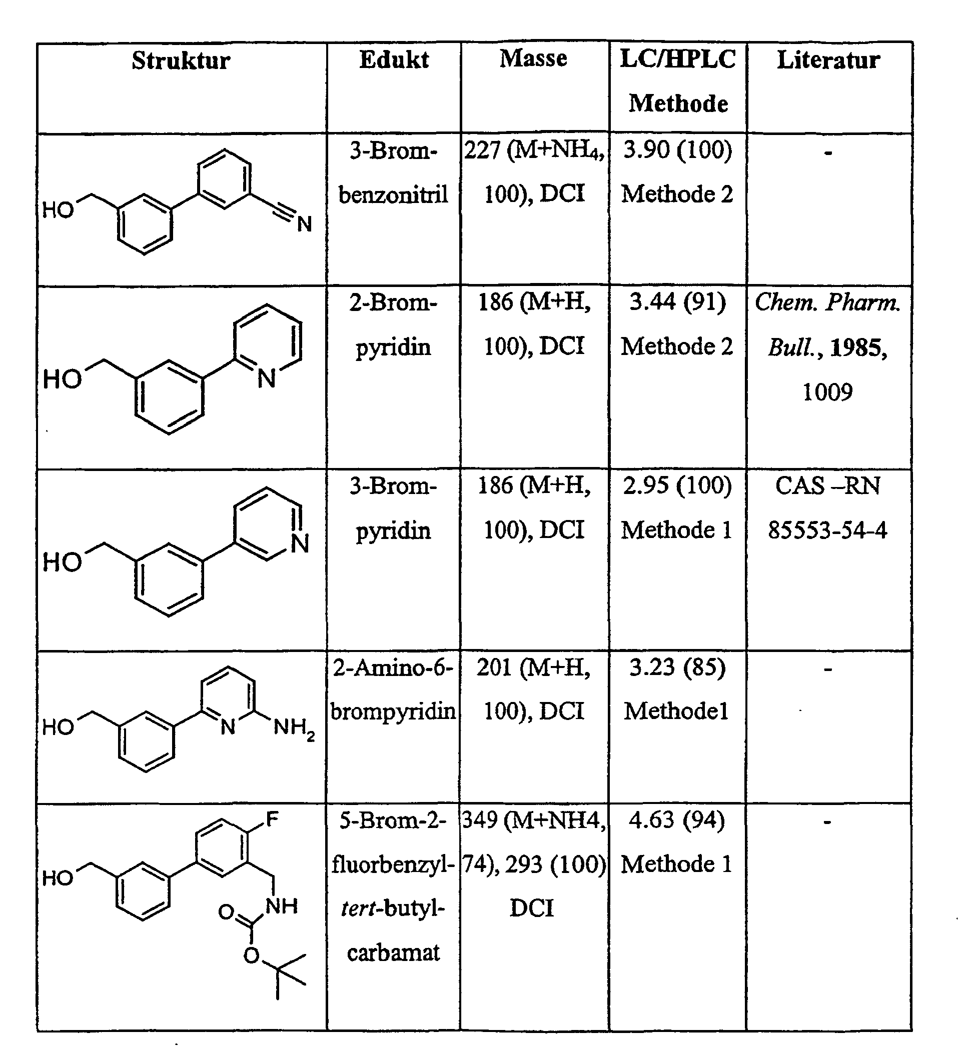

Gemäß allgemeiner Arbeitsvorschrift (Suzuki-Kupplung) kann ausgehend von 3-Aminophenylboronsäure Henüsulfat und 4-Brombenzylalkohol (3'-Amino[1,1'-biphenyl]-4-yl)methanol erhalten werden.

### Allgemeine Arbeitsvorschrift (VII) zu Synthese von methylbiphenyl-substituierten 5-Chlor-N-(1-oxo-2,3-dihydro-1H-isoindol-4-yl)-2-thiophencarboxamid Derivaten

Zu einer wassergekühlten Mischung aus 5-Chlor-*N*-(1,3-dioxo-2,3-dihydro-1*H-*isoindol-4-yl)-2-thiophencarboxamid (1.0 eq.), entsprechendem hydroxymethylsubstituiertem Biphenyl-Derivat (1.2 eq.) und Triphenylphosphin (1.2 eq.) in absolutem THF (ca. 0.1 mol/1) wird Azodicarbonsäurediethylester (1.2 bis 1.5 eq.) getropft. Die Reaktionsmischung wird ca. 1 h kräftig bei Raumtemperatur gerührt, bevor konzentriert wird. Das Produkt kann durch Kristallisation mit Ether oder durch Chromatographie an Silicagel (Essigester/Cyclohexan-Gemische) isoliert und gereinigt werden.

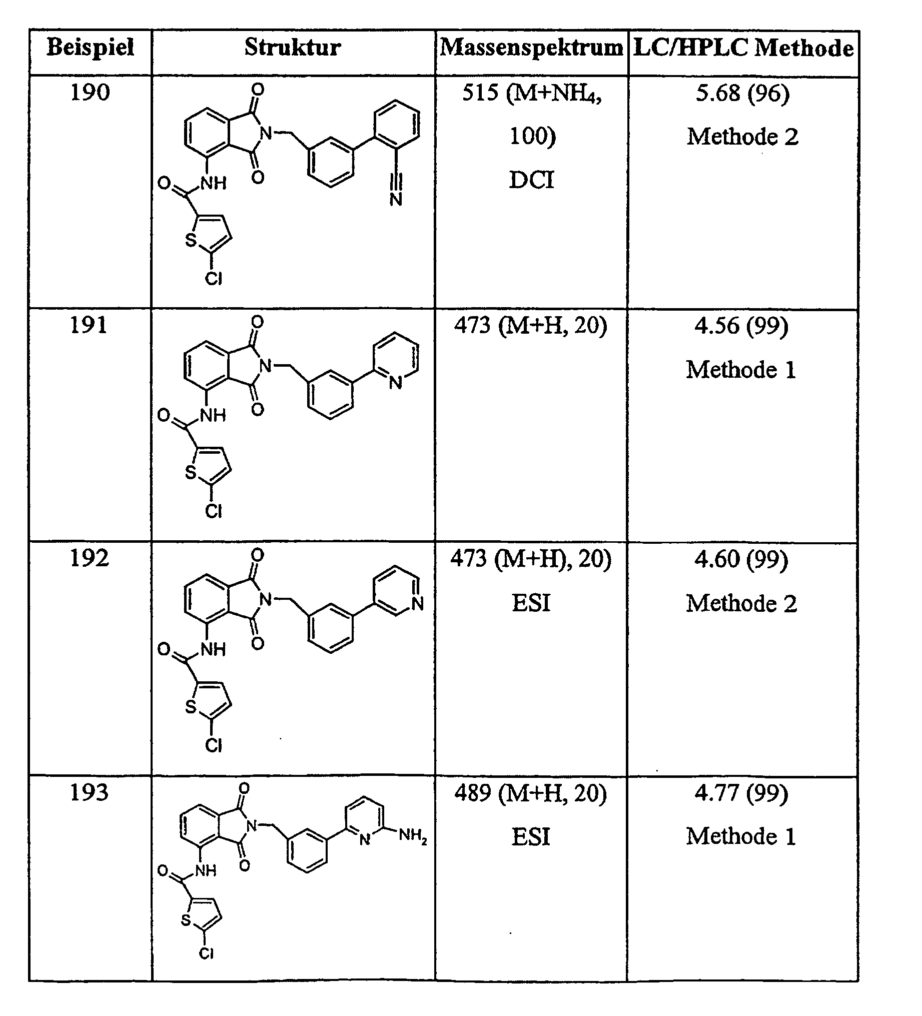

### Beispiel 194

Ausgehend von *tert*-Butyl-{3'-[(4-{[(5-chlor-2-thienyl)carbonyl]amino}-1,3-dioxo-1,3-dihydro-2*H*-isoindol-2-yl)methyl]-4-fluoro[1,1'-biphenyl]-3-yl}methylcarbamat lässt sich durch Behandlung des Rohprodukts mit etherischer Salzsäure-Lösung ***N-*(2-{[3'-(Aminomethyl)-4'-fluoro[1,1'-biphenyl]-3-yl]methyl}-1,3-dioxo-2,3-dihydro-1*H*-isoindol-4-yl)-5-chlor-2-thiophencarboxamid Hydrochlorid** gewinnen.

MS (ESI): m/z (%) = 520 (M-Cl, 15), 201 (100); HPLC (Methode 1): rt (%) = 4.81 (92).

### Beispiel 195

### N-(2-{[4-trans-(Aminomethyl)cyclohexyl]methyl}-1,3-dioxo-2,3-dihydro-1H-isoindol-4-yl)-5-chlor-2-thiophencarboxamid

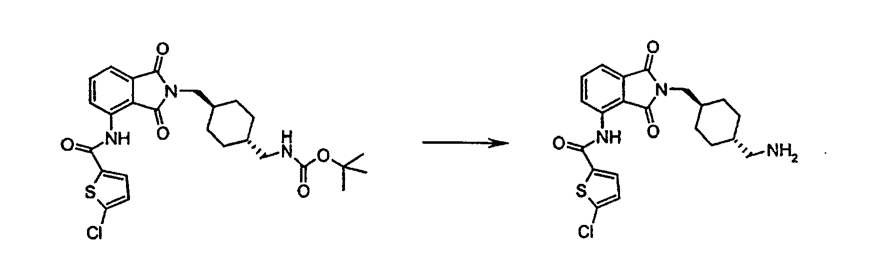

Zu einer eisgekühlten Lösung von 3.23 g *tert-*Butyl*-*{4*-trans-*[(4-{[(5-chlor-2-thienyl)carbonyl]amino}-1,3-dioxo-1,3-dihydro-2*H*-isoindol-2-yl)methyl]cyclohexyl}methylcarbamat (5.65 mmol) in 100 ml Chloroform werden 100 ml einer Mischung von TFA und Wasser (9:1) getropft. Die Eiskühlung wird entfernt und die Mischung wird 1.5 h bei Raumtemperatur gerührt, bevor eingeengt wird. Der Rückstand wird in Dichlormethan/Methanol aufgenommen und mit ges. Natriumhydrogencarbonat-Lösung gewaschen, über Magnesiumsulfat getrocknet und konzentriert.

Das anfallende Gemisch wird durch präparative RP-HPLC aufgetrennt. Ausbeute: 314 mg (12.9% der Theorie).

MS (DCI, NH₄): m/z (%) = 43 (M+H, 100); LC: rt (%) = 8.73 (96.2).

### Beispiel 196

Als Nebenkomponente kann **5-Chlor-N-{1,3-diozo-2-[(4-{[(2,2,2-trifluoroacetyl)amino]-methyl}cyclohexyl)methyl]-2,3-dihydro-1*H*-isoindol-4-yl}-2-thiophencarboxamid** isoliert werden.

MS (DCI, NH₄): m/z (%) = 545 (M+NH₄,100); LC: rt (%) = 11.30 (98.9).

### Beispiel 197

### 5-Chlor-N-{2-[(4-{[trans-(cyanomethyl)amino]methyl}cyclohexyl)methyl]-1,3-dioxo-2,3-dihydro-1H-isoindol-4-yl}-2-thiophencarboxamid

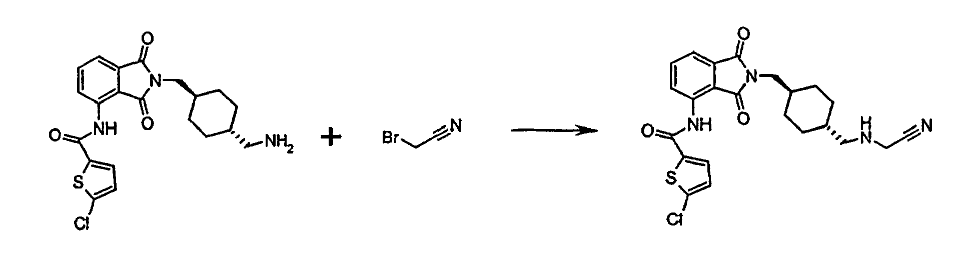

Zu 100 mg (0.23 mml) *N*-(2-{[4-*trans*-(Aminomethyl)cyclohexyl]methyl}-1,3-dioxo-2,3-dihydro-1*H*-isoindol-4-yl)-5-chlor-2-thiophencarboxamid in 2.5 ml absolutem DMF werden nacheinander 0.02 ml (0.28 mmol) Bromacetonitril und 0.06 ml (0.46 mmol) Triethylamin gegeben. Die Mischung wird über Nacht bei Raumtemperatur gerührt, bevor im Vakuum eingeengt und der Rückstand in Dichlormethan aufgenommen wird. Es wird mit gesättigter Natriumhydrogencarbonatlösung gewaschen, getrocknet und eingeengt. Das Produkt wird durch Chromatographie an Silicagel (Dichlormethan/Methanol 99:1) isoliert. Ausbeute: 52 mg (47.7% der Theorie).

MS (DCI, NH₄): m/z (%) = 488 (M+NH₄, 35), 471 (M+H, 20), 444 (100).

### Beispiel 198

### 5-Chlor-N-(2-{[cis-3-(cyanomethyl)cyclohexyl]methyl}-1-hydroxy-3-oxo-2,3-dihydro-1H-isoindol-4-yl)-2-thiophencarboxamid

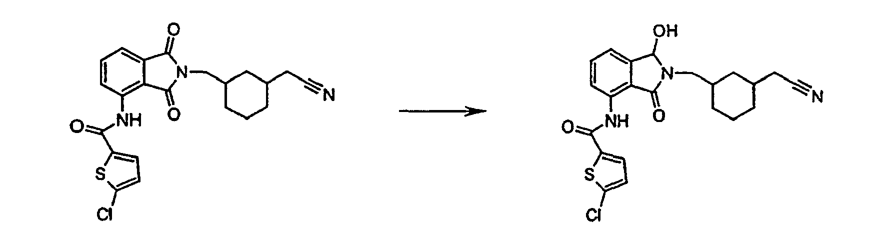

In einer Mischung aus 17 ml Dichlormethan und 17 ml Methanol werden 340 mg (0.77 mmol) 5-Chlor-*N*-(2-{[cis-3-(cyanomethyl)cyclohexyl]methyl}-1,3-dioxo-2,3-dihydro-1*H*-isoindol-4-yl)-2-thiophencarboxamid gelöst. Bei 0°C wird portionsweise Natriumborhydrid (43.6 mg, 1.15 mmol) hinzugegeben. Nach 3 h wird die Mischung mit 1N Salzsäure-Lösung leicht sauer gestellt und mit Dichlormethan mehrfach extrahiert. Die vereinigten organischen Extrakte werden über Magnesiumsulfat getrocknet und eingeengt. Das Produktgemisch wird durch Chromatographie an Silicagel (Dichlormethan/Methanol 98:2) getrennt. Ausbeute: 122 mg (35.7% der Theorie);

MS (ES⁺): m/z (%) = 444 (M+H, 100); MS (ES⁻): m/z (%) = 442 (M-H, 100).

Auf analoge Art lässt sich folgende Verbindung ausgehend von 5-Chlor-*N*-{1,3-dioxo-2-[(4- {[(2,2,2-trifluoroacetyl)amino]-methyl} cyclohexyl)methyl]-2,3-dihydro-1*H*-isoindol-4-yl}-2-miophencarboxamid darstellen:

### Beispiel 199

### 5-Chlor-N-{3-methoxy-1-oxo-2-[(4-{[(trifluoroacetyl)amino]methyl}-cyclohexyl)-methyl]-2,3-dihydro-1H-isoindol-4-yl}-2-thiophencarboxamid

MS (ESI): m/z (%) = 566 (M+Na, 34), 512 (100); HPLC (Methode 1): rt (%) = 4.85 (100).

### Beispiel 200

### 5-Chlor-N-(2-{(cis-3-(cyanomethyl)cyclohexyl]methyl}-3-oxo-2,3-dihydro-1H-isoindol-4-yl)-2-thiophencarboxamid

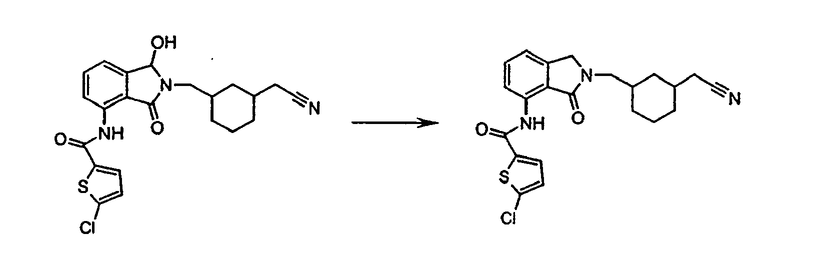

282 mg (0.635 mmol) 5-Chlor-*N*-(2-{[*cis*-3-(cyanomethyl)cyclohexyl]methyl}-1-hydroxy-3-oxo-2,3-dihydro-1*H*-isoindol-4-yl)-2-thiophencarboxamid werden in 8.5 ml Dichlormethan gelöst und mit 0.2 ml (1.27 mmol) Triethylsilan und 0.59 ml (7.62 mmol) TFA versetzt. Die Mischung wird über Nacht bei Raumtemperatur gerührt, bevor eingeengt wird. Der Rückstand wird in Dichlormethan aufgenommen und mit 1N Natriumhydroxid-Lösung gewaschen. Die organische Phase wird über Magnesiumsulfat getrocknet, eingeengt und der Rückstand durch Chromatographie an Silicagel (Dichlormethan/Methanol 99:1) gereinigt. Ausbeute: 150 mg (55.2% der Theorie);

MS (ESI): m/z (%) = 450 (M+Na, 100), 428 (M+H, 78); HPLC (Methode 1): rt (%) = 5.26 (100).

### Beispiel 201

### 5-Chlor-N-{1-oxo-2-[(4-{[(trifluoroacetyl)amino]methyl}cyclohexyl)-methyl]-2,3-dihydro-1H-isoindol-4-yl}-2-thiophencarboxamid

ausgehend von 5-Chlor-*N*-{3-methoxy-1-oxo-2-[(4-([(bsfluoroacetyl)amino]methyl}-cyclohexyl)methyl]-2,3-dihydro-1*H*-isoindol-4-yl}-2-thiophencarboxamid;

MS (ES⁺): m/z (%) = 514 (M+H, 100); HPLC (Methode 1): rt (%) = 4.57 (96).

### Beispiel 202

### 5-Chlor-N-{3-oxo-2-[(4-{[(trifluoroacetyl)amino]methyl}cyclohexyl)-methyl]-2,3-dihydro-1H-isoindol-4-yl}-2-thiophencarboxamid

ausgehend von 5-Chlor-*N*-{1-hydroxy-3-oxo-2-[(4-{[(trifluoroacetyl)amino]methyl}cyclohexyl)-methyl]-2,3-dihydro-1*H*-isoindol-4-yl}-2-thiophencarboxamid;

MS (ES⁺): m/z (%) = 514 (M+H, 100); HPLC (Methode 1): rt (%) = 5.22 (100).

### Beispiel 203

### N-(2-{[4-(Aminomethyl)cyclohexyl]methyl}-1-oxo-2,3-dihydro-1H-isoindol-4-yl)-5-chlor-2-thiophencarboxamid

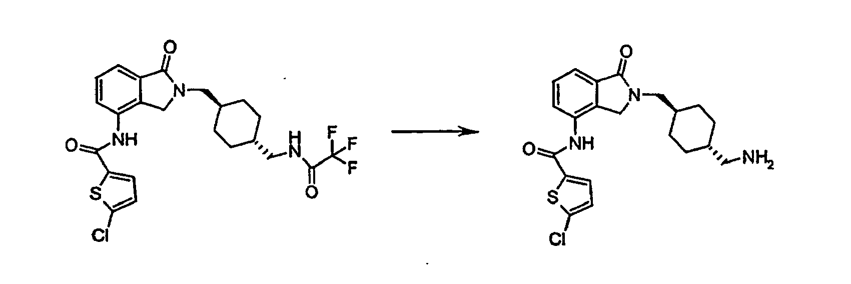

Zu einer Suspension von 102 mg (0.2 mmol) 5-Chlor-*N*-{1-oxo-2-[(4-{[(trifluoroacetyl)amino]methyl}cyclohexyl)-methyl]-2,3-dihydro-1*H*-isoindol-4-yl}-2-thiophencarboxamid in 6 ml Methanol werden einige Tropfen Wasser und 20 mg Natriummethanolat gegeben. Die resultierende Lösung wird über Nacht bei Raumtemperatur gerührt, bevor 1 ml IN Natriumhydroxid-Lösung hinzugefügt und auf 50°C erhitzt wird. Nach 3 h wird abgekühlt und mit Dichlormethan/Methanol 95:5 verdünnt. Es wird mit 1N Natriumhydroxid-Lösung gewaschen, über Natriumsulfat getrocknet, eingeengt und am Hochvakuum getrocknet. Ausbeute: 81.1 mg (98% der Theorie);

MS (ESI): m/z (%) = 418 (M+H, 40), 305 (100); HPLC (Methode 1): rt (%) = 3.93 (98).

### Beispiel 204

### N-(2-{[4-(Aminomethyl)cyclohexyl]methyl}-3-oxo-2,3-dihydro-1H-isoindol-4-yl)-5-chlor-2-thiophencarboxamid

ist analog ausgehend von 5-Chlor-N {3-oxo-2-[(4-{[(trifluoroacetyl)amino]methyl}cyclohexyl)-methyl]-2,3-dihydro-1*H*-isoindol-4-yl}-2-thiophencarboxamid darstellbar,

MS (ESI): m/z (%) = 418 (M+H, 80); HPLC (Methode 1): rt (%) = 4.40 (92).

### Beispiel 205

### 5-Chlor-N-{2-[3-(bydroxymethyl)benzyl]-1,3-dioxo-2,3-dihydro-1H-isoindol-4-yl}-2-thiophencarboxamid

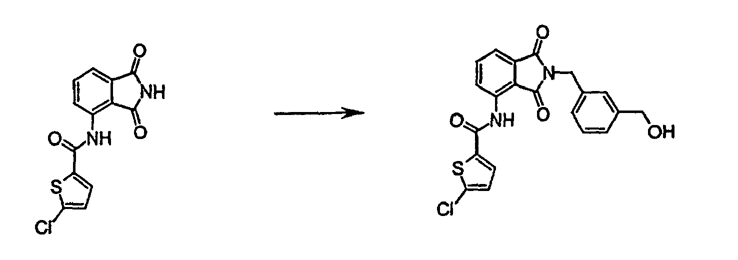

Zu einer Lösung von 5-Chlor-*N*-(1,3-dioxo-2,3-dihydro-1*H*-isoindol-4-yl)-2-thiophencarboxamid (5.0 g, 16.3 mmol), 3-(Hydroxymethyl)-benzylalkohol (2.9 g, 21.2 mmol) und Triphenylphosphin (5.6 g, 21.2 mmol) in Tetrahydrofuran (75 ml) wird unter Argon bei 0°C Azodicarbonsäurediethylester (3.3 ml, 21.2 mmol) getropft. Das Reaktionsgemisch wird für 2 h bei Raumtemperatur gerührt und mit Wasser versetzt. Nach Zugabe von Dichlormethan und Phasentrennung wird die wässrige Phase mit Dichlormethan extrahiert. Die vereinigten organischen Phasen werden getrocknet (Natriumsulfat), filtriert und im Vakuum eingeengt. Das gewünschte Produkt wird mittels Flash-Chromatographie (Dichlormethan-Methanol-Gemische) gereinigt. Ausbeute: 5.6 g, 81% der Theorie.

MS (DCI, NH₃): m/z (%) = 444 ([M+NH₄]⁺, 100), Cl-Muster; HPLC (Methode 1): rt = 5.05 min.

### Darstellung der Derivate von N-{2-[3-(Aminomethyl)benzyl]-1,3-dioxo-2,3-dihydro-1H-isoindol-4-yl}-5-chlor-2-thiophencarboxamid

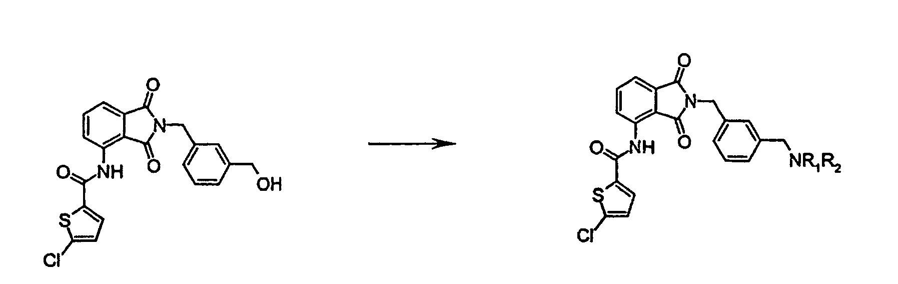

Zu einer Lösung von 5-Chlor-*N*-{2-[3-(hydroxymethyl)benzyl]-1,3-dioxo-2,3-dihydro-1*H*-isoindol-4-yl}-2-thiophencarboxamid und Triphenylphosphin (1 eq.) in Tetrahydrofuran (0.05 mol/l) wird unter Argon bei -20°C portionsweise *N-*Bromsuccinimid gegeben. Es wird 5 min nachgerührt und anschließend das Amin zugegeben. Nach weiteren 5 min wird die Reaktionsmischung langsam auf Raumtemperatur erwärmt, für 10 h refluxiert und mit Wasser versetzt. Nach Zugabe von Dichlormethan und Phasentrennung wird die wässrige Phase mit Dichlormethan extrahiert. Die vereinigten organischen Phasen werden getrocknet (Natriumsulfat), filtriert und im Vakuum eingeengt. Das gewünschte Produkt wird mittels Flash-Chromatographie (Cyclohexan-Ethylacetat-Gemische) gereinigt. Folgende Verbindungen lassen sich entsprechend darstellen:

### Beispiel 206

### 5-Chlor-N-{1,3-dioxo-2-[3-(1-pyrrolidinylmethyl)benzyl]-2,3-dihydro-1H-isoindol-4-yl}-2-thiophencarbozamid

MS (ESI): m/z (%) = 480 ([M+H]⁺, 88), Cl-Muster; HPLC (Methode 1): rt = 4.68 min.

### Beisniel 207

### 5-Chlor-N-(2-{3-[(dimethylamino)methyl]benzyl}-1,3-dioxo-2,3-dihydro-1H-isoindol-4-yl)-2-thiophencarboxamid

MS (ESI): m/z (%) = 454 ([M+H]⁺, 30), Cl-Muster; HPLC (Methode 1): rt = 4.70 min.

### Beispiel 208

### 5-Chlor-N-{1,3-dioxo-2-[3-(1-piperidinylmethyl)benzyl]-2,3-dihydro-1H-isoindo1-4-yl}-2-thiophenearboxamid

MS (ESI): m/z (%) = 494 ([M+H]⁺, 45), Cl-Muster; HPLC (Methode 1): rt = 4.78 min.

### 3-({[tert-Butyl(dimethyl)silyl]oxy}methyl)benzylamin

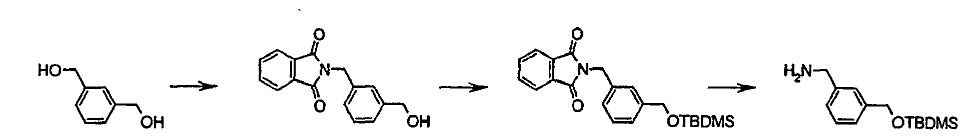

### 2-[3-(Hydroarymethyl)benzyl]-1H-isoindol-1,3(2H)-dion

Zu einer Lösung von Phthalimid (5.0 g, 34.0 mmol), 3-(Hydroxymethyl)-benzylalkohol (5.6 g, 40.8 mmol) und Triphenylphosphin (10.7 g, 40.8 mmol) in Tetrahydrofuran (68 mL) wird unter Argon bei 0°C Azodicarbonsäurediethylester getropft. Das Reaktionsgemisch wird für 15 h bei Raumtemperatur gerührt und mit Wasser versetzt. Nach Zugabe von Dichlormethan und Phasentrennung wird die wässrige Phase mit Dichlormethan extrahiert. Die vereinigten organischen Phasen werden getrocknet (Natriumsulfat), filtriert und im Vakuum eingeengt. Das gewünschte Produkt wird mittels Flash-Chromatographie (Petrolether-Ethylacetat-Gemische) gereinigt. Ausbeute: 6.5 g, 71% der Theorie.

MS (DCI, NH₃): m/z (%) = 285 ([M+NH₄]⁺, 100); R_{f} (Kieselgel, Dichlormethan-Methanol 20:1) = 0.75.

### 2-[3-({[tert-Butyl(dimethyl)silyl]oxy}methyl)benzyl]-1H-isoindol-1,3(2H)-dion

Zu einer Lösung von 2-[3-(Hydroxymethyl)benzyl]-1*H*-isoindol-1,3(2H)-dion (2.0 g, 7.5 mmol) in Tetrahydrofuran (22.5 ml) werden unter Argon bei Raumtemperatur Imidazol (1.0 g, 15.0 mmol) und tert-Butyldimethylsilylchlorid (1.7 g, 11.2 mmol) gegeben. Das Reaktionsgemisch wird für 16 h bei Raumtemperatur gerührt und mit Wasser versetzt. Nach Zugabe von Dichlormethan und Phasentrennung wird die wässrige Phase mit Dichlormethan extrahiert. Die vereinigten organischen Phasen werden getrocknet (Natriumsulfat), filtriert und im Vakuum eingeengt. Das gewünschte Produkt wird mittels Flash-Chromatographie (Petrolether-Ethylacetat-Gemische) gereinigt. Ausbeute: 2.7 g, 95% der Theorie.
MS (ESI): m/z (%) = 404 ([M+Na]⁺, 34); R_{f} (Kieselgel, Petrolether-Ethylacetat 3:1) = 0.84.

### 3-({[tert-Butyl(dimethyl)silyl]oxy}methyl)benzylamin

Eine Lösung von 2-[3-({[tert-Butyl(dimethyl)silyl]oxy}methyl)benzyl]-1*H*-isoindol-1,3(2*H*)-dion (1.7 g, 4.4 mmol) in Ethanol (44 ml) wird bei Raumtemperatur mit Hydrazinlösung (35%ig in Wasser, 0.6 ml, 6.6 mmol) versetzt und 15 h bei Raumtemperatur gerührt und mit Wasser versetzt. Nach Zugabe von Dichlormethan und Phasentrennung wird die wässrige Phase mit Dichlonnethan extrahiert. Die vereinigten organischen Phasen werden getrocknet (Natriumsulfat), filtriert und im Vakuum eingeengt. Das gewünschte Produkt wird ohne weitere Reinigung in der nächsten Reaktion eingesetzt.
MS (ESI): m/z (%) = 252 ([M+H]⁺, 100); LC (Methode 5): rt = 3.07 min.

### Beispiel 209

### 5-Chlor-N-[2-(3-formylbenzyl)-1-oxo-2,3-dihydro-1H-isoindol-4-yl]-2-thiophencarboxamid

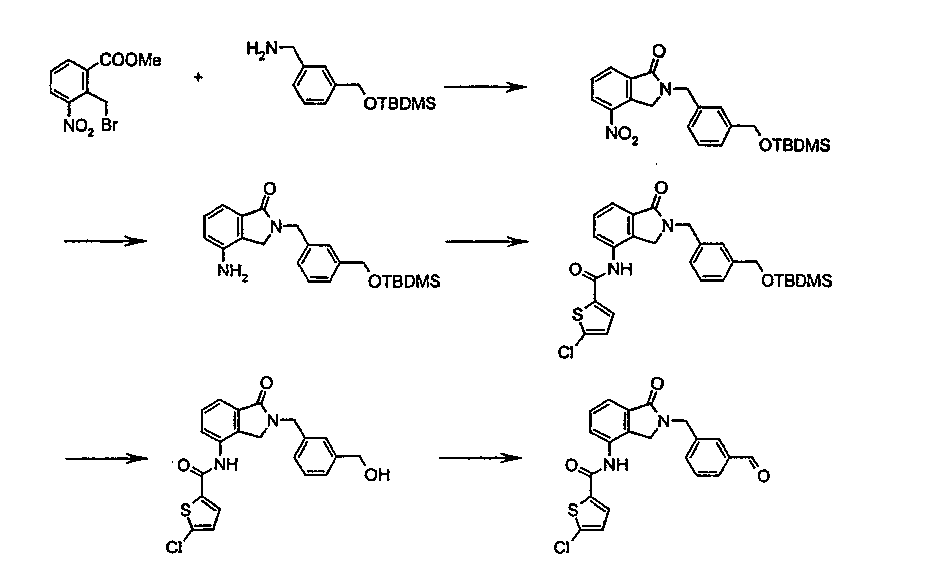

### 4-Nitro-2-[3-(silylmethyi)benzyl]-1-isoindolinon

Zu einer Lösung von 2-(Brommethyl)-3-nitrobenzoesäuremethylester *(J. Org. Chem.,* **1999,** 9731-9734) (6.2 g, 22.6 mmol) und 3-({[tert-Butyl(dimethyl)silyl]oxy}-methyl)benzylamin (5.7 g, 22.6 mmol) in Methanol (113ml) wird bei Raumtemperatur Triethylamin (3.5 ml, 24.9 mmol) gegeben. Das Reaktionsgemisch wird 16 h refluxiert und anschließend mit gesättigter wässriger Ammoniumchloridlösung versetzt. Nach Zugabe von Dichlormethan und Phasentrennung wird die wässrige Phase mit Dichlormethan extrahiert. Die vereinigten organischen Phasen werden getrocknet (Natriumsulfat), filtriert und im Vakuum eingeengt. Das gewünschte Produkt wird mittels Flash-Chromatographie (Cyclohexan-Ethylacetat-Gemische) gereinigt. Ausbeute: 5.1 g, 54% der Theorie.
MS (ESI): m/z (%) = 413 ([M+H]⁺, 10), 435 ([M+Na]⁺, 25); R_{f} (Kieselgel, Petrolether-Ethylacetat 2:1) = 0.53.

### 4-Amino-2-[3-(silylmethyl)benzyl]-1-isoindolinon

Eine Lösung von 4-Nitro-2-[3-(silylmethyl)benzyl]-1-isoindolinon (5.2 g, 12.5 mmol) in Tetrahydrofuran (60 ml) wird in Gegenwart von Palladium (10%ig auf Kohle, 375 mg) für 20 h unter Wasserstoff-Atmospähre gerührt, filtriert und das Filtrat im Vakuum eingeengt. Das gewünschte Produkt wird mittels Flash-Chromatographie (Cyclohexan-Ethylacetat-Gemische) gereinigt. Ausbeute: 3.8 g, 79% der Theorie.

MS (ESI): m/z (%) = 383 ([M+H]⁺, 23), 400 ([M+Na]⁺, 100); R_{f} (Kieselgel, Petrolether-Ethylacetat 2:1, dreimal gelaufen) = 0.53.

### 5-Chlor-N-{1-oxo-2-[3-(silylmethyl)benzyl]-2,3-dihydro-1H-isoindol-4-yl}-2-thiophencarboxamid

Zu einer Lösung von 4-Amino-2-[3-(silyhnethyl)benzyl]-1-isoindolinon (2.9 g, 7.7 mmol) in Tetrahydrofuran (26 ml) und Pyridin (12 ml) wird unter Argon bei 0°C 5-Chlor-2-thiophencarbonsäurechlorid (1.7 g, 9.2 mmol) und 4-Dimethylaminopyridin (93 mg, 0.8 mmol) gegeben. Das Reaktionsgemisch wird für 12 h bei Raumtemperatur gerührt und mit Wasser versetzt. Nach Zugabe von Dichlormethan und Phasentrennung wird die wässrige Phase mit Dichlormethan extrahiert. Die vereinigten organischen Phasen werden getrocknet (Natriumsulfat), filtriert und im Vakuum eingeengt. Das gewünschte Produkt wird mittels Flash-Chromatographie (Dichlormethan-Methanol-Gemische) gereinigt. Ausbeute: 3.9 g, 97% der Theorie.
MS (ESI): m/z (%) = 383 ([M+H]⁺, 23), 400 ([M+Na]⁺, 100); R_{f} (Kieselgel, Petrolether-Ethylacetat 2:1, dreimal gelaufen) = 0.53.

### 5-Chlor-N {2-[3-(hydroxymethyl)benzyl]-1-oxo-2,3-dihydro-1H- isoindol-4-yl}-2-thiophencarboxamid

Eine Lösung von 5-Chlor-*N*-{1-oxo-2-[3-(silylmethyl)benzyl]-2,3-dihydro-1*H-*isoindol-4-yl}-2-thiophencarboxamid (2.0 g, 3.8 mmol) in Tetrahydrofuran (38 ml) wird unter Argon bei Raumtemperatur mit Tetra-n-butylammoniumfluorid-Lösung (1 M in THF, 4.6 ml, 4.6 mmol) versetzt. Das Reaktionsgemisch wird für 14 h bei Raumtemperatur gerührt und im Vakuum eingeengt. Das Rohprodukt wird ohne weitere Reinigung in der nächsten Reaktion eingesetzt.

MS (ESI): m/z (%) = 413 ([M+H]⁺, 58),435 ([M+H]⁺, 100); HPLC (Methode 1): rt = 4.17min.

### 5-Chlor-N-[2-(3-formylbenzyl)-1-oxo-2,3-dihydro-1H-isoindol-4-yl]-2-thiophencarboxamid

Eine Lösung von 5-Chlor-*N*-{2-[3-(hydroxymethyl)benzyl]-1-oxo-2,3-dihydro-1*H-*isoindol-4-yl}-2-thiophencarboxamid (1.6 g, 3.8 mmol) in Dichlormethan (38 ml) und Pyridin (1.5 ml) wird mit Dess-Martin-Periodinan (1.9 g, 4.6 mmol) versetzt, für 6 h bei Raumtemperatur gerührt und anschließend mit gesättigter wässriger Natriumhydrogencarbonatlösung versetzt. Nach Zugabe von Ethylacetat und Phasentrennung wird die wässrige Phase mit Ethylacetat extrahiert. Die vereinigten organischen Phasen werden getrocknet (Natriumsulfat), filtriert und im Vakuum eingeengt. Das gewünschte Produkt wird mittels Flash-Chromatographie (Dichlormethan-Methanol-Gemische) gereinigt. Ausbeute: 1.5 g, 97% der Theorie.
MS (ESI): m/z (%) = 411 ([M+H]⁺, 100); HPLC (Methode 1): rt = 4.35 min.

### Reduktive Aminierung von 5-Chlor-N-[2-(3-formylbenzyl)-1-oxo-2,3-dihydro-1H-isoindol-4-yl]-2-thiophenearboxamid

Zu einer Suspension von 5-Chlor-*N*-[2-(3-formylbenzyl)-1-oxo-2,3-dihydro-1*H-*isoindol-4-yl]-2-thiophencarboxamid in Tetrahydrofuran (0.05 mol/1) und Methanol (0.05 mol/1) wird unter Argon bei Raumtemperatur Eisessig (2 eq.) und das entsprechende Amin (2 eq.) gegeben. Das Reaktionsgemisch wird 6-9 h bei 70°C gerührt, wobei der Niederschlag in Lösung geht. Nach Abkühlen der Reaktionsmischung wird diese mit Natriumcyanoborhydrid (2 eq.) versetzt, weitere 8-10 h bei Raumtemperatur gerührt und mit gesättigter wässriger Natriumhydrogencarbonatlösung versetzt. Nach Zugabe von Dichlormethan und Phasentrennung wird die wässrige Phase mit Dichlormethan extrahiert. Die vereinigten organischen Phasen werden getrocknet (Natriumsulfat), filtriert und im Vakuum eingeengt. Das gewünschte Produkt wird mittels Präparativer Dünnschichtchromatographie (Dichlormethan-Methanol-Gemische) gereinigt. Folgende Verbindungen lassen sich entsprechend darstellen:

### Beispiel 210

### 5-Chlor-N-[1-oxo-2-(3-{[(4-pyridinylmethyl)amino]methyl}benzyl)-2,3-dihydro-1H-isoindol-4-yl]-2-thiophencarboxamid

MS (ESI): m/z (%) = 503 ([M+H]⁺, 100), Cl-Muster; HPLC (Methode 1): rt = 3.91 min.

### Beispiel 211

### 5-Chlor-N-(2-{3-[(4-methyl-1-piperazinyl)methyl]benzyl}-1-oxo-2,3-dihydro-1H-isoindol-4-yl)-2-thiophencarboxamid

MS (ESI): m/z (%) = 495 ([M+H]⁺, 100), Cl-Muster; HPLC (Methode 4): rt = 3.15 min.

### Beispiel 212

### 5-Chlor-N-(2-{3-[(3-isoxazolyiamino)methyl]benzyl}-1-oxo-2,3-dibydro-1H-isoindol-4-yl)-2-thiophencarboxamid

MS (ESI): m/z (%) = 479 ([M+H]⁺, 100), Cl-Muster; HPLC (Methode 2): rt = 4.52 min.

### Beispiel 213

### 5-Chlor-N-(2-{3-[(dimethylamino)methyl]benzyl}-1-oxo-2,3-dihydro-1H-isoindol-4-yl)-2-thiophencarboxamid

MS (ESI): m/z (%) = 440 ([M+H]⁺, 100), Cl-Muster; HPLC (Methode 1): rt = 3.96 min.

### Beispiel 214

### 5-Chlor-N-(2-{3-[(methylamino)methyl]benzyl}-1-oxo-2,3-dihydro-1H-isoindol-4-yl)-2-thiophencarboxamid

MS (ESI): m/z (%) = 426 ([M+H]⁺, 100), Cl-Muster; HPLC (Methode 4): rt = 2.77 min.

### Beispiel 215

### 5-Chlor-N-{1-oxo-2-[3-(1-piperidinylmethyl)benzyl]-2,3-dihydro-1H-isoindol-4-yl}-2-thiophencarboxamid

MS (ESI): m/z (%) = 480 ([M+H]⁺, 100), C1-Muster; HPLC (Methode 1): rt = 4.07 min.

### Beispiel 216

### 5-Chlor-N-[2-(3-{[(4-fluorophenyl)amino]methyl}benzyl)-1-oxo-2,3-dihydro-1H isoindol-4-yl]-2-thiophencarboxamid

MS (ESI): m/z (%) = 506 ([M+H]⁺, 100), Cl-Muster; HPLC (Methode 2): rt = 4.19 min.

### Beispiel 217

### 5-Chlor-N-(2-{3-[(isopropylamino)methyl]benzyl}-1-oxo-2,3-dihydro-1H-isoindol-4-yl)-2-thiophencarboxamid

MS (ESI): m/z (%) = 454 ([M+H]⁺, 100), Cl-Muster, HPLC (Methode 1): rt = 4.01 min.

### Beispiel 218

### N {2-[3-((E)-{[Amino(imino)methyl]hydrazono}methyl)benzyl]-1,3-dioxo-2,3-dihydro-1H isoindol-4-yl}-5-chlor-2-thiophencarboxamid aus 5-Chlor-N-{2-[3-(hydroxymethyl)benzyl]-1,3-dioxo-2,3-dihydro-1H-isoindol-4-yl}-2-thiophencarboxamid

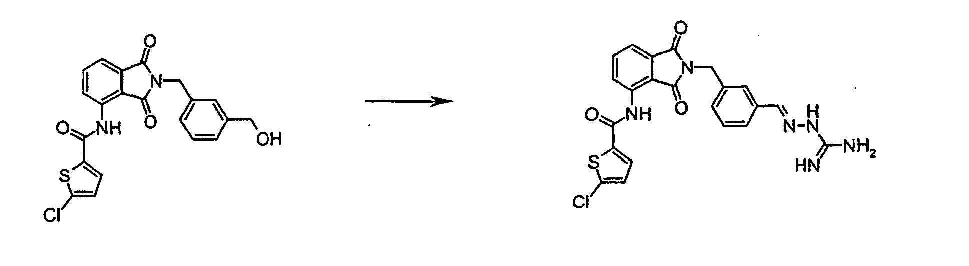

Zu einer Suspension von 5-Chlor-*N*- {2-[3-(hydroxymethyl)benzyl]-1,3-dioxo-2,3-dihydro-1*H*-isoindol-4-yl}-2-thiophencarboxamid in Tetrahydrofuran (0.2 mol/l) und Dichlormethan (0.2 mol/l) wird unter Argon bei Raumtemperatur Dess-Martin-Periodinan (1.2 eq.) gegeben. Die Reaktionsmischung wird 4 h bei Raumtemperatur gerührt und mit gesättigter wässriger Natriumhydrogencarbonatlösung versetzt. Nach Zugabe von Dichlormethan und Phasentrennung wird die wässrige Phase mit Dichlormethan extrahiert. Die vereinigten organischen Phasen werden getrocknet (Natriumsulfat), filtriert und im Vakuum eingeengt. Das Rohprodukt wird ohne weitere Reinigung in der nächsten Reaktion eingesetzt.

Eine Suspension aus 5-Chlor-*N*-{2-[3-(hydroxymethyl)benzyl]-1,3-dioxo-2,3-dihydro-1*H*-isoindol-4-yl}-2-thiophencarboxamid und Aminoguanidinnitrat (1.2 eq.) in Ethanol (0.02 mol/l) wird für 1 d refluxiert, wobei sich der Niederschlag löst und erneut ein farbloser Feststoff ausfällt. Der Niederschlag wird abfiltriert, mit Ethanol gewaschen und getrocknet.
MS (ESI): m/z (%) = 481 ([M+H]⁺, 100), Cl-Muster, HPLC (Methode 1): rt = 4.61 min.

## Patentansprüche

1. Verbindungen der Formel (I) 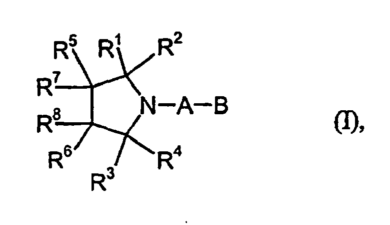
worin
R¹ und R² gemeinsam für O stehen und
R³ und R⁴ gemeinsam für O stehen,
oder
R¹ Wasserstoff, Hydroxy oder (C₁-C₄)-Alkoxy bedeutet,
R² Wasserstoff bedeutet und
R³ und R⁴ gemeinsam für O stehen,
oder
R¹ und R² gemeinsam für O stehen,
R³ Wasserstoff, Hydroxy oder (C₁-C₄)-Alkoxy bedeutet und
R⁴ Wasserstoff bedeutet,
R⁵ und R⁶ für Wasserstoff stehen und
R⁷ und R⁸ gemeinsam für 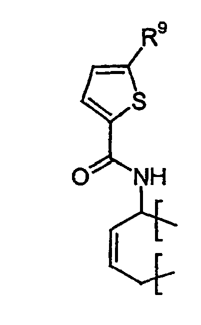 stehen,
worin
R⁹ Halogen, Trifluormethyl oder Methyl bedeutet, oder
R⁵, R⁶, R⁷ und R⁸ gemeinsam für 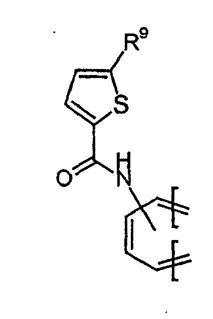 stehen, worin
R⁹ die oben angegebene Bedeutung besitzt,
A (C₁-C₄)-Alkandiyl oder (C₂-C₄)-Alkendiyl bedeutet,
B Phenylen oder Cyclohexandiyl bedeutet, die durch Amino, Harnstoff, Sulfamoyl, -C(=N-NH-C(=NH)-NH₂)-H, -C(=NR¹⁰)-R¹¹
worin
R¹⁰ Wasserstoff oder -NH-C(=NH)-NH₂ bedeutet,
R¹¹ -NR¹²R¹³ oder 5- bis 10-gliedriges Heterocyclyl bedeutet,
worin
R¹² und R¹³, unabhängig voneinander, Wasserstoff, (C₁-C₄)-Alkyl oder (C₃-C₇)-Cycloalkyl bedeuten,
(C₁-C₄)-Alkyl, das seinerseit durch Cyano, (C₁-C₄)-Alkoxycarbonyl, gegebenenfalls durch (C₁-C₄)-Alkyl substituiertes 5- bis 10-gliedriges Heterocyclyl, 5- bis 10-gliedriges Heteroaryl, Tri-(C₁-C₄)-alkylammonium, -NR¹⁴R¹⁵, -C(=NR¹⁶)-R¹⁷, -N-C(=O)-R¹⁸ oder -N-C(=O)-NH-R¹⁹ substituiert sein kann,
worin
R¹⁴ Wasserstoff, Mono- oder Di-(C₁-C₄)-alkylamino, gegebenenfalls durch 5- bis 10-gliedriges Heteroaryl substituiertes (C₁-C₄)-Alkyl, 5- bis 10-gliedriges Heterocyclyl, (C₆-C₁₀)-Aryl oder 5- bis 10-gliedriges Heteroaryl, wobei die Ringe ihrerseits durch Halogen substituiert sein können, bedeutet,
R¹⁵ Wasserstoff oder gegebenenfalls durch Cyano substituiertes (C₁-C₄)-Alkyl bedeutet,
R¹⁶ Wasserstoff oder Hydroxy bedeutet,
R¹⁷ Amino, 5- bis 10-gliedriges Heterocyclyl, gegebenenfalls durch Amino oder Trifluormethyl substituiertes Mono- oder Di-(C₁-C₄)-Alkylamino, gegebenenfalls durch Trifluormethyl substituiertes (C₃-C₇)-Cycloalkylamino bedeutet,
R¹⁸ Trifluormethyl, (C₁-C₄)-Alkyl, (C₁-C₄)-Alkoxy oder gegebenenfalls durch (C₁-C₄)-Alkyl substituiertes 5- bis 10-gliedriges Heterocyclyl bedeutet,
R¹⁹ Wasserstoff, Amino, Dimethylamino, 5- bis 10-gliedriges Heterocyclyl, 5- bis 10-gliedriges Heteroaryl, oder gegebenenfalls durch (C₁-C₄)-Alkoxycarbonyl, Dimethylamino, Carbamoyl oder 5- bis 10-gliedriges Heteroaryl substituiertes (C₁-C₄)-Alkyl bedeutet,
(C₁-C₄)-Alkoxy, das seinerseits ein- oder zweifach, unabhängig voneinander, durch Cyano, Thiocarbamoyl, gegebenenfalls durch (C₁₋C₄)-Alkyl substituiertes 5- bis 10-gliedriges Heterocyclyl, gegebenenfalls durch Halogen substituiertes 5- bis 10-gliedriges Heteroaryl, gegebenenfalls durch -C(=NR²⁰)-R²¹ substituiertes (C₆-C₁₀)-Aryl oder -C(=NR²⁰)-R²¹ substituiert sein kann,
worin
R²⁰ Wasserstoff, Hydroxy, (C₃-C₇)-Cycloalkyl oder gegebenenfalls durch Hydroxy substituiertes (C₁-C₄)-Alkyl bedeutet,
R²¹ Amino, (C₁-C₄)-Alkylthio, (C₃-C₇)-Cycloalkylamino, Benzylamino oder 5- bis 10-gliedriges Heterocyclyl bedeutet,
5- bis 10-gliedriges Heterocyclyl, das seinerseits durch (C₁-C₄)-Alkyl substituiert sein kann,
(C₆-C₁₀)-Aryl, das seinerseits ein- oder zweifach, unabhängig voneinander, durch Halogen, Cyano, Carbamoyl oder gegebenenfalls durch Amino substituiertes (C₁-C₄)-Alkyl substituiert sein kann,
5- bis 10-gliedriges Heteroaryloxy, das seinerseits durch Amino oder N-(C₁-C₄)-Alkylaminocarbonyl substituiert sein kann,
oder 5- bis 10-gliedriges Heteroaryl, das seinerseits durch Amino substituiert sein kann, substituiert sein können,
sowie deren Salze, Hydrate, Hydrate der Salze und Solvate.

2. Verbindungen nach Anspruch 1,
worin
R¹ und R² gemeinsam für O stehen und
R³ und R⁴ gemeinsam für O stehen,
oder
R¹ Wasserstoff, Hydroxy, Methoxy oder Ethoxy bedeutet,
R² Wasserstoff bedeutet und
R³ und R⁴ gemeinsam für O stehen,
oder
R¹ und R² gemeinsam für O stehen, R³ Wasserstoff, Hydroxy, Methoxy oder Ethoxy bedeutet und
R⁴ Wasserstoff bedeutet,
R⁵ und R⁶ für Wasserstoff stehen und R⁷ und R⁸ gemeinsam für 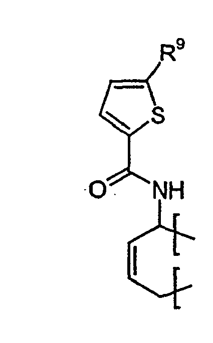
stehen,
worin
R⁹ Halogen oder Trifluormethyl bedeutet,
oder
R⁵, R⁶, R⁷ und R⁸ gemeinsam für 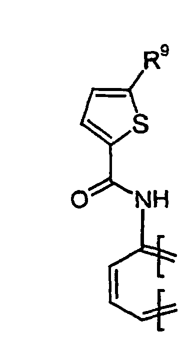
stehen,
worin
R⁹ die oben angegebene Bedeutung besitzt,
A (C₁-C₃)-Alkandiyl oder (C₂-C₃)-Alkendiyl bedeutet,
B Phenylen oder Cyclohexandiyl bedeutet, die durch -C(=NR¹⁰)-R¹¹
worin
R¹⁰ Wasserstoffbedeutet,
R¹¹ -NR¹²R¹³ oder 5- bis 10-gliedriges Heterocyclyl bedeutet,
worin
R¹² und R¹³, unabhängig voneinander, Wasserstoff, (C₁-C₄)-
Alkyl oder (C₃-C₇)-Cycloalkyl bedeuten,
Methyl oder Ethyl, die ihrerseits durch Cyano, Methoxycarbonyl, gegebenenfalls durch Methyl oder Ethyl substituiertes 5- oder 6-
gliedriges Heterocyclyl, 5- oder 6-gliedriges Heteroaryl, Tri-(C₁-C₂)-alkylammonium,-NR¹⁴R¹⁵, -C(=NR¹⁶)-R¹⁷ , -N-C(=O)-R¹⁸ oder -N-C(=O)-NH-R¹⁹ substituiert sein können,
worin
R¹⁴ Wasserstoff, Dimethylamino, gegebenenfalls durch 5- oder 6-gliedriges Heteroaryl substituiertes Methyl oder Ethyl, 5- oder 6-gliedriges Heterocyclyl oder 5- oder 6-gliedriges Heteroaryl, das durch Halogen substituiert sein kann, bedeutet,
R¹⁵ Wasserstoff, Methyl oder Ethyl bedeutet, R¹⁶ Wasserstoff bedeutet,
R¹⁷ Amino, 5- oder 6-gliedriges Heterocyclyl, gegebenenfalls durch Amino oder Trifluormethyl substituiertes Mono- oder Di-(C₁-C₄)-Alkylamino, gegebenenfalls durch Trifluonnethyl substituiertes (C₃-C₇)-Cycloalkylamino bedeutet,
R¹⁸ Trifluormethyl oder (C₁-C₄)-Alkyl bedeutet,
R¹⁹ Wasserstoff, Amino oder gegebenenfalls durch Methoxy- oder Ethoxycarbonyl substituiertes (C₁-C₄)-Alkyl bedeutet,
Methoxy oder Ethoxy, die ihrerseits ein- oder zweifach, unabhängig voneinander, durch gegebenenfalls durch Methyl substituiertes 5- oder 6-gliedriges Heterocyclyl oder -C(=NR²⁰)-R²¹ substituiert sein können,
worin
R²⁰ Wasserstoff bedeutet,
R²¹ Amino, (C₃-C₆)-Cycloalkylamino, Benzylamino oder 5- oder 6-gliedriges Heterocyclyl bedeutet,
oder Pyridyl substituiert sein können,
sowie deren Salze, Hydrate, Hydrate der Salze und Solvate.

3. Verbindungen nach Anspruch 1,
worin
R¹ und R² gemeinsam für O stehen und
R³ und R⁴ gemeinsam für O stehen,
oder
R¹ Wasserstoff, Hydroxy oder Methoxy bedeutet,
R² Wasserstoff bedeutet und
R³ und R⁴ gemeinsam für O stehen,
oder
R¹ und R² gemeinsam für O stehen, R³ Wasserstoff, Hydroxy oder Methoxy bedeutet und
R⁴ Wasserstoff bedeutet,
R⁵ und R⁶ für Wasserstoff stehen und
R⁷ und R⁸ gemeinsam für 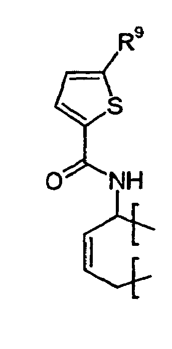
stehen,
worin
R⁹ Chlor oder Brom bedeutet,
oder
R⁵ R⁶, R⁷ und R⁸ gemeinsam für 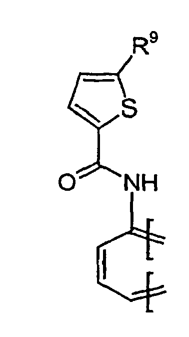
stehen,
worin
R⁹ die oben angegebene Bedeutung besitzt,
A Methandiyl oder Ethandiyl bedeutet,
B Phenylen bedeutet, das durch -C(=NR¹⁰)-R¹¹
worin
R¹⁰ Wasserstoff bedeutet,
R¹¹ Amino bedeutet,
Methyl, das seinerseits durch Cyano, gegebenenfalls durch Methyl substituiertes Imidazolinyl oder Tetrahydropyrimidinyl, -NR¹⁴R¹⁵ oder -C(=NR¹⁶)-R¹⁷ substituiert sein kann,
worin
R¹⁴ gegebenenfalls durch Pyridyl substituiertes Methyl oder Pyridyl bedeutet,
R¹⁵ Wasserstoff bedeutet,
R¹⁶ Wasserstoff bedeutet,
R¹⁷ Amino, Piperidinyl, Morpholinyl, Pyrrolidinyl, gegebenenfalls durch Amino oder Trifluormethyl substituiertes Mono- oder Di-(C₁-C₃)-Alkylamino oder gegebenenfalls durch Trifluormethyl substituiertes Cyclopropyl-, Cyclopentyl- oder Cyclohexylamino bedeutet,
oder Methoxy oder Ethoxy, die ihrerseits durch -C(=NH)-NH₂ substituiert sein können, substituiert sein kann,
sowie deren Salze, Hydrate, Hydrate der Salze und Solvate.

4. Verfahren zur Herstellung von Verbindungen der Formel (1), wie in Anspruch 1 definiert, **dadurch gekennzeichnet, dass** man
entweder
[A] Verbindungen der Formel (IV) 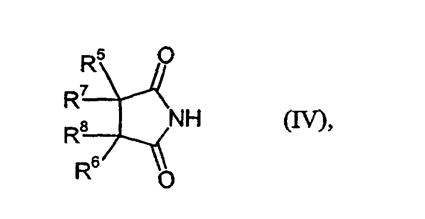
worin R⁵, R⁶, R⁷ und R⁸die in Anspruch 1 angegebene Bedeutung besitzen,
mit Verbindungen der Formel (V)
Y-A-B (V),
worin A und B die in Anspruch 1 angegebene Bedeutung besitzen und Y für eine geeignete Abgangsgruppe steht,
zu Verbindungen der Formel (I) umsetzt,
worin R¹ und R², sowie R³ und R⁴, jeweils gemeinsam, für O stehen und R⁵, R⁶, R⁷, R⁸, A und B die in Anspruch 1 angegebene Bedeutung besitzen,
oder
[B1] Verbindungen der Formel (VIII) oder (VIIIa) 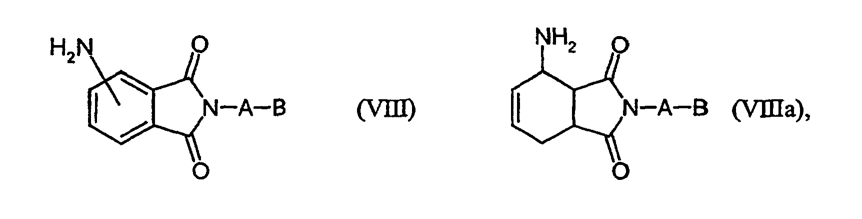
worin A und B die in Anspruch 1 angegebene Bedeutung besitzen,
mit Verbindungen der Formel (III) 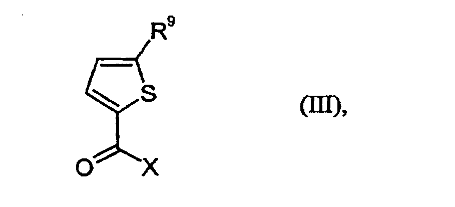
worin R⁹ die inAnspruch 1 angegebene Bedeutung hat und X für eine Abgangsgruppe steht,
zu Verbindungen der Formel (I) umsetzt,
worin R¹ und R², sowie R³ und R⁴, jeweils gemeinsam, für O stehen und R⁵, R⁶, R⁷, R⁸, A und B die in Anspruch 1 angegebene Bedeutung besitzen,
oder
[B2] Verbindungen der Formel (IX) 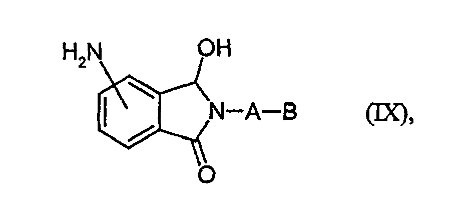
worin A und B die in Anspruch 1 angegebene Bedeutung besitzen,
oder Verbindungen der Formel (X) 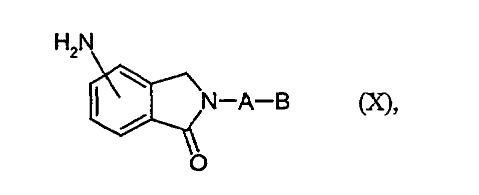
worin A und B die in Anspruch 1 angegebene Bedeutung besitzen,
mit Verbindungen der Formel (III) zu Verbindungen der Formel (I) umsetzt,
worin R¹ und R² gemeinsam für O, R³ für Wasserstoff oder Hydroxy, R⁴ für Wasserstoff stehen oder R³ und R⁴ gemeinsam für O, R¹ für Wasserstoff oder Hydroxy, R² für Wasserstoff stehen und R⁵, R⁶, R⁷, R⁸, A und B die in Anspruch 1 angegebene Bedeutung besitzen, oder
[C] Verbindungen der Formel (XIV) 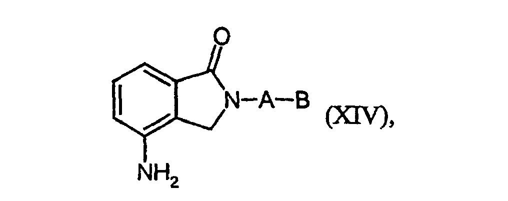
worin A und B die in Anspruch 1 angegebene Bedeutung besitzen,
mit Verbindungen der Formel (III) zu Verbindungen der Formel (I) umsetzt,
worin R¹ und R² für Wasserstoff, R³ und R⁴ gemeinsam für O stehen und R⁵, R⁶, R⁷, R⁸, A und B die in Anspruch 1 angegebene Bedeutung besitzen,
wobei sich bei den so erhaltenen Verbindungen der Formel (I) gegebenenfalls weitere Derivatisierungen, die nach üblichen Methoden durchgeführt werden können, anschließen können.

5. Verbindungen der Formel (I), wie in Anspruch 1 definiert, zur Prävention und/oder Behandlung von Erkrankungen.

6. Arzneimittel enthaltend mindestens eine Verbindung der Formel (1), wie in Anspruch 1 definiert, und mindestens einen weiteren Hilfsstoff.

7. Arzneimittel enthaltend mindestens eine Verbindung der Formel (I), wie in Anspruch 1 definiert, und mindestens einen weiteren Wirkstoff.

8. Verwendung von Verbindungen der Formel (I), wie in Anspruch 1 definiert, zur Herstellung eines Arzneimittels zur Prävention und/oder Behandlung von thromboembolischen Erkrankungen, insbesondere Herzinfarkt, Angina Pectoris (eingeschlossen instabile Angina), Reokklusionen und Restenosen nach einer Angioplastie oder aortokoronarem Bypass, Hirnschlag, transitorische ischämische Attacken, periphere arterielle Verschlusskrankheiten, Lungenembolien oder tiefe venöse Thrombosen.

9. Verwendung von Verbindungen der Formel (I), wie in Anspruch 1 definiert, zur Herstellung eines Arzneimittels zur Prävention und/oder Behandlung der disseminierten intravasalen Gerinnung (DIC).

10. Verwendung von Verbindungen der Formel (I), wie in Anspruch 1 definiert, zur Herstellung eines Arzneimittels zur Prävention und/oder Behandlung von Erkrankungen wie Atherosklerose, Arthritis, Alzheimer'sche Erkrankung oder Krebs.

11. Verfahren zur Verhinderung der Blutkoagulation in vitro, insbesondere bei Blutkonserven oder biologischen Proben, die Faktor Xa enthalten, **dadurch gekennzeichnet, dass** Verbindungen der Formel (I), wie in Anspruch 1 definiert, zugegeben werden.

## Claims

1. Compounds of the formula (I) 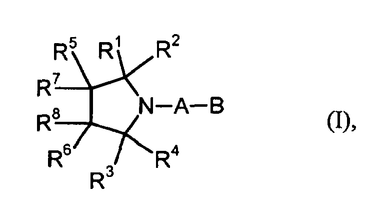
in which
R¹ and R² together represent O and
R³ and R⁴ together represent O,
or
R¹ represents hydrogen, hydroxy or (C₁-C₄)-alkoxy,
R² represents hydrogen and
R³ and R⁴ together represent O,
or
R¹ and R² together represent O, R³ represents hydrogen, hydroxy or (C₁-C₄)-alkoxy and
R⁴ represents hydrogen,
R⁵ and R⁶ represent hydrogen and
R⁷ and R⁸ together represent 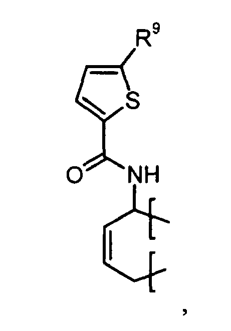
in which
R⁹ represents halogen, trifluoromethyl or methyl,
or
R⁵, R⁶, R⁷ and R⁸ together represent 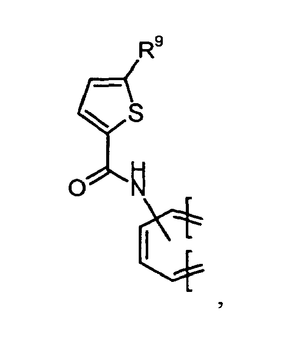 in which
R⁹ is as defined above,
A represents (C₁-C₄)-alkanediyl or (C₂-C₄)-alkenediyl,
B represents phenylene or cyclohexanediyl, which radicals may be substituted by amino, urea, sulphamoyl, -C(=N-NH-C(=NH)-NH₂)-H, -C(=NR¹⁰)-R¹¹,
in which
R¹⁰ represents hydrogen or -NH-C(=NH)-NH₂, R¹¹ represents -NR¹²R¹³ or 5- to 10-membered heterocyclyl,
in which R¹² and R¹³, independently of one another, represent hydrogen,
(C₁-C₄)-alkyl or (C₃-C₇)-cycloalkyl, (C₁-C₄)-alkyl, which for its part may be substituted by cyano, (C₁-C₄)-alkoxycarbonyl, optionally (C₁-C₄)-alkyl-substituted 5- to 10-membered heterocyclyl, 5- to 10-membered heteroaryl, tri-(C₁-C₄)-alkylammonium, -NR¹⁴R¹⁵, -C(=NR¹⁶)-R¹⁷, -N-C(=O)-R¹⁸ or -N-C(=O)-NH-R¹⁹,
in which
R¹⁴ represents hydrogen, mono- or di-(C₁-C₄)-alkylamino, (C₁-C₄)-alkyl which is optionally substituted by 5- to 10-memberedheteroaryl, represents 5- to 10-membered heterocyclyl, (C₆-C₁₀)-aryl or 5- to 10-membered heteroaryl, where the rings for their part may be substituted by halogen,R¹⁵ represents hydrogen or optionally cyano-substituted (C₁-C₄)-alkyl,
R¹⁶ represents hydrogen or hydroxy,
R¹⁷ represents amino, 5- to 10-membered heterocyclyl, optionally amino- or trifluoromethyl-substituted mono- or di-(C₁-C₄)-alkylamino, optionally trifluoromethyl-substituted (C₃-C₇)-cycloalkylamino,
R¹⁸ represents trifluoromethyl, (C₁-C₄)-alkyl, (C₁-C₄)-alkoxy or 5-to 10-membered heterocyclyl which is optionally substituted by (C₁-C₄)-alkyl,
R¹⁹ represents hydrogen, amino, dimethylamino, 5- to 10-membered heterocyclyl, 5- to 10-membered heteroaryl, or represents (C₁-C₄)-alkyl which is optionally substituted by (C₁₋C₄)-alkoxycarbonyl, dimethylamino, carbamoyl or 5- to 10-membered heteroaryl,
(C₁-C₄)-alkoxy, which for its part may be mono- or disubstituted, independently of one another, by cyano, thiocarbamoyl, optionally (C₁-C₄)-alkyl-substituted 5- to 10-membered heterocyclyl, optionally halogen-substituted 5- to 10-membered heteroaryl, optionally -C(=NR²⁰)-R²¹-substituted (C₆-C₁₀)-aryl or -C(=NR²⁰)-R²¹,
in which
R²⁰ represents hydrogen, hydroxy, (C₃-C₇)-cycloalkyl or optionally hydroxy-substituted (C₁-C₄)-alkyl,
R²¹ represents amino, (C₁-C₄)-alkylthio, (C₃-C₇)-cycloalkylamino, benzylamino or 5- to 10-membered heterocyclyl,
5- to 10-membered heterocyclyl which for its part may be substituted by (C₁-C₄)-alkyl,
(C₆-C₁₀)-aryl which for its part may be mono- or disubstituted, independently of one another, by halogen, cyano, carbamoyl or optionally amino-substituted (C₁-C₄)-alkyl,
5- to 10-membered heteroaryloxy which for its part may be substituted by amino or N-(C₁-C₄)-alkylaminocarbonyl,
or 5- to 10-membered heteroaryl which for its part may be substituted by amino,
and their salts, hydrates, hydrates of the salts and solvates.

2. Compounds according to Claim 1
in which
R¹ and R² together represent O and
R³ and R⁴ together represent O,
or
R¹ represents hydrogen, hydroxy, methoxy or ethoxy,
R² represents hydrogen and
R³ and R⁴ together represent O,
or
R¹ and R² together represent O,
R³ represents hydrogen, hydroxy, methoxy or ethoxy and
R⁴ represents hydrogen,
R⁵ and R⁶ represent hydrogen and
R⁷ and R⁸ together represent 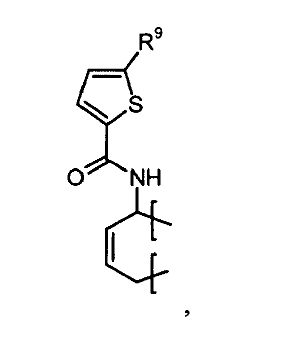
in which
R⁹ represents halogen or trifluoromethyl,
or
R⁵, R⁶, R⁷ and R⁸ together represent 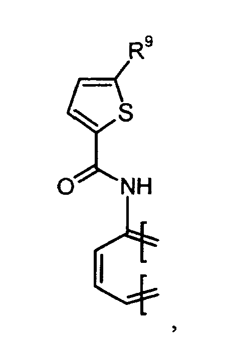
in which
R⁹ is as defined above,
A represents (C₁-C₃)-alkanediyl or (C₂-C₃)-alkenediyl,
B represents phenylene or cyclohexanediyl, which radicals may be substituted by -C(=NR¹⁰)-R¹¹
in which
R¹⁰ represents hydrogen,
R¹¹ represents -NR¹²R¹³ or 5- to 10-membered heterocyclyl,
in which
R¹² and R¹³, independently of one another, represent hydrogen, (C₁-C₄)-alkyl or (C₃-C₇)-cycloalkyl,
methyl or ethyl which for their part may be substitued by cyano, methoxycarbonyl, optionally methyl- or ethyl-substituted 5- or 6-membered heterocyclyl, 5- or 6-membered heteroaryl, tri-(C₁-C₂)-alkylammonium, -NR¹⁴R¹⁵, -C(=NR¹⁶)-R¹⁷ , -N-C(=O)-R¹⁸ or -N-C(=O)-NH-R¹⁹,
in which
R¹⁴ represents hydrogen, dimethylamino, methyl or ethyl, which radicals are optionally substituted by 5- or 6-membered heteroaryl, represents 5- or 6-membered heterocyclyl or 5- or 6-membered heteroaryl, which may be substituted by halogen,
R¹⁵ represents hydrogen, methyl or ethyl,
R¹⁶ represents hydrogen,
R¹⁷ represents amino, 5- or 6-membered heterocyclyl, optionally amino- or trifluoromethyl-substituted mono- or di-(C₁-C₄)-alkylamino, optionally trifluoromethyl-substituted (C₃-C₇)-cycloalkylamino,
R¹⁸ represents trifluoromethyl or (C₁-C₄)-alkyl,
R¹⁹ represents hydrogen, amino or optionally methoxy- or ethoxycarbonyl-substituted (C₁-C₄)-alkyl,
methoxy or ethoxy which for their part may be mono- or disubstituted, independently of one another, by optionally methyl-substituted 5- or 6-membered heterocyclyl or -C(=NR²⁰-R²¹,
in which
R²⁰ represents hydrogen,
R²¹ represents amino, (C₃-C₆)-cycloalkylamino, benzylamino or 5-or 6-membered heterocyclyl,
or pyridyl,
and their salts, hydrates, hydrates of the salts and solvates.

3. Compounds according to Claim 1
in which
R¹ and R² together represent O and
R³ and R⁴ together represent O,
or
R¹ represents hydrogen, hydroxy or methoxy,
R² represents hydrogen and
R³ and R⁴ together represent O,
or
R¹ and R² together represent O,
R³ represents hydrogen, hydroxy or methoxy and
R⁴ represents hydrogen,
R⁵ and R⁶ represent hydrogen and
R⁷ and R⁸ together represent 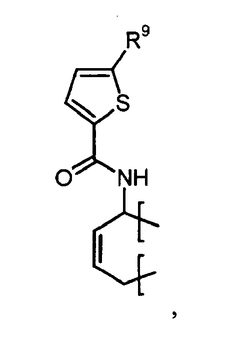
in which
R⁹ represents chlorine or bromine,
or
R⁵, R⁶, R⁷ and R⁸ together represent 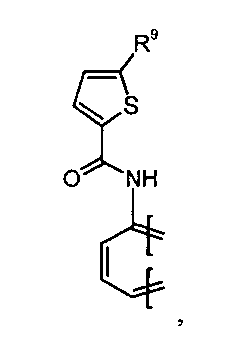
in which
R⁹ is as defined above,
A represents methanediyl or ethanediyl,
B represents phenylene which may be substituted by -C(=NR¹⁰)-R¹¹
in which
R¹⁰ represents hydrogen,
R¹¹ represents amino,
methyl which for its part may be substituted by cyano, optionally methyl-substituted imidazolinyl or tetrahydropyrimidinyl, -NR¹⁴R¹⁵ or -C(=NR¹⁶)-R¹⁷,
in which
R¹⁴ represents optionally pyridyl-substituted methyl or pyridyl,
R¹⁵ represents hydrogen,
R¹⁶ represents hydrogen,
R¹⁷ represents amino, piperidinyl, morpholinyl, pyrrolidinyl, optionally amino- or trifluoromethyl-substituted mono- or di-(C₁-C₃)-alkylamino or optionally trifluoromethyl-substituted cyclopropyl-, cyclopentyl- or cyclohexylamino,
or methoxy or ethoxy which for their part may be substituted by -C(=NH)-NH₂,
and their salts, hydrates, hydrates of the salts and solvates.

4. Process for preparing compounds of the formula (1) as defined in Claim 1, **characterized in that**
either
[A] compounds of the formula (IV) 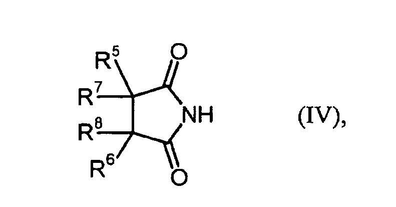
in which R⁵, R⁶, R⁷ and R⁸ are as defined in Claim 1
are reacted with compounds of the formula (V)
Y-A-B (V),
in which A and B are as defined in Claim 1 and Y represents a suitable leaving group
to give compounds of the formula (I)
in which both R¹ and R² and R³ and R⁴, in each case together, represent O and R⁵, R⁶, R⁷, R⁸, A and B are as defined in Claim 1,
or
[B1] compounds of the formula (VIII) or (VIIIa) 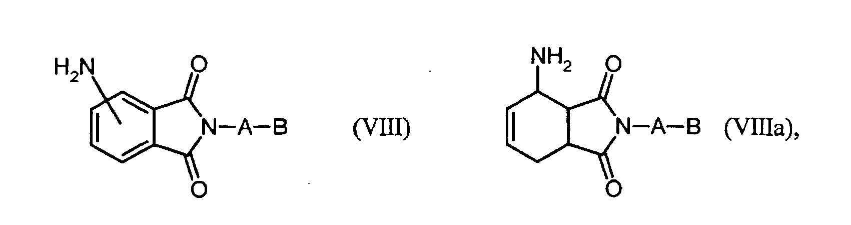
in which A and B are as defined in Claim 1
are reacted with compounds of the formula (III) 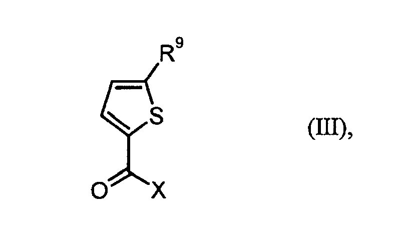
in which R⁹ is as defined in Claim 1 and X represents a leaving group
to give compounds of the formula (I)
in which both R¹ and R² and R³ and R⁴, in each case together, represent O and R⁵, R⁶, R⁷, R⁸, A and B are as defined in Claim 1,
or
[B2] compounds of the formula (IX) 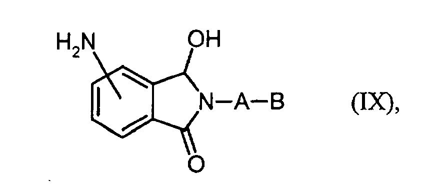
in which A and B are as defined in Claim 1
or compounds of the formula (X) 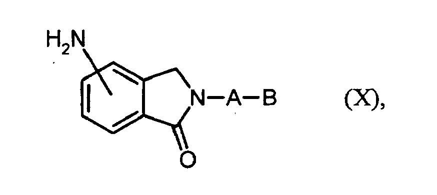
in which A and B are as defined in Claim 1
are reacted with compounds of the formula (III) to give compounds of the formula (I)
in which R¹ and R² together represent O, R³ represents hydrogen or hydroxy, R⁴ represents hydrogen or R³ and R⁴ together represent O, R¹ represents hydrogen or hydroxy, R² represents hydrogen and R⁵, R⁶, R⁷, R⁸, A and B are as defined in Claim 1,
or
[C] compounds of the formula (XIV) 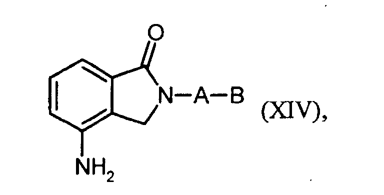
in which A and B are as defined in Claim 1
are reacted with compounds of the formula (III) to give compounds of the formula (I)
in which R¹ and R² represent hydrogen, R³ and R⁴ together represent O and R⁵, R⁶, R⁷, R⁸, A and B are as defined in Claim 1,
where the resulting compounds of the formula (I) may, if appropriate, subsequently be subjected to further derivatization which can be carried out by customary methods.

5. Compounds of the formula (I) as defined in Claim 1 for the prevention and/or treatment of disorders.

6. Medicament, comprising at least one compound of the formula (I) as defined in Claim 1 and at least one further auxiliary.

7. Medicament, comprising at least one compound of the formula (I) as defined in Claim 1 and at least one further active compound.

8. Use of compounds of the formula (I) as defined in Claim 1 for preparing a medicament for the prevention and/or treatment of thromboembolic disorders, in particular myocardial infarction, angina pectoris (including unstable angina), reocclusions and restenoses after angioplasty or aortocoronary bypass, stroke, transitory ischaemic attacks, peripheral arterial occlusive diseases, pulmonary embolisms or deep venous thromboses.

9. Use of compounds of the formula (I) as defined in Claim 1 for preparing a medicament for the prevention and/or treatment of disseminated intravascular coagulation (DIC).

10. Use of compounds of the formula (I) as defined in Claim 1 for preparing a medicament for the prevention and/or treatment of disorders such as atherosclerosis, arthritis, Alzheimer's disease or cancer.

11. Method for preventing the coagulation of blood in vitro, in particular in the case of banked blood or biological samples containing factor Xa, **characterized in that** compounds of the formula (I) as defined in Claim 1 are added.

## Revendications

1. Composés de formule (I) 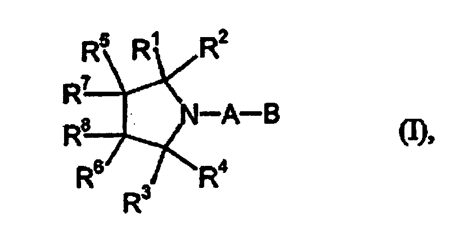
dans laquelle
R¹ et R² représentent ensemble O et
R³ et R⁴ représentent ensemble O,
ou bien
R¹ représente l'hydrogène, le groupe hydroxy ou un reste alkoxy en C₁ à C₄,
R² représente l'hydrogène et
R³ et R⁴ représentent ensemble O,
ou bien
R¹ et R² représentent ensemble O,
R³ est l'hydrogène, le groupe hydroxy ou un reste alkoxy en C₁ à C₄ et
R⁴ représente l'hydrogène,
R⁵ et R⁶ représentent l'hydrogène et
R⁷ et R⁸ forment ensemble un groupe 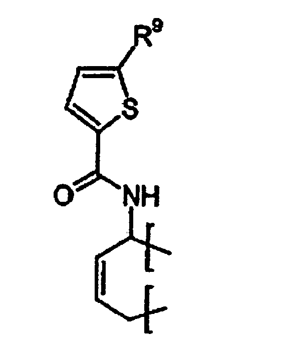
dans lequel
R⁹ représente un halogène, un reste trifluorométhyle ou méthyle,
ou bien
R⁵, R⁶, R⁷ et R⁸ forment ensemble un groupe 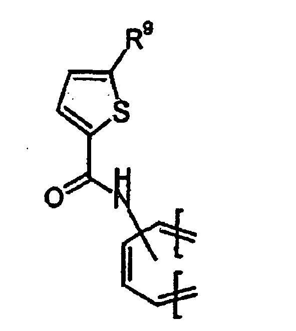
dans lequel
R⁹ a la définition indiquée ci-dessus,
A est un reste alcanediyle en C₁ à C₄ ou alcènediyle en C₂ à C₄,
B désigne un reste phénylène ou un reste cyclohexanediyle qui peut être substitué par un groupe amino, urée, sulfamoyle, -C(=N-NH-C(=NH)-NH₂)-H, -C(=NR¹⁰)-R¹¹
ou
R¹⁰ représente l'hydrogène ou un reste -NH-C (=NH) -NH₂,
R¹¹ représente un reste -NR ¹²R¹³ ou un reste
hétérocyclyle pentagonal à décagonal, où
R¹² et R¹³ représentent, indépendamment l'un de l'autre, l'hydrogène, un reste alkyle en C₁ à C₄ ou cycloalkyle en C₃ à C₇,
un reste alkyle en C₁ à C₄ qui peut lui-même être substitué par un radical cyano, (alkoxy en C₁ à C₄)carbonyle, hétérocyclyle pentagonal à décagonal éventuellement substitué par un radical alkyle en C₁ à C₄, hétéroaryle pentagonal à décagonal, tri(alkyle en C₁ à C₄) ammonium, -NR¹⁴R¹⁵, -C(=NR¹⁶)-R¹⁷, -N-C(=O)-R¹⁸ ou -N-C (=O) -NH-R¹⁹,
où
R¹⁴ désigne l'hydrogène, un reste mono- ou di(alkyle en C₁ à C₄)amino, un reste alkyle en C₁ à C₄ éventuellement substitué par un radical hétéroaryle pentagonal à décagonal, un reste hétérocyclyle pentagonal à décagonal, un reste aryle en C₆ à C₁₀ ou un reste hétéroaryle pentagonal à décagonal, les noyaux pouvant eux-mêmes être substitués par un halogène,
R¹⁵ représente l'hydrogène ou un reste alkyle en C₁ à C₄ éventuellement substitué par un radical cyano,
R¹⁶ représente l'hydrogène ou un groupe hydroxy,
R¹⁷ représente un groupe amino, un reste hétérocyclyle pentagonal à décagonal, un reste mono- ou di (alkyle en C₁ à C₄) amino éventuellement substitué par un radical amino ou trifluorométhyle, un reste (cycloalkyle en C₃ à C₇)amino éventuellement substitué par un radical trifluorométhyle,
R¹⁸ désigne un reste trifluorométhyle, alkyle en C₁ à C₄, alkoxy en C₁ à C₄ ou un reste hétérocyclyle pentagonal à décagonal éventuellement substitué par un radical alkyle en C₁ à C₄,
R¹⁹ représente l'hydrogène, un groupe amino, un reste diméthylamino, hétérocyclyle pentagonal à décagonal, hétéroaryle pentagonal à décagonal ou un reste alkyle en C₁ à C₉ éventuellement substitué par un radical (alkoxy en C₁ à C₄)carbonyle, diméthylamino, carbamoyle ou hétéroaryle pentagonal à décagonal,
un reste alkoxy en C₁ à C₄ qui peut lui-même porter un ou deux substituants, indépendamment l'un de l'autre, cyano, thiocarbamoyle, hétérocyclyle pentagonal à décagonal éventuellement substitué par un radical alkyle en C₁ à C₄, hétéroaryle pentagonal à décagonal éventuellement substitué par un halogène, aryle en C₆à C₁₀ éventuellement substitué par un groupe -C (=NR²⁰) R²¹ ou un reste -C(=NR²⁰)-R²¹,
où
R²⁰ représente l'hydrogène, un groupe hydroxy, un reste cycloalkyle en C₃ à C₇ ou un reste alkyle en C₁ à C₄ éventuellement substitué par un radical hydroxy,
R²¹ désigne un groupe amino, un reste alkylthio en C₁ à C₄, cycloalkylamino en C₃ à C₇, benzylamino ou hétérocyclyle pentagonal à décagonal,
un reste hétérocyclyle pentagonal à décagonal qui peut lui-même être substitué par un radical alkyle en C₁ à C₄,
un reste aryle en C₆ à C₁₀ qui peut lui-même porter un
ou deux substituants, indépendamment l'un de l'autre, halogéno, cyano, carbamoyle ou alkyle en C₁ à C₄ éventuellement substitué par un radical amino,
un reste hétéroaryloxy pentagonal à décagonal qui peut lui-même être substitué par un radical amino ou N(alkyle en C₁ à C₄)aminocarbonyle
ou un reste hétéroaryle en C₅ à C₁₀ qui peut lui-même être substitué par un radical amino,
ainsi que leurs sels, leurs hydrates, les hydrates de leurs sels, et leurs produits de solvatation.

2. Composés suivant la revendication 1, dans lesquels
R¹ et R² représentent ensemble O et
R³ et R⁴ représentent ensemble O,
ou bien
R¹ représente l'hydrogène, un groupe hydroxy, un reste méthoxy ou éthoxy,
R² représente l'hydrogène et R³ et R⁴ représentent ensemble O,
ou bien
R¹ et R² représentent ensemble O,
R³ représente l'hydrogène, un groupe hydroxy, un reste méthoxy ou éthoxy et
R⁴ représente l'hydrogène,
R⁵ et R⁶ représentent l'hydrogène et
R⁷et R⁸ forment ensemble un groupe 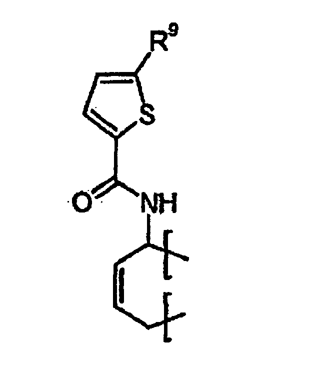
dans lequel
R⁹ est un halogène ou le reste trifluorométhyle,
ou bien
R⁵, R⁶, R⁷ et R⁸ forment ensemble un groupe 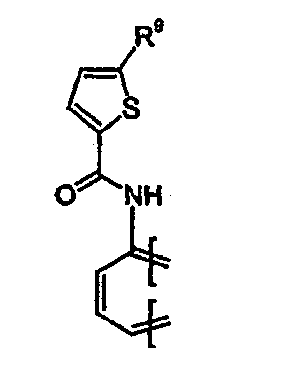
dans lequel
R⁹ a la définition indiquée ci-dessus,
A est un reste alcanediyle en C₁ à C₃ ou alcènediyle en
C₂ ou C₃,
B est un reste phénylène ou un reste cyclohexanediyle, qui peuvent être substitués par un groupe -C (=NR¹⁰)-R¹¹,dans lequel
R¹⁰ représente l'hydrogène,
R¹¹ est un groupe -NR¹²R¹³ ou un reste hétérocyclyle
pentagonal à décagonal,
où
R¹² et R¹³, indépendamment l'un de l'autre, désignent l'hydrogène, un reste alkyle en C₁ à C₄ ou cycloalkyle en C₃ à C₇,
un reste méthyle ou un reste éthyle qui peuvent eux-mêmes être substitués par un radical cyano, méthoxycarbonyle, hétérocyclyle pentagonal ou hexagonal éventuellement substitué par un radical méthyle ou éthyle, hétéroaryle pentagonal ou hexagonal, tri (alkyle en C₁ ou C₂) ammonium, -NR¹⁴R¹⁵, -C(=NR¹⁶)-R¹⁷, -N-C(=O)-R¹⁸ ou -N-C(=O)-NH-R¹⁹,
où
R¹⁴ représente l'hydrogène, un reste diméthylamino, un reste éthyle ou méthyle éventuellement substitué par un radical hétéroaryle pentagonal ou hexagonal, un reste hétérocyclyle pentagonal ou hexagonal ou un reste hétéroaryle pentagonal ou hexagonal qui peut être substitué par un halogène,
R¹⁵ représente l'hydrogène, un reste méthyle ou éthyle,
R¹⁶ représente l'hydrogène,
R¹⁷ est un groupe amino, un reste hétérocyclyle pentagonal ou hexagonal, un reste mono- ou di(alkyle en C₁ à C₄)amino éventuellement substitué par un radical amino ou trifluorométhyle, un reste cycloalkylamino en C₃ à C₇ éventuellement substitué par un radical trifluorométhyle,
R¹⁸ est un reste trifluorométhyle ou alkyle en C¹ à C⁴,
R¹⁹ représente l'hydrogène, un groupe amino ou un reste alkyle en C₁ à C₄ éventuellement substitué par un radical méthoxycarbonyle ou éthoxy-carbonyle,
un reste méthoxy ou un reste éthoxy qui peuvent eux-mêmes porter un ou deux substituants, indépendamment l'un de l'autre, hétérocyclyle pentagonal ou hexagonal portant éventuellement un substituant méthyle, ou -C(=NR²⁰)-R²¹,
où
R²⁰ représente l'hydrogène,
R²¹ est un groupe amino, cycloalkylamino en C₃ à C₆, benzylamino ou hétérocyclyle pentagonal ou hexagonal, ou un reste pyridyle,
ainsi que leurs sels, leurs hydrates, les hydrates de leurs
sels et leurs produits de solvatation.

3. Composés suivant la revendication 1,
dans lesquels
R¹ et R² représentent ensemble O et
R³ et R⁴ représentent ensemble O,
ou bien
R¹ est l'hydrogène, un groupe hydroxy ou méthoxy,
R² est l'hydrogène et
R³ et R⁴ représentent ensemble O,
ou bien
R¹ et R² représentent ensemble O,
R³ représente l'hydrogène, le groupe hydroxy ou méthoxy, et
R⁴ représente l'hydrogène,
R⁵ et R⁶ représentent l'hydrogène et
R⁷ et R⁸ forment ensemble un groupe 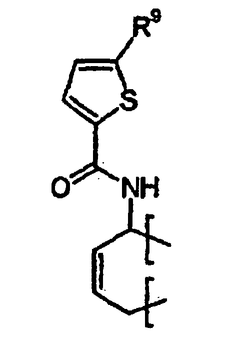
dans lequel
R⁹ est le chlore ou le brome,
ou bien
R⁵, R⁶, R⁷ et R⁸ forment ensemble un groupe 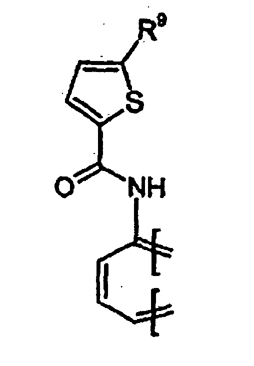
dans lequel
R⁹ a la définition indiquée ci-dessus,
A est un groupe méthanediyle ou éthanediyle,
B désigne un groupe phénylène qui peut être substitué par un reste -C (=NR¹⁰)-R¹¹,
dans lequel
R¹⁰ représente l'hydrogène,
R¹¹ est un groupe amino,
un reste méthyle qui peut lui-même être substitué par un radical cyano, imidazolinyle éventuellement substitué par un radical méthyle ou tétrahydropyrimidinyle, -NR¹⁴R¹⁵ ou -C (=NR¹⁶) -R¹⁷,
où
R¹⁴ est un reste méthyle éventuellement substitué par un radical pyridyle, ou un reste pyridyle,
R¹⁵ représente l'hydrogène,
R¹⁶ représente l'hydrogène,
R¹⁷ est un groupe amino, pipéridinyle, morpholinyle, pyrrolidinyle, un groupe mono- ou di(alkyle en C₁ à C₃)amino éventuellement substitué par un radical amino ou trifluorométhyle, ou un reste cyclopropylamino, cyclopentylamino ou cyclohexylamino éventuellement substitué par un radical trifluorométhyle,
ou bien un reste méthoxy ou un reste éthoxy qui peuvent eux-mêmes être substitués par un radical -C (=NH) -NH₂,
ainsi que leurs sels, leurs hydrates, les hydrates de leurs
sels et leurs produits de solvatation.

4. Procédé de production de composés de formule (1) telle que définie dans la revendication 1, **caractérisé en ce que** :
[A] on fait réagir des composés de formule (IV) 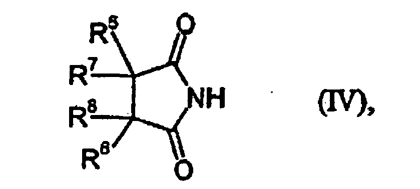
dans laquelle R⁵, R⁶, R⁷ et R⁸ ont la définition indiquée dans la revendication 1,
avec des composés de formule (V)
Y-A-B (V),
dans laquelle
A et B ont la définition indiquée dans la revendication 1 et Y représente un groupe partant convenable,
pour obtenir des composés de formule (I)
dans laquelle R¹ et R², ainsi que R³ et R⁴, représentent ensemble O dans chaque cas, et R⁵, R⁶, R⁷, R⁸, A et B ont la définition indiquée dans la revendication 1,
ou bien
[B1] on fait réagir des composés de formule (VIII) ou (VIIIa) 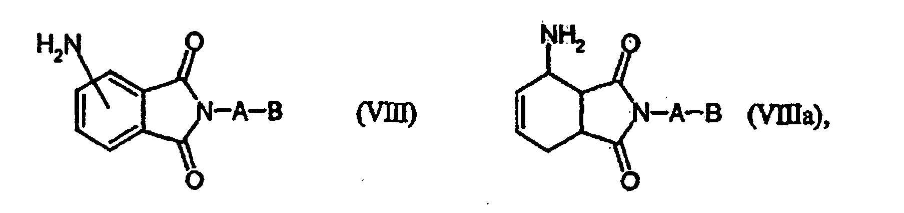
dans laquelle A et B ont la définition indiquée dans la revendication 1,
avec des composés de formule (III) 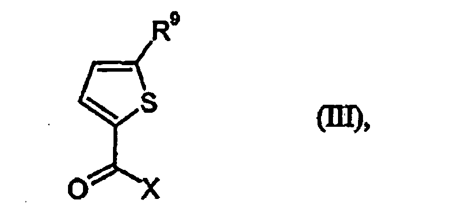
dans laquelle R⁹ a la définition indiquée dans la revendication 1 et X représente un groupe partant,
pour obtenir des composés de formule (I) dans laquelle R¹ et R², ainsi que R³ et R⁴, représentent ensemble O dans chaque cas, et R⁵, R⁶, R⁷ R⁸, A et B ont la définition indiquée dans la revendication 1,
ou bien
[B2] on fait réagir des composés de formule (IX) 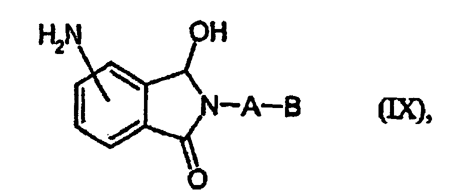
dans laquelle A et B ont la définition indiquée dans la revendication 1,
ou des composés de formule (X) 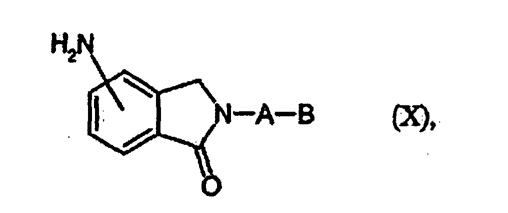
dans laquelle A et B ont la définition indiquée dans la revendication 1,
avec des composés de formule (III) pour obtenir des composés de formule (I),
où R¹ et R² représentent ensemble O, R³ est l'hydrogène
ou un groupe hydroxy, R⁴ est l'hydrogène, ou bien R³ et R⁴ forment ensemble O, R¹ est l'hydrogène ou le groupe hydroxy, R² est l'hydrogène et R⁵, R⁶, R⁷, R⁸, A et B ont la définition indiquée dans la revendication 1,
ou bien
[C] on fait réagir des composés de formule (XIV) 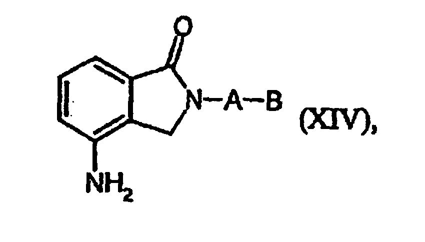
dans laquelle A et B ont la définition indiquée dans la revendication 1,
avec des composés de formule (III) pour obtenir des composés de formule (I),
où R¹ et R² représentent l'hydrogène, R³ et R⁴ représentent ensemble O et R⁵, R⁶, R⁷, R⁸, A et B ont la définition indiquée dans la revendication 1,
d'autres dérivatisations pouvant être conduites selon des modes opératoires usuels pouvant éventuellement avoir lieu ensuite pour les composés de formule (I) ainsi obtenus.

5. Composés de formule (I) telle que définie dans la revendication 1, destinés à la prévention et/ou au traitement de maladies.

6. Médicament contenant au moins un composé de formule (I) telle que définie dans la revendication 1 et au moins une autre substance active.

7. Médicament contenant au moins un composé de formule (I) telle que définie dans la revendication 1 et au moins une autre substance active.

8. Utilisation de composés de formule (I) telle que définie dans la revendication 1, pour la préparation d'un médicament destiné à la prévention et/ou au traitement de maladies thromboemboliques, en particulier de l'infarctus du myocarde, de l'angor (y compris l'angor instable), de réocclusions et de resténoses après une angioplastie ou un pontage aorto-coronarien, de l'ischémie cérébrale, des attaques ischémiques transitoires, des occlusions artérielles périphériques, des embolies pulmonaires ou des thromboses veineuses profondes.

9. Utilisation de composés de formule (I) telle que définie dans la revendication 1, pour la préparation d'un médicament destiné à la prévention et/ou au traitement de la coagulation intravasculaire disséminée (DIC).

10. Utilisation de composés de formule (I) telle que définie dans la revendication 1, pour la préparation d'un médicament destiné à la prévention et/ou au traitement de maladies telles que l'athérosclérose, l'arthrite, la maladie l'Alzheimer ou le cancer.

11. Procédé pour empêcher la coagulation du sang in vitro, en particulier dans du sang conservé ou des échantillons biologiques qui contiennent le facteur Xa, **caractérisé en ce qu'**on ajoute des composés de formule (I) telle que définie dans la revendication 1.
